(19) Europäisches Patentamt
European Patent Office
Office européen des brevets

(11) Numéro de publication: **0 203 870**
**B1**

# (12) FASCICULE DE BREVET EUROPÉEN

(45) Date de publication du fascicule du brevet: **25.01.89**

(51) Int. Cl.⁴: **C 07 H 21/00,** A 61 K 31/70

(21) Numéro de dépôt: **86401146.5**

(22) Date de dépôt: **29.05.86**

(54) **Nouveaux composés de couplage, leur procédé de préparation et leurs applications comme médiateurs dans le développement des effets des interférons.**

(30) Priorité: **30.05.85 FR 8508169**

(43) Date de publication de la demande: **03.12.86 Bulletin 86/49**

(45) Mention de la délivrance du brevet: **25.01.89 Bulletin 89/4**

(84) Etats contractants désignés: **AT BE CH DE FR GB IT LI LU NL SE**

(56) Documents cité:

(73) Titulaire: **Etablissement Public dit: CENTRE NATIONAL DE LA RECHERCHE SCIENTIFIQUE (CNRS), 15, Quai Anatole France, F-75007 Paris (FR)**

(72) Inventeur: **Lebleu, Bernard Rés. le Jardin aux Fontaines B2, 140, rue Pioch de Boutonnet, F-34000 Montpellier (FR)**
Inventeur: **Bayard, Bernard, 11, rue des Marronniers, F-34170 Castelnau Le Lez (FR)**

(74) Mandataire: **Gutmann, Ernest, S.C. Ernest Gutmann - Yves Plasseraud 67, boulevard Haussmann, F-75008 Paris (FR)**

**EP 0 203 870 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

**Description**

L'invention a pour objet de nouveaux composés de couplage obtenus entre un oligonucléotide et un vecteur d'introduction dans les cellules, leur procédé de preparation et leurs applications biologiques, comme médiateurs dans le développement de l'action des interférons, notamment dans le développement d'une partie au moins de l'action antivirale des interférons.

On sait que les interférons constituent une famille de protéines caractérisées notamment par leurs propriétés antivirales.

On a observé que l'effet antiviral des interférons est médiatisé par la synthèse de protéines particulières. Des essais spécifiques ont permis d'identifier la fonction de deux d'entre elles, qui sont toutes les deux des enzymes (BAGLIONI C., 1979, Interferon induced enzymatic activities and their role in the antiviral state, Cell 17, 255 - 264).

L'une d'entre elles est une oligonucléotide polymérase (2-5A synthétase). Cette oligonucléotide polymérase catalyse, après activation par les ARM bicaténaires et à partir d'ATP, la synthèse d'une famille d'oligonucléotides.

Ces oligonucléotides sont des chaînes courtes d'adénosines reliées par des liaisons phosphodiester 2' → 5' (KERR I. M. et BROWN R. E., 1978, pppA2'p5'A2'p5'A: An inhibitor of protein synthesis synthesized with an enzyme fraction from interferon treated cells, PNAS 75, 256 - 260) dont la formule générale peut être réprésentée par pppA $(2'p5'A)_n$. Ces oligonucléotides peuvent être désignés par "oligonucléotides 2'-5'", notamment "oligoadénylates 2'-5'" ou par $(2'-5')(A)_n$. L'un de ces oligoadénylates peut être représenté par la formule suivante:

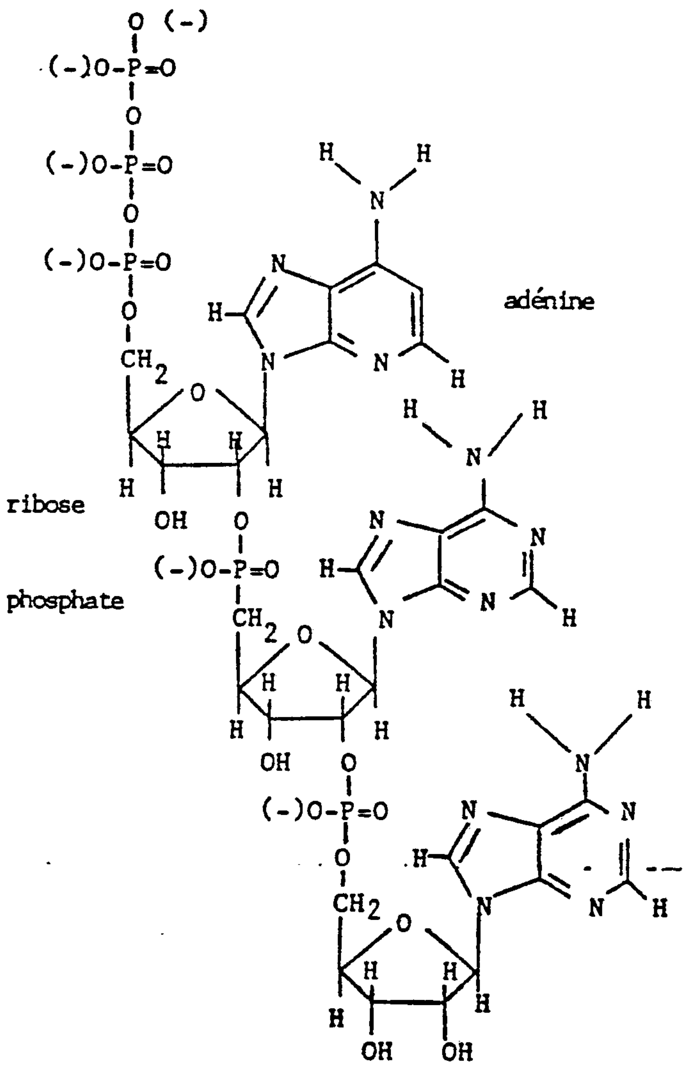


Il est composé de chaînes courtes contenant plusieurs groupes adénosine (adénine + ribose), liés entre eux par des liaisons phosphodiester, comme représenté, et dans laquelle la position en 5' du noyau d'adénine de l'adénosine terminal est lié à un nombre variable de groupes phosphate (jusqu'à 3 sur l'oligoadénylate 2' → 5' représenté).

Lorsque l'oligoadénylate 2' → 5' est totalement déphosphorylé, c'est-à-dire lorsque la position en 5' du noyau d'adénine de l'adénosine terminal est libre du susdit nombre variable de groupes phosphate, le composé résultant est désigné par "noyau (2' → 5')A3", qui est une abréviation pour "noyau riboadénylyl (2' → 5') riboadénylyl (2' → 5') riboadénosine".

Les noyaux 2'-5' correspondant aux oligoadénylates (2'-5') déphosphorylés sont également appelés "cores".

Dans la suite de la description, on désignera également par "oligoadénylate 2'-5' non modifiés" les oligoadénylates 2'-5' induits dans les cellules traitées par de l'interféron.

Il est convenu que l'expression "oligoadénylate 2' → 5" mentionnée ci-dessus et utilisée ci-après désignera également, par commodité de langage, le noyau $(2'\text{-}5')(A)_n$ partiellement ou entièrement déphosphoryle.

La découverte de ces oligoadénylates 2' → 5' a révélé une nouvelle classe d'oligonucléotides biologiquement actifs, que l'on suppose présenter un rôle important comme médiateurs de l'action de l'interféron, notamment dans l'activation de l'endoribonucléase L, qui l'interféron que dans celles non traitées, et dans l'inhibition de la synthèse des protéines. Mais les liaisons phosphodiester 2' → 5' de ces adénylates sont rapidement clivées par une enzyme désignée par 2'-phosphodiesterase (cf. la référence de BAGLIONI mentionnée ci-dessus).

L'endoribonucléase L ainsi que la 2'-phosphodiestérase sont présentes à des taux sensiblement égaux dans les cellules traitées ainsi que dans les cellules non traitées par l'interféron.

Lorsque la cellule est traitée par l'interféron, la concentration d'oligonucléotide 2'-5' polymérase augmente. L'infection par certains virus de cellules ainsi traitées entraîne la production au site de réplication virale d'ARN bicaténaires activant l'oligonucléotide 2'-5, polymérase. Il en résulte une augmentation, transitoire et éventuellement localisée au site de réplication du virus, de la concentration en oligoadénylate 2' → 5' (Nilsen T. W. et Baglioni C., 1984, Interferon 5, J. Gresser Ed., Academic Press, New York). Ces oligonucléotides activent eux-mêmes en s'y liant spécifiquement l'endoribonucléase L qui degrade les ARN messagers viraux.

Lorsque l'interféron est enlevé du milieu de culture, l'activité de l'oligonucléotide 2' → 5' polymérase décroît et la cellule perd son état antiviral.

La synthèse des protéines induites par l'interféron est transitoire et, par conséquent, les cellules maintenues dans les cultures de tissu ne maintiennent pas un niveau élevé de ces protéines.

Par ailleurs, les oligoadénylates 2' → 5' induits dans les cellules traitées par l'interféron présentent l'inconvénient d'avoir une stabilité métabolique faible. En effet, les oligoadénylates 2' → 5' non modifiés sont, d'une part, rapidement hydrolysés par une phosphodiestérase spécifique dégradant la molécule de manière processive à partir de son ribose 2' terminal, d'autre part, sont dégradés sous l'action d'une phosphatase du côté du premier ribose relié au nombre variable de groupes phosphate (Lebleu B. et Content J., 1982, Interferon 3, J. Gresser Ed., Academic Press, New York).

Des recherches ont été entreprises pour trouver des composés analogues aux oligoadénylates 2' → 5' non modifiés et présentant une activité accrue, en comparaison avec l'activité d'oligoadénylates 2' → 5' induits dans les cellules traitées par l'interféron (BAGLIONI C. et coll., 1981, Analogs of (2'-5')oligo(A). Endonuclease activation and inhibition of protein synthesis in intact cells, The Journal of Biological Chemistry, vol. 256, n° 7, p. 3253 - 3257).

Différentes recherches ont été effectuées pour synthetiser (par voie enzymatique et/ou chimique) des analogues modifiés des oligoadénylates 2' → 5' induits dans les cellules traitées par l'interféron, qui seraient résistants aux actions de dégradation, sans perdre leur activité biologique.

Parmi ces recherches, on peut citer la synthèse enzymatique à l'aide de la 2-5A synthétase de "cordycépine 2 → 5A" à partir de 2'déoxyadénosine triphosphate (DOETSCH et coll., 1981).

La cordycépine a été considérée comme inhibant la synthèse des protéines en système acellulaire et le compose dephosphorylé correspondant ("core ou noyau") a été considéré comme bloquant la transformation blastique de lymphocytes humains.

Ces résultats ont cependant été infirmés (CHAPEKAR M. S. et coll., 1983, Biochem. Res. Comm., 115, 137 - 143) et il semble que les effets observés aient été provoqués essentiellement par l'accumulation de produits toxiques de dégradation de la cordycépine.

On peut également citer, parmi les recherches effectuées, la synthèse chimique d'un analogue du noyau des oligoadénylates 2' → 5', en série xylose (IMBACH J. L. et coll., 1981, Tetrahedron Letters, vol. 22, n° 47, p. 4693 - 4702), dénommé "xylo 2'-5-A". Cet analogue s'est révélé présenter une stabilité plus importante vis-à-vis des phosphodiestérases que le noyau des oligoadénylates 2'-5' non modifiés, une activité intéressante vis-à-vis de virus à ADN, tel que l'Herpès, mais pas vis-à-vis de virus à ARN, (EPPSTEIN D., et coll., 1983, Nature, 302, 723 - 724.

On peut aussi mentionner la synthèse chimique et la modification à son extrémité 2' terminale d'un oligoadénylate 2'-5'A en un composé appelé "tailed 2'-5'A" dans lequel une chaîne d'hexylamine a été associée à un noyau morpholine, lui-même condensé par un groupe phosphate sur le group OH en 2' du ribose terminal. Ce dérivé est très stable vis-à-vis des phosphodiestérases et active l'endoribonucléase L en système acellulaire (IMAI J. et coll., 1982, J. Biol. Chem., 257, 12739 - 12741), mais son activité antivirale n'a pas été établie.

Parmi ces recherches, on peut également mentionner la synthèse chimique de dérivés modifiés d'oligoadénylates 2'-5' tels que les dérivés du 2'-5'A triphosphatés (représenté par la formule pppA2'p5'A2'p5A) dans lesquels les atomes de phosphore en bêta et gamma du groupe triphosphate en 5' sont séparés par un groupe méthylène.

Une autre modification pour obtenir des oligoadénylates 2'-5'A modifiés concerne le remplacement d'un groupe hydroxyle en 3' par un groupe $OCH_3$, soit dans l'adenosine terminal, soit dans tous les adénosines (J. A. J. DEN HARTOG et coll., 1981, J. Org. Chem., 46, 2242 - 2251).

Mais il s'est révélé que ces deux derniers groupes de composés étaient faiblement actifs, voire inactifs et ne présentaient pas une stabilité métabolique satisfaisante (cf. la référence mentionnée ci-dessus et BAGLIONI et coll., 1981, J. Biol. Chem., 256, 2353-2357).

D'autres analogues, tels que des 5'S-methylthiophosphorothioates ont été synthétisés. Certains de ces analogues se sont révélés stables. Mais a priori, les différences apparentes des propriétés de ces analogues ne semblent pas permettre d'envisager leur utilisation sur des cellules humaines à des fins thérapeutiques (HAUGH M. C., CAYLEY P. J. et coll., 1983, Europ. J. Biochem., 132, 77 - 84).

Des recherches ont également porte sur l'incidence de la modification du ou des groupes phosphate portés par le carbone en 5' des oligoadénylates 2'-5' vis-à-vis de l'activité antimitogénique (cf. TORRENCE et coll. 1983, J. Medicinal Chemistry 26, n° 12, 1674 - 1678). Les composés préparés dans le cadre de ces recherches se sont révélés présenter une activité antimitogénique, mais on a trouvé que certains d'entre eux n'activent pas l'endoribonucléase L dans un système cellulaire in vitro, ce qui empêche d'établir une corrélation entre l'activité antimitogénique et l'action antivirale.

D'autres oligonucléotides analogues des $(2'-5')(A)_n$ ont été synthétisés par voie enzymatique en remplaçant l'adénosine, notamment par la 8-azaadénosine, la toyocamycine, la sangivamycine, la formycine, la 8-bromoadénosine, la tubercidine et la guanosine. Il s'est révélé que la plupart de ces composés étaient dégradés dans des extraits cellulaires. Seuls des tests d'inhibition de la synthèse des protéines et de la prolifération cellulaire ont été effectués dans des cellules intactes, mais l'activité antivirale n'a pas été établie (B. G. HUGUES et R. K. ROBINS 1983, Biochemistry, 22, n° 9, 2127 - 2135).

On a également proposé des oligonucléotides $(2'-5')(A)_n$ modifiés à leurs extrémités 3' et/ou 5' présentant une augmentation très forte de la stabilité par rapport aux oligonucléotides non modifiés, vis-à-vis des phosphatases et des phosphodiestérases, sans modifier les propriétés de reconnaissance de l'endoribonucléase L. Ces oligonucléotides $(2'-5')(A)_n$ modifiés en 3' et/ou 5' stabilisés inhibent la synthèse protéique et exercent une activité antivirale efficace lorsqu'ils sont introduits dans les cellules par microinjection à l'aide de micropipettes (B. BAYARD et coll., 1984, Eur. J. Biochem 142 p. 291 à 298; B. BAYARD et coll. 1985, Nucleosidy and nucleotides, vol. 4 (112), p. 157 à 160).

Mais le problème qui se pose à ce jour est l'introduction des oligonucléotides 2'-5' dans les cellules, qui n'a pas encore trouvé de solutions satisfaisantes.

On a utilisé, dans ce but, les dérivés déphosphorylés, des oligonucléotides 2'-5' ci-dessous désignés par "core" 2-5A. Ces composés se sont effectivement révélés avoir une certaine activité biologique anti-herpès et antimitogène, mais une étude approfondie de leur mode d'action pour certains cas a montré que leur activité biologique pourrait résulter de la dégradation de ces composés en produits toxiques, ce qui les rend inutilisables dans le traitement thérapeutique des affections virales.

On a également proposé des modifications chimiques des $(2'-5')(A)_n$, visant à augmenter l'hydrophobicité ou à diminuer le caractère polaire, tout en maintenant les groupes phosphate à l'extremite 5'. On peut par exemple citer la synthèse de dérivés couplés à leur extrémité 3' à une chaîne d'hexylamine par un noyau N-morpholine (IMAI J. et coll. 1982, J. Biol. Chem. 257, 12739 - 12741) ou la synthèse de dérivés de structure générale $A5'p_x5'A2''(p5'A)_n$ (IMAI J. et TORRENCE P., 1984, Biochemistry, 23, 766 - 774).

Mais l'ensemble des composés proposés à ce jour sont soit biologiquement inactifs, soit incapables de traverser efficacement la membrane plasmique et il n'est par conséquent pas possible d'envisager leur utilisation dans le traitenent thérapeutique des affections virales.

La Société Demanderesse a trouvé de nouveaux composés de couplage, dont une partie de la structure moléculaire correspond à celle d'oligonucléotides, notamment oligoadénylates 2'-5'A non modifies ou leurs analogues ou des oligonucléotides modifiés, et une autre partie de la structure moléculaire correspond à celle d'un vecteur approprié susceptible d'être internalisé par les cellules, c'est-a-dire de traverser la membrane des cellules, lesquels composés présentent une activité antivirale semblable à celle de l'interféron, sont résistants vis-à-vis de la dégradation par la 2'-phosphodiestérase et par les phosphates, et peuvent traverser efficacement la membrane des cellules, permettant ainsi d'envisager leur utilisation dans le traitement des affections virales.

Ces nouveaux composés de couplage sont obtenus notamment par couplage entre un oligonucléotide et un vecteur approprié.

La Société Demanderesse a en effet trouvé que le couplage entre un oligonucléotide et un vecteur approprié, notamment un enchaînement polypeptidique approprié conduit à l'obtention d'un composé de couplage, qui malgre la modification de charge de l'enchaînement polypeptidique dû à la présence de la partie oligonucléotidique, peut néanmoins traverser la membrane des cellules et relarguer à l'intérieur de la cellule l'oligonucléotide, sans que l'activité biologique dudit oligonucléotide ne soit modifiée, ni par la présence du vecteur se trouvant également à l'intérieur de la cellule, ni par le fait que le susdit oligonucléotide a été modifié en vue du couplage.

La Société Demanderesse a également trouvé que le couplage entre un oligonucléotide et un vecteur approprié, notamment un enchaînement polypeptidique approprie dont la structure permet de reconnaître des récepteurs spécifiques de certaines cellules, conduit à l'obtention d'un composé de couplage dont la reconnaissance spécifique de certaines cellules par l'enchaînement polypeptidique n'a pas été affecté, lequel

composé de couplage est doué de la propriété de traverser lesdites cellules et de relarguer à l'intérieur de la cellule le susdit oligonucléotide, sans que l'activité biologique dudit oligonucléotide ne soit modifiée, ni par la présence du vecteur se trouvant également à l'intérieur de la cellule, ni par le fait que le susdit oligonucléotide a été modifié en vue du couplage.

L'un des aspects de l'invention est de proposer de nouveaux composés de couplage, mettant en jeu des oligonucléotides, qui peuvent être reconnus par l'endoribonucléase L, c'est-à-dire qui peuvent former avec l'endoribonucléase L un complexe actif.

Un autre aspect de l'invention est de proposer de nouveaux composés de couplage mettant en jeu des oligonucléotides, qui ont une résistance accrue vis-à-vie de la dégradation par les phosphodiestérases.

Un autre aspect de l'invention est de fournir de nouveaux composés de couplage, mettant en oeuvre des oligonucléotides, et qui sont susceptibles d'inhiber la synthèse des protéines cellulaires ou virales dans les cellules intactes.

Un autre aspect de l'invention est de fournir de nouveaux composés de couplage biologiquement actifs, qui présentent notamment une activité antivirale efficace.

Un autre aspect de l'invention est de fournir de nouveaux composés de couplage, mettant en jeu des oligonucléotides et qui sont susceptibles d'être internalisés par les cellules, c'est-à-dire de traverser efficacement la membrane plasmique des cellules, tout en gardant leur activité biologique.

Un autre aspect de l'invention est de fournir de nouveaux composés de couplage susceptibles de réagir spécifiquement avec des récepteurs particuliers de la surface membranaire de la cellule, d'être internalisés par les cellules possédant lesdits récepteurs et d'exercer, à l'intérieur de ces cellules, leur activité biologique.

Un autre aspect de l'invention est de fournir de nouveaux composés de couplage biologiquement actifs, qui présentent notamment une activité antivirale efficace et qui peuvent être ciblés, c'est-à-dire dirigés spécifiquement sur les cellules vis-à-vis desquelles il est souhaitable que l'activité des composés de couplage s'exerce.

Ces différents aspects sont obtenus par de nouveaux composés de couplage:

- correspond à celle d'un oligonucléotide, non modifié ou modifié, comprenant une chaîne de n unités nucléosidiques identiques ou différentes, n étant égal ou supérieur à 3, ces unités nucléosidiques étant reliées par des liaisons 2'-5' qui comprennent un groupe phosphate, et dans lesquels:
  . la première unité nucléosidique de la susdite chaîne peut être reliée par l'intermédiaire de son carbone en 5' à un nombre variable de groupes phosphate, l'un au moins des atomes d'oxygène des groupes phosphate, lequel atome d'oxygène relié uniquement au phosphore des groupes phosphate et n'intervenant pas dans la liaison entre deux groupes phosphate, peut être remplacé par un atome de soufre, de sélénium ou un groupe NH;
  . et/ou l'une au moins des liaisons entre deux groupes phosphate adjacents peut comporter un groupe NH ou un atome de soufre;
  . et/ou la liaison 2'-5' phosphonate qui relie deux unités nucléosidiques adjacentes est telle que l'oxygène, relié uniquement au phosphore et lequel atome d'oxygène n'entre pas dans la liaison directe entre les deux unités nucléosidiques adjacentes est remplacé par un atome de soufre, de sélénium ou un groupe NH ou bien est telle que l'oxygène qui relie le phosphore à l'extrémité 5' de l'une des unités nucléosidiques est remplacé par un atome de soufre ou un groupe NH;
- et dont une autre partie de la structure moléculaire correspond à celle d'un enchaînement peptidique qui comporte au moins une séquence latérale dont l'extrémité est constituée par un groupe aminé, notamment $NH_2$.

Une unité nucléosidique désigne un composé constitué par un pentose lié à une base purique ou pyrimidique, dans laquelle le pentose peut être sous la forme pyrane ou furane.

Dans la suite de la description, les formules représenteront les pentoses généralement sous la forme furane.

Les composés de couplage selon l'invention ont leurs unités nucléosidiques avantageusement constituées par des adénosines, l'adénosine désigne le composé constitué par du ribose lié à de l'adénine et peut être représenté par la formule:

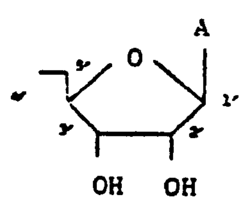

dans laquelle le ribose est sous la forme furane, mais peut être également sous la forme pyrane et dans laquelle A représente l'adénine.

Dans le cadre de l'invention, l'adénine désigne la molécule représentée par la formule suivante:

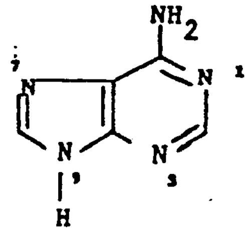

Les composés de couplage selon l'invention peuvent avoir leurs unités nucléosidiques qui sont constituées par des dérivés d'adénosines, les dérivés d'adénosine désignant le composé constitué par du ribose, lié à un dérivé de l'adénine. Parmi ces dérivés de l'adénine, on peut citer ceux de formule suivante:

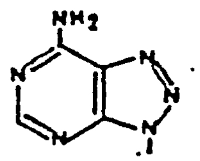
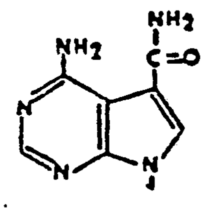
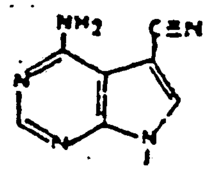
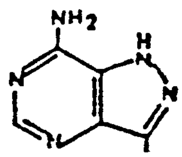
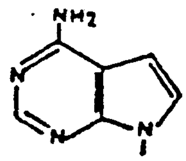
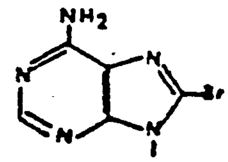

Les dérivés d'adénosine correspondants seront respectivement désignés par 8-asaadénosine, sangivamycine, toyocamycine, formycine, tubercidine, 8-bromoadénosine.

Le nombre d'unités nucléosidiques entrant dans la constitution des composés de couplage selon l'invention est égal ou supérieur à 3, ceci pouvant être expliqué par le fait qu'on a constaté la nécessité d'une certaine distance entre l'endoribonucléase L et le vecteur sus-défini sur lequel est fixé l'oligonucléotide, afin que l'affinité de l'oligonucléotide vis-à-vis de l'endoribonucléase L ne soit pas modifiée.

Le nombre d'unités nucléosidiques entrant dans la constitution des composés de couplage de l'invention n'est pas limité dans les valeurs supérieures, sous reserve que les composés de couplage obtenus puissent être associés à un véhicule physiologiquement acceptable.

Le nombre d'unités nucléosidiques peut rapidement être limité dans la mesure où l'augmentation de ce nombre et la synthèse correspondante plus difficiles ne seraient pas supportées par une augmentation suffisante de l'activité.

Le nombre d'unités nucléosidiques devrait être choisi de façon à ce que le poids moléculaire de l'oligonucléotide entrant dans la constitution du composé de couplage selon l'invention soit de préférence compris de 1 500 à 5 000 daltons.

Dans une classe préférée de composés de couplage selon l'invention, la valeur de n n'est pas supérieure à 10, et va notamment jusqu'à 7 ou 8.

Les composés de couplage selon l'invention dans lesquels la valeur de n est 3, 4 ou 5 sont particulièrement préférés.

Dans une classe préférée d'oligonucléotides selon l'invention, la première unité nucléosidique est liée à un ou plusieurs groupes phosphate.

De préférence, le nombre de ces groupes phosphate est de 2 ou 3.

Dans une classe préférée de composés de couplage de l'invention, la première unité nucléosidique est liée aux groupes phosphate suivants:

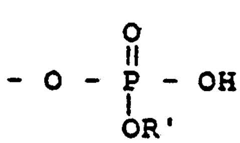 , 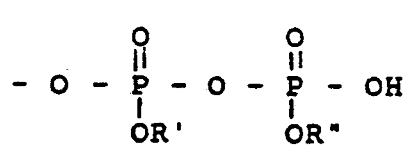 ,

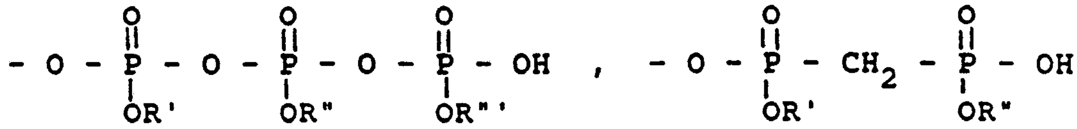

dans lesquelles R', R'', R''' représentent, indépendamment les une des autres:
- un atome d'hydrogène,
- un radical alcoyle ayant de 1 à 4 atomes de carbone, en particulier un méthyle,
- un radical éthyle substitué en bêta par un groupe cyano, aryle ou arylsulfonyle,
- un radical trihalogénoéthyle.

Dans une classe préférée de composés de couplage selon l'invention, la liaison 2' → 5' reliant deux unités nucléosidiques et comprenant au moins un atome de phosphore est une liaison phosphodiester, une liaison phosphotriester, une liaison alcoylphosphonate.

La liaison 2' → 5' phosphodiester qui relie deux unités nucléosidiques adjacents dans les composés de couplage selon l'invention peut être représentée comme suit:

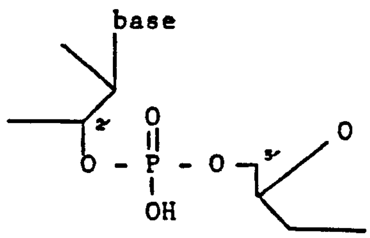

La liaison 2' → 5' phosphotriester qui relie deux unités nucléosidiques adjacents dans les composés de couplage de l'invention peut être représentée comme suit:

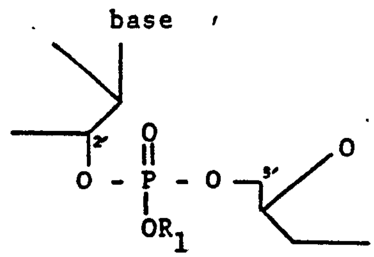

dans laquelle $R_1$ représente un radical alcoyle ayant de 1 à 4 atomes de carbone;
- un radical alcoyle ayant de 1 à 4 atomes de carbone, en particulier un méthyle,
- un radical éthyle substitué en bêta par un groupe cyano, aryle ou arylsulfonyle,
- un radical trihalogénoéthyle.

La liaison 2' → 5' phosphonate qui relie deux unités nucléosidiques adjacentes dans les composés de couplage selon l'invention peut être représentée comme suit:

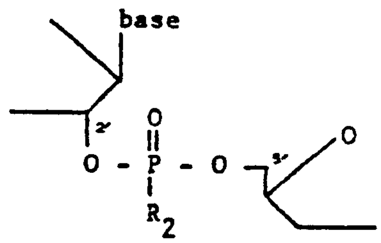

dans laquelle $R_2$ peut représenter un alcoyle ayant de 1 à 4 atomés de carbone, en particulier le méthyle.

Par oligonucleotide modifie, on designe une chaîne de n unités nucléosidiques dans lesquelles:
- soit l'un au moins des atomes d'oxygène des groupes phosphate reliés à la première unité nucléosidique, lequel atome d'oxygène est relié uniquement au phosphore des groupes phosphate et n'intervient pas dans la liaison entre deux groupes phosphate, est remplacé par un atome de soufre, de selenium ou un groupe NH;
- soit l'une au moins des liaisons entre deux groupes phosphate adjacents comporte un groupe NH ou un atome de soufre;

soit l'une au moins des liaisons phosphonate entre deux nucléosides adjacents comporte à la place de l'oxygène qui est relié uniquement au phosphore un atome de S, Se ou un groupe NH;
- soit l'une au moins des liaisons phosphonate qui relient deux nucléosides adjecents comporte, à la place de l'oxygène qui lie le phosphore à l'extrémité 5' de l'une des unités nucléosidiques, un atome de soufre ou un groupe NH.

Par oligonucléotide non modifié, on désigne une chaîne de n unités nucléosidiques dans lesquelles aucun des atomes d'oxygène des groupes phosphate n'est remplacé par un autre atome.

L'enchaînement polypeptidique selon l'invention comporte au moins une séquence latérale dont l'extrémité est constituée par un groupe aminé, notamment $NH_2$ qui est accessible, c'est-à-dire capable de réagir avec un groupe fonctionnel, notamment une fonction aldéhyde des oligonucléotides avec lesquels on souhaite effectuer le couplage entre le susdit oligonucléotide et l'enchaînement polypeptidique.

L'enchaînement polypeptidique entrant dans la constitution des composés de couplage selon l'invention doit être non toxique vis-à-vis des cellules dans lesquelles les composés de couplage sont introduits.

On désigne par groupe aminé, notamment $NH_2$, d'une séquence latérale, le groupe aminé, notamment $NH_2$, d'un polypeptide qui n'entre pas dans les liaisons

-C-NH
 ||
 O

peptidiques.

De façon avantageuse, la séquence latérale dont l'extrémité est constituée par un groupe $NH_2$ appartient au residu lysyle.

L'enchaînement polypeptidique peut également avantageusement comporter des résidus arginyle, étant donné qu'il présente une séquence latérale terminée par un groupe $NH_2$.

L'enchaînement polypeptidique entrant dans la constitution des composés de couplage selon l'invention comporte avantageusement une charge globale positive pour pouvoir induire par effet de charge la pénétration dans les cellules des oligonucleotides sus-definis.

L'enchaînement polypeptidique entrant dans la constitution des composés de couplage selon l'invention comporte avantageusement des molécules susceptibles d'être reconnues spécifiquement par des récepteurs de la membrane cellulaire et induire la pénétration dans les cellules des oligonucléotides sus-définis.

L'enchaînement polypeptidique entrant dans la constitution des composés de couplage selon l'invention comporte avantageusement au moins 5 acides aminés, de préference au moins 6 acides aminés.

Dans le cas de polypeptides synthétiques, on a avantageusement recours à des polypeptides de 5 à environ 100 acides aminés.

L'enchaînement polypeptidique doit avoir un poids moléculaire suffisant notamment pour être reconnu par les récepteurs spécifiques de la surface membranaire des cellules.

L'enchaînement polypeptidique entrant dans la constitution des composés de couplage selon l'invention a avantageusement un poids moléculaire moyen au moins d'environ 1 000.

L'enchaînement polypeptidique entrant dans la constitution des composés de couplage selon l'invention est avantageusement constitué par la polyarginine, et de préference par la polylysine.

La polylysine entrant dans les composés de couplage de l'invention comporte avantageusement 5 à environ 100 résidus lysyle.

La polylysine entrant dans les composés de couplage selon l'invention a avantageusement un poids moléculaire moyen d'environ 14 000.

Les composés de couplage de l'invention sont tels qu'il y ait au moins une molécule d'oligonucléotides fixée sur le même enchaînement polypeptidique, par l'intermédiaire de deux séquences latérales terminées par un groupe aminé, notamment $NH_2$, appartenant à l'enchaînement peptidique.

De façon avantageuse, le nombre de molécules d'oligonucléotides par rapport à une molécule d'enchaînement polypeptidique est au moins égal à 2, sans limite supérieure théorique.

En pratique, le nombre de molécules d'oligonucléotides couplées à une molécule polypeptidique est limité par le nombre de $NH_2$ disponibles sur l'enchaînement polypeptidique, c'est-à-dire par le nombre de séquences latérales terminées par des groupes aminés, notamment $NH_2$, ainsi que par le rendement de la réaction de couplage, lequel peut être modulé entre autres par les quantités respectives d'oligonucléotide et de polypeptide mises en oeuvre.

De façon avantageuse, les composés de couplage selon l'invention comportent 2 à environ 10 molécules d'oligonucléotides par molécule de polypeptide.

L'invention a egalement pour objet de nouveaux composés de couplage, dans lesquels une partie de la structure moléculaire correspond aux oligonucléotides ci-dessus définis et dans lesquels l'autre partie de la structure moléculaire est constituée par un enchaînement polypeptidique dans lequel des groupes $NH_2$ ont été fixés sur les groupes carboxyliques.

Les groupes carboxyliques d'un polypeptide sur lesquels on fixe un groupe $NH_2$ sont:
- le groupe carboxylique terminal libre qui figure dans tout polypeptide;

- et/ou les groupes carboxyliques libres des résidus glutamyle et aspartyle.

On fixe par exémple le groupe $-NH_2$ sur un groupe -COOH, par l'intermédiaire de l'hydrazine $NH_2NH_2$, ce qui transforme

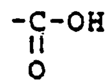

en

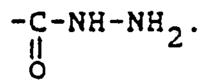

Dans le cas où le groupe COOH sur lequel on a fixé le groupe $NH_2$ appartient à un résidu aspartyle, celui-ci devient un aspartate hydrazide.

Dans le cas où le groupe COOH sur lequel on a fixé le groupe $NH_2$ appartient à un résidu glutamyle, celui-ci devient un glutamate hydrazide.

On appellera "polypeptide hydrazide" le polypeptide dans lequel l'un au moins des groupes COOH a été transformé en

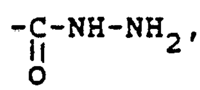

comme indiqué ci-dessus.

La présence de ces groupes $NH_2$ libres permet d'effectuer le couplage entre les oligonucléotides sus-définis et le polypeptide hydrazide.

L'invention vise également les composés de couplage dans lesquels une partie de la structure moléculaire correspond aux oligonucléotides sus-définis, et l'autre partie de la structure moléculaire correspond à un polypeptide qui comporte des séquences latérales terminées par un groupe $-NH_2$ et des groupes carboxyliques qui ont été transformés en

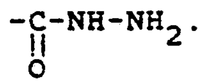

De tels polypeptides sont par exemple constitués par des asialoglycoprotéines, telles que l'asialofétuine.

L'invention vise également les composés de couplage dans lesquels une partie de la structure moléculaire correspond aux oligonucléotides sus-définis et l'autre partie de le structure moléculaire correspond à une néoglycoprotéine, par exemple une néoglycoprotéine à mannose, telle que le mannose-polylysine qui est constitué par de la polylysine, sur laquelle a été fixée au moins une molécule de mannose.

L'invention concerne plus généralement les composés de couplage dans lesquels une partie de la structure moléculaire correspond aux oligonucléotides susdéfinis, et l'autre partie de la structure moléculaire que des anticorps vis-à-vis d'antigènes, de la surface cellulaire des hormones, des glycoprotéines, des chaînes B de toxines animales ou végétales, lequel vecteur peut être couplé aux oligonucléotides par l'intermédiaire du $-NH_2$ libre des séquences latérales du polypeptide ou bien par l'intermédiaire du $-NH_2$ des groupes

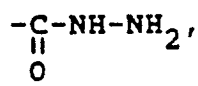

provenant de la réaction avec l'hydrazine du groupe carboxyle terminal du polypeptide ou des groupes carboxyle appartenant aux residus aspartyle et ou glutamyle.

Les composés de couplage selon l'invention peuvent être représentés par la formule (I) définie ci-dessous:

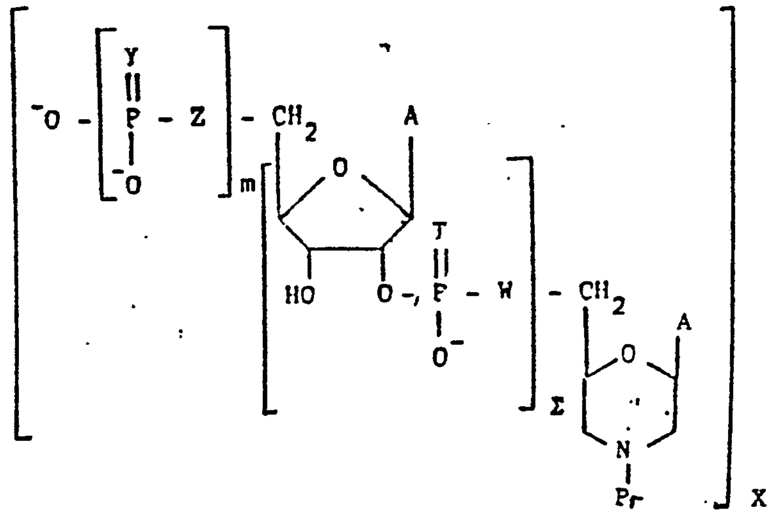

(I)

dans laquelle:

- Y et T, identiques ou différents, représentent indépendamment l'un de l'autre O, S, Se ou NH;
- Z et W, identiques ou différents, représentent indépendamment l'un de l'autre O, S ou NH;
- A représente l'adénine ou l'un de ses dérivés comme défini ci-dessus;
- Σ est un nombre entier égal à n - 1, n étant un nombre entier superieur ou egal à 3;

m est un nombre entier égal à 0 et de préférence supérieur ou égal à 2;

- X est un nombre entier supérieur ou égal à 1, de préférence compris de 2 à 10;
- Pr est un enchaînement polypeptidique comportant au moins 5 acides aminés, et l'atome d'azote appartenant au cycle:

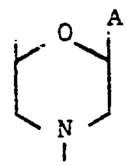

appartenant également à l'extrémité d'une séquence latérale du polypeptide pret/ou appartenant à un groupe

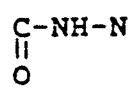

provenant d'un groupe COOR modifié par l'hydrazine.

Un groupe préféré de composés de couplage selon l'invention est constitue par ceux de formule (I):

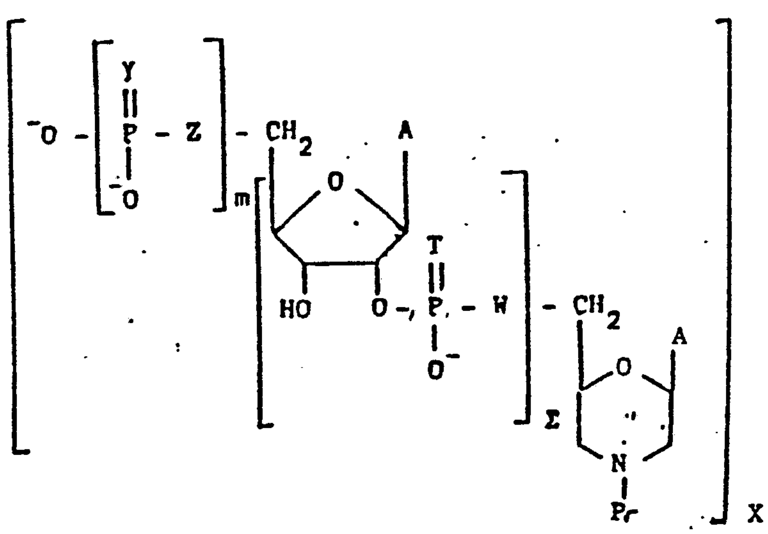

(I)

dans laquelle:
- Y et T, identiques ou différents, representent indépendamment l'un de l'autre O, S, Se ou NH;
- Z et W, identiques ou différents, représentent indépendamment l'un de l'autre O, S ou NH;
- A représente l'adénine ou l'un de ses dérivés comme défini ci-dessus;
- Σ est un nombre entier égal à n - 1, n étant un nombre entier superieur ou égal à 3;
- m est un nombre entier supérieur ou égal à 0 et de préférence supérieur ou égal à 2;
- X est un nombre entier supérieur ou égal à 1, de préférence compris de 2 à 10;
- Pr est un enchaînement polypeptidique comportant au moins 5 acides aminés, et l'atome d'azote appartenant au cycle:

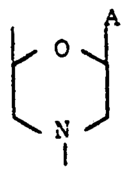

appartenant également à l'extrémité d'une séquence latérale du polypeptide Pr.

Un autre groupe préféré de composés selon l'invention est constitué par ceux de formule (II):

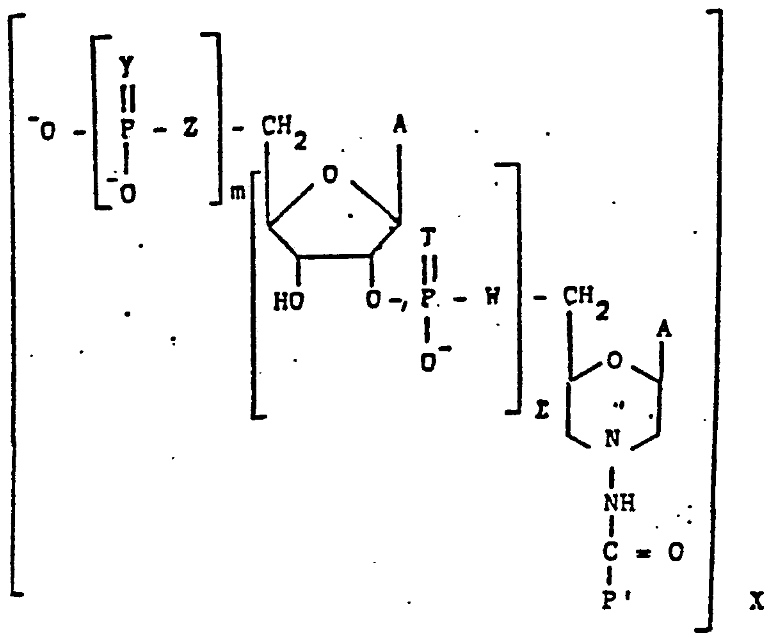

(II)

dans laquelle Y, Z, T, W, Σ, X ont les significations ci-dessus définies, P' représente un enchaînement polypeptidique comportant au moins 5 acides amines, et dans lequel le groupe

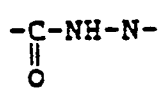

représenté sur la figure provient du groupe

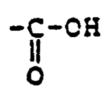

terminal du polypeptide et/ou d'un groupe

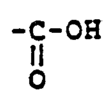

libre d'un résidu aspartyle et/ou glutamyle.

Un groupe préféré de composés de couplage selon l'invention est constitué par ceux de formule (III) suivante:

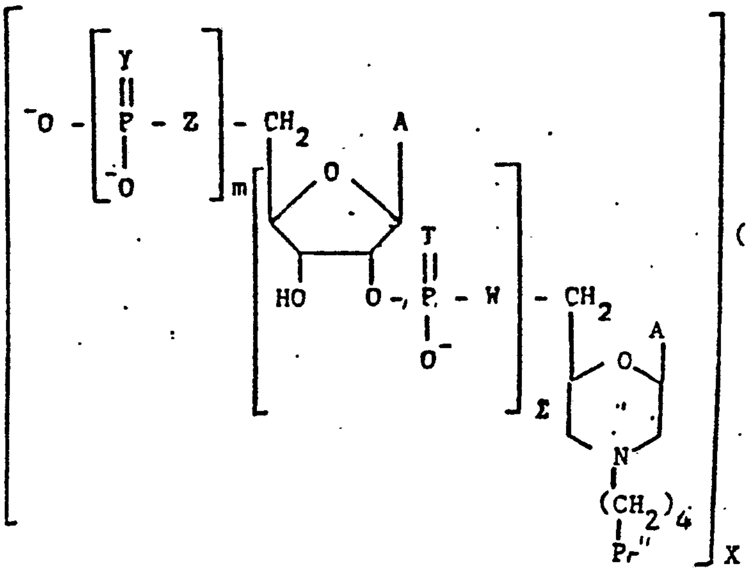

(III)

dans laquelle:

- Y et T identiques ou différents, représentent indépendamment l'un de l'autre O, S, Se ou NH;
- Z et W, identiques ou différents, représentent indépendamment l'un de l'autre O, S ou NH;
- A représente l'adénine ou l'un de ses dérivés comme définis ci-dessus;
- Σ est un nombre entier égal à n - 1, n étant un nombre entier supérieur ou égal à 3;
- X est un nombre entier égal ou supérieur à 1, de préférence compris de 2 à 10;
- m est un nombre entier supérieur ou égal à 0 et de préférence supérieur ou égal à 2;
- Pr" est un enchaînement polypeptidique comportant au moins 5 acides aminés et dans laquelle le groupe $-(CH_2)_4-$ représenté dans la formule appartient à la séquence latérale d'un résidu lysyle entrant dans la constitution de l'enchaînement polypeptidique Pr".

Un autre groupe préféré de composés de couplage selon l'invention est constitué par ceux de formule (I), dans laquelle Y, T, Z, W, A, Σ, n, X, m ont les significations ci-dessus indiquées, Pr est un enchaînement peptidique comportant au moins 5 acides aminés et l'atome d'azote appartenant au cycle:

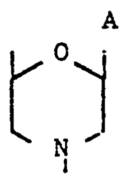

appartient également à l'extrémité de la séquence latérale d'un résidu d'arginyle.

Un tel groupe de composés peut être représenté par la formule (IV) suivante:

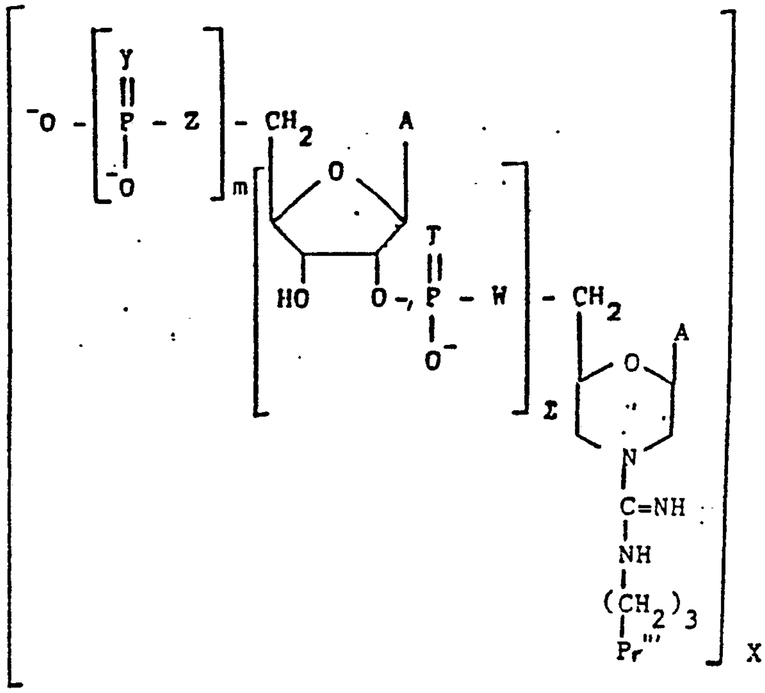

(IV)

Un groupe préféré de composés de couplage selon l'invention est constitué par ceux de formule suivante (V):

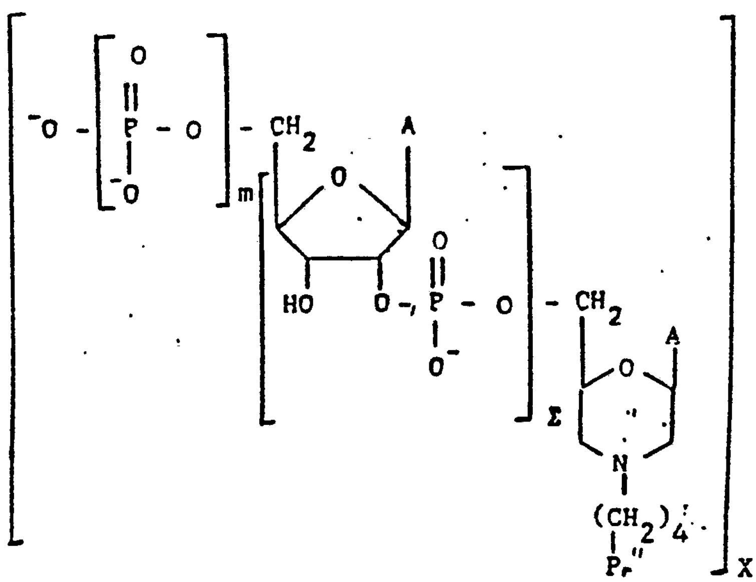

(V)

dans laquelle:
- A représente l'adénine ou l'un de ses dérivés comme défini ci-dessus;

$\Sigma$ est un nombre entier égal à n - 1, n étant un nombre entier supérieur ou égal à 3;
- m est un nombre entier supérieur ou égal à 0 et de préférence supérieur à 2;
- X est un nombre entier égal à 1 ou, de préférence compris de 2 à 10;
- Pr'' est un enchaînement peptidique comportant au moins 5 acides aminés et dans laquelle le groupe $-(CH_2)_4-$ représenté dans la formule appartient à la séquence latérale d'un résidu lysyle entrant dans la constitution de l'enchaînement polypeptidique Pr''.

Un groupe préféré de composés de couplage selon l'invention est constitué par ceux de formule suivante:

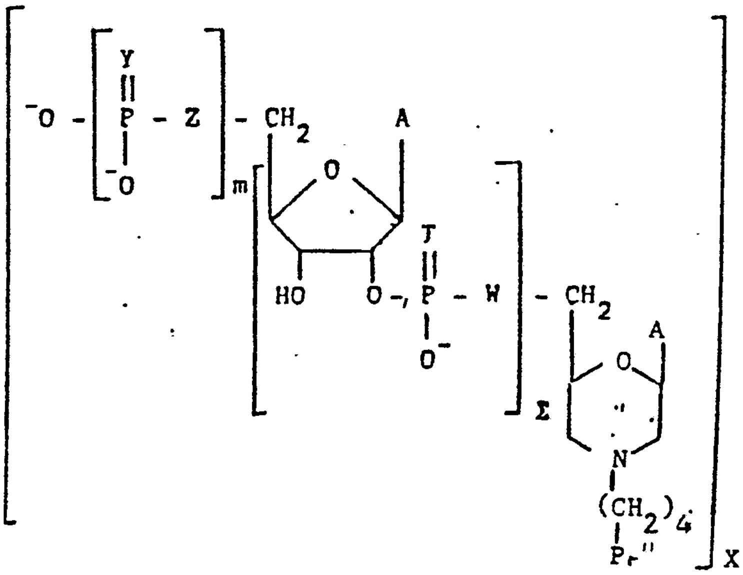

(III)

dans laquelle:

- Y et T, identiques ou différents, représentent indépendamment l'un de l'autre O, S, Se ou NH;
- Z et W, identiques ou différents, représentent indépendamment l'un de l'autre O, S ou NH;
- A représente l'adénine ou l'un de ses dérivés comme définis ci-dessus;
- Σ est un nombre entier égal à n - 1, n étant un nombre entier supérieur ou égal à 3;
- m est un nombre entier supérieur ou égal à 0 et de préférence supérieur ou égal à 2;
- X est un nombre entier égal à 1, de préférence compris de 2 à 10;
- Pr'' est un enchaînement peptidique comportant au moins 5 acides aminés et dans lequel le groupe $(CH_2)_4$- représenté dans la formule appartient à la séquence latérale d'un résidu lysyle entrant dans la constitution de l'enchaînement polypeptidique Pr'';

sous réserve que l'un au moins des éléments Y ou Z est différent de l'oxygène.

Une classe préférée de composés de couplage selon l'invention est constituée par ceux répondant à la formule (I), (II), (III), (IV) ou (V) ci-dessus définies dans lesquelles A est choisi parmi l'adénine ou ses dérivés, notamment ceux de formule:

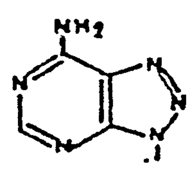
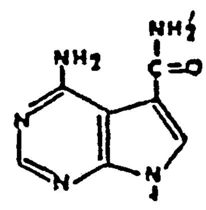
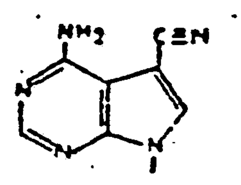
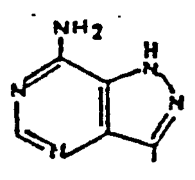
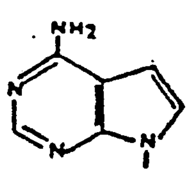
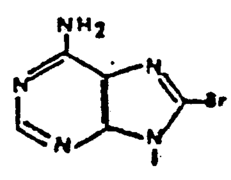

Dans une classe préférée de composés de couplage selon l'invention, le nombre m varie de 1 à 3.

Parmi les groupes reliés à la première unité nucléosidique, et de formule:

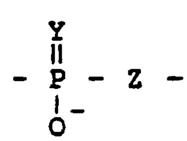

l'un au moins est tel que Y represente Se, S ou NH et/ou Z représente S ou NH, et les autres groupes:

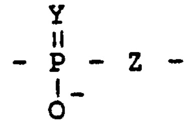

représentant des groupes phosphate.

Dans une classe préférée d'oligonucléotides selon l'invention, Y représente S, et Z représente O.

Une classe préférée de composés de couplage selon l'invention est constituée par ceux de formule (VI) suivante:

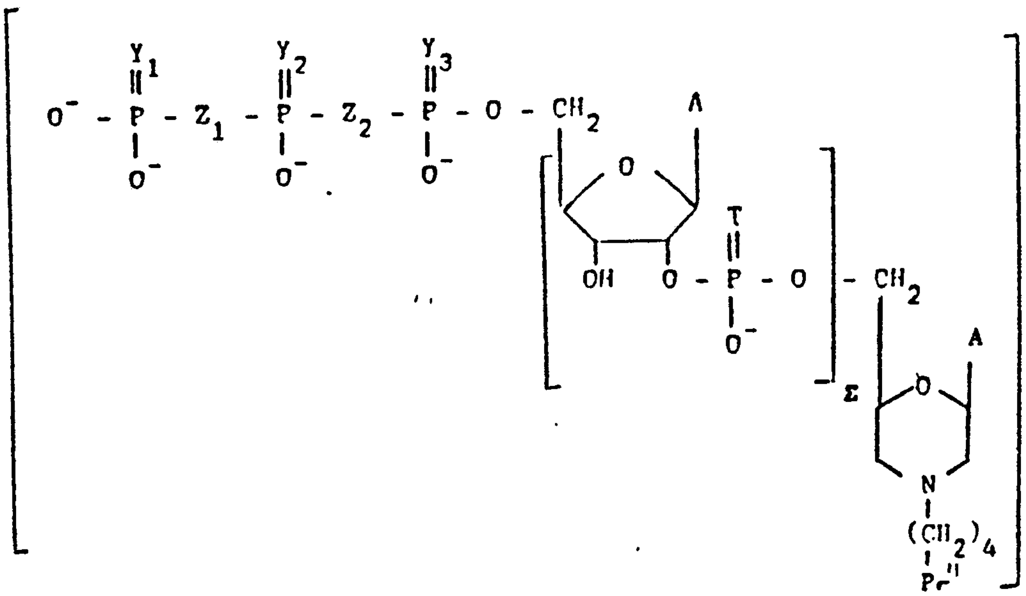

(VI)

dans laquelle:

- $Y_1$, $Y_2$, $Y_3$, T sont identiques ou différents et représentent O, S, Se, NH;
- $Z_1$ et $Z_2$ sont identiques ou différents et représentent O, S, NH;

l'un au moins des éléments $Y_1$, $Y_2$, $Y_3$, $Z_1$, $Z_2$ étant différent de l'oxygène;

- Σ représente un nombre entier égal à n - 1, n étant un nombre entier égal ou supérieur à 3;
- X est un nombre entier égal à 1, ou de préférence compris de 2 à 10;
- A est une base choisie parmi l'adénine ou ses dérivés, notamment ceux de formule:

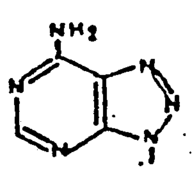
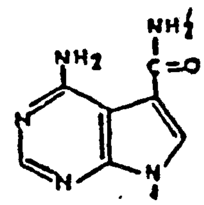
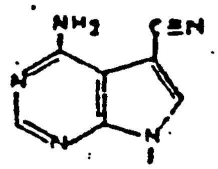

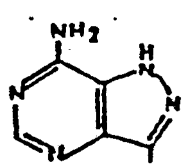
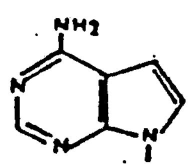
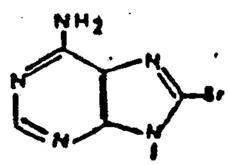

- Pr'' est un enchaînement peptidique comportant au moins 5 acides aminés et dans lequel le groupe $-(CH_2)_4-$ représente dans la formule appartient à la séquence latérale d'un résidue lysyle dans la constitution de l'enchaînement polypeptidique.

Le phosphore du groupe:

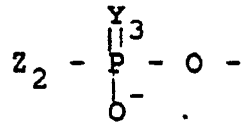

sera appelé phosphore alpha.

Le phosphore du groupe:

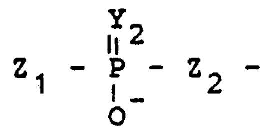

sera appelé phosphore bêta.

Le phosphore du groupe:

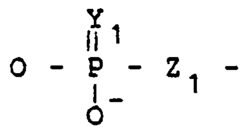

sera appelé phosphore gamma.

A l'intérieur de la classe de composés de couplage de formule (VI), une classe préférée de composés de couplage selon l'invention est constituée par les composés de couplage de formule (VII):

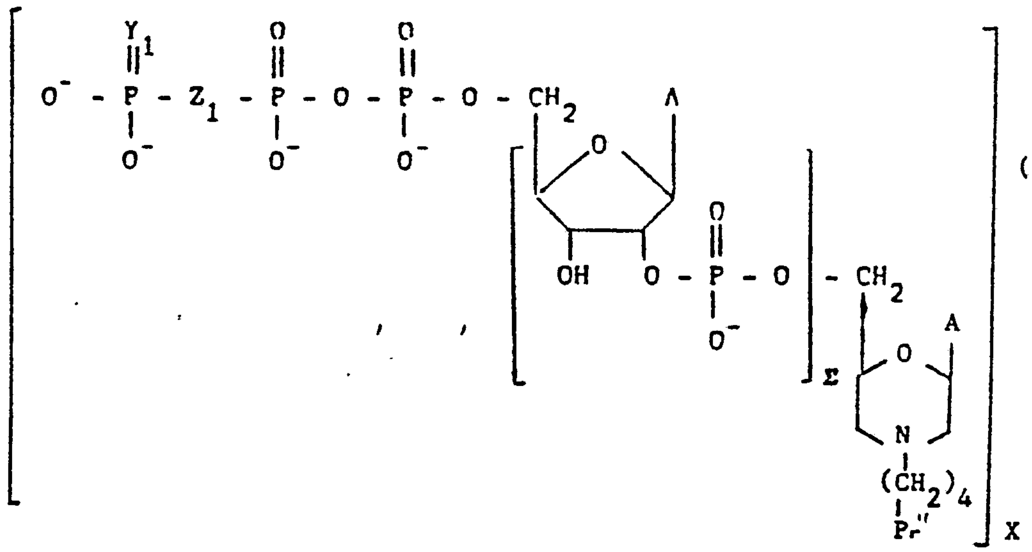

(VII)

dans laquelle:

- $Y_1$ représente S, Se ou NH;
- $Z_1$ represente O, NH ou S;
- Σ, X, A et Pr'' ayant les significations indiquées ci-dessus.

Une autre classe préférée de composés de couplage selon l'invention est constituée par ceux de formule (VIII) suivante:

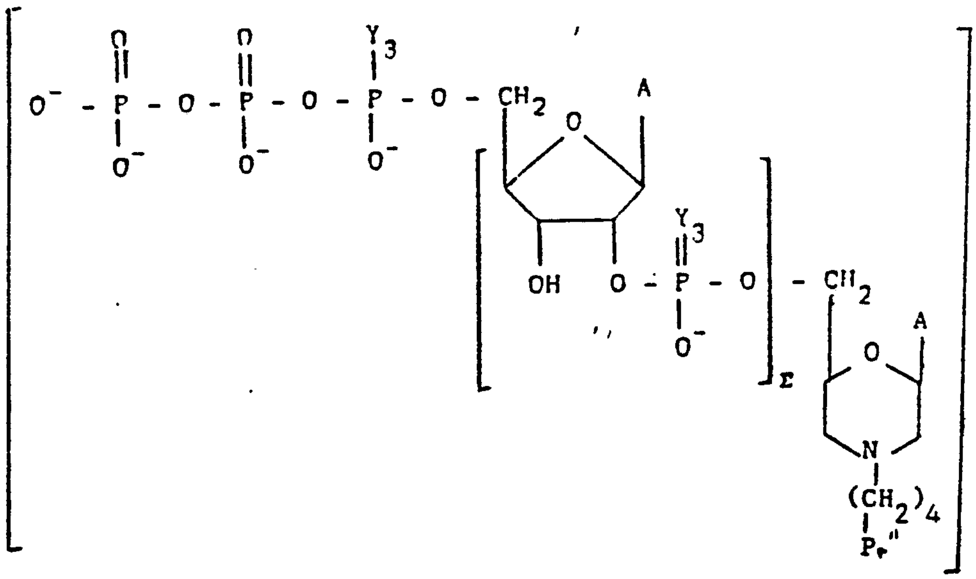

(VIII)

dans laquelle:
- $Y_3$ représente S, Se ou NH;
- Σ, X, A et Pr'' ont les significations indiquées ci-dessus.

Une autre classe de composés de couplage préférés selon l'invention est constituée par ceux de formule (IX) suivante:

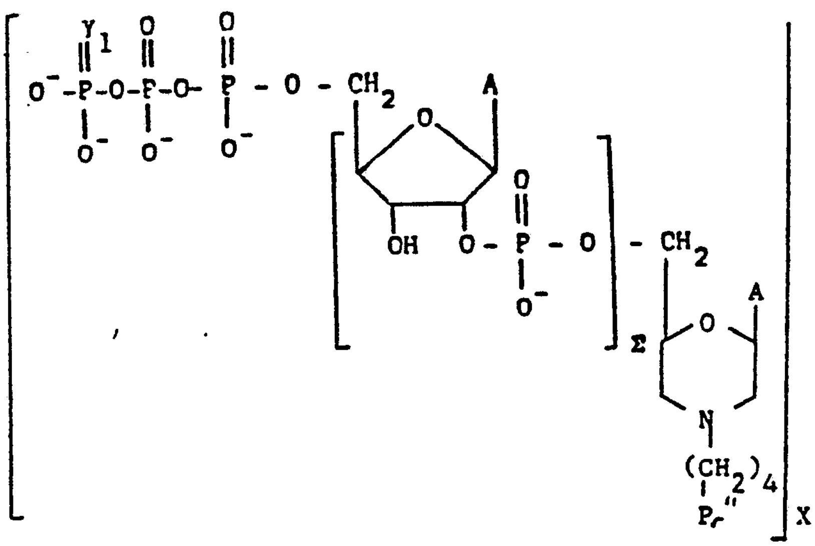

(IX)

dans laquelle:
- $Y_1$ représente Se, S ou NH;
- Σ, X, A et Pr'' ont les significations indiquées ci-dessus.

Une autre classe préférée de composés de couplage selon l'invention est constituée par ceux de formule (X) suivante:

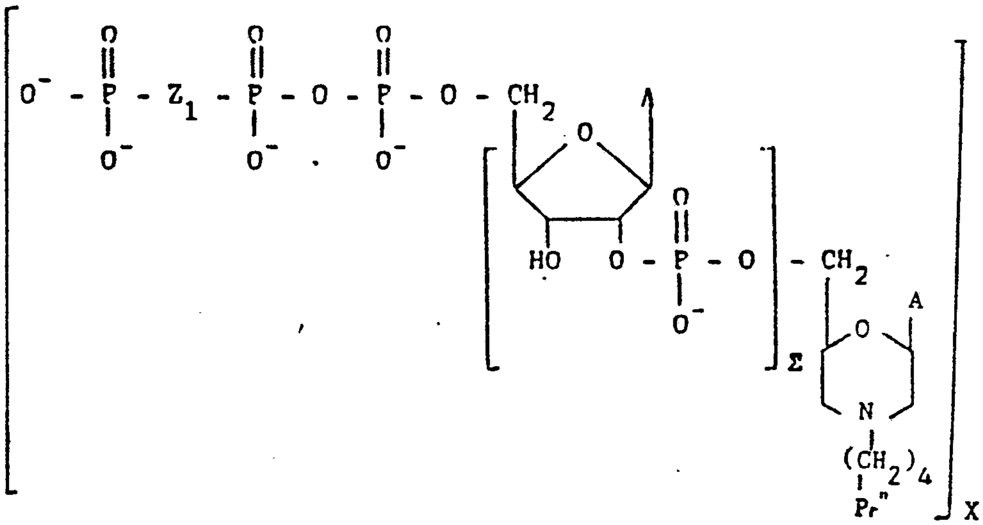

(X)

dans laquelle:
- $Z_1$ représente S ou NH;
- Σ, X, A et Pr″ ont les significations ci-dessus indiquées.

Une autre classe préférée de composés de couplage selon l'invention est constituée par ceux de formule (XI) suivante:

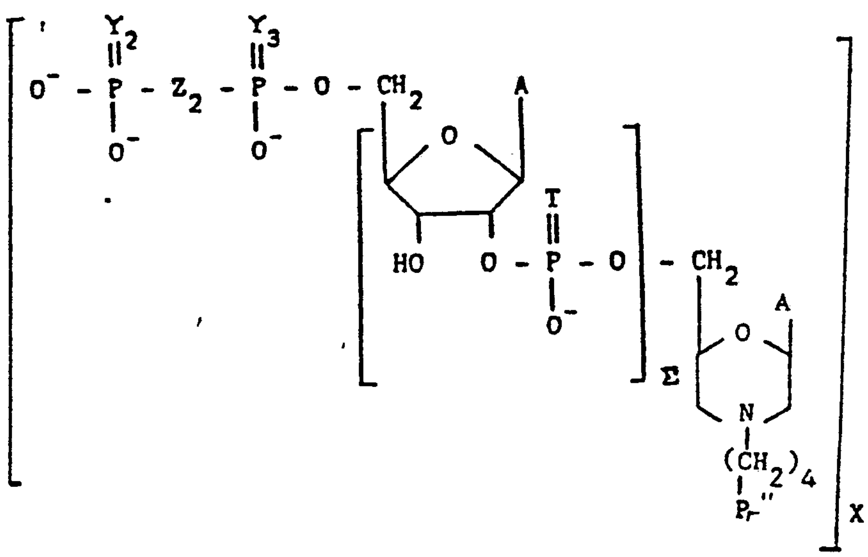

(XI)

dans laquelle:
- $Y_2$, $Y_3$ et T sont identiques ou différents et représentent O, S, Se, NH;
- $Z_2$ représente O, S, NH;

l'un au moins des éléments $Y_2$, $Y_3$, $Z_3$ étant différent de l'oxygène;
- Σ est un nombre entier égal à n - 1, n étant un nombre entier variant de 3 à 10;
- X est un nombre entier égal à 1 ou de préférence compris de 2 à 10;
- A est une base choisie parmi l'adénine ou ses dérivés notamment ceux de formule:

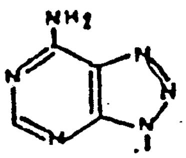
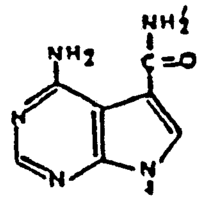
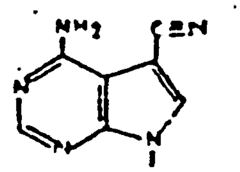

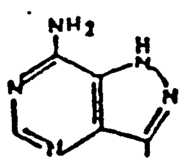
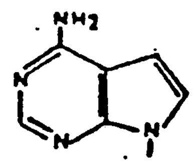
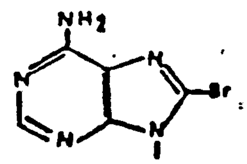

A l'intérieur de cette classe de composés de couplage, une classe préférée est constituée par ceux dans lesquels T représente l'oxygène.

Une autre classe preférée de composés de couplage selon l'invention est constituée par ceux de formule (XII) suivante:

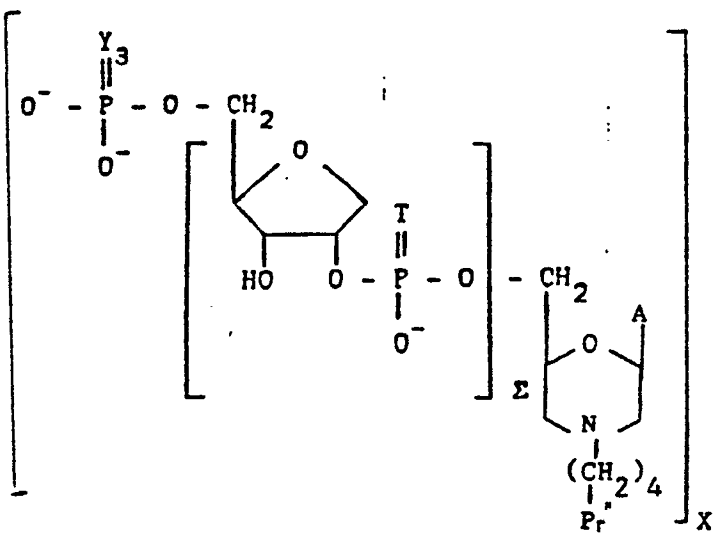

(XII)

dans laquelle:
- $Y_3$ représente S, Se, NH;
- T représente O, S, Se, NH;
- Σ est un nombre entier égal à n - 1, n variant de 3 à 10;
- X est un nombre entier égal à 1 ou de préférence compris de 2 à 10;
- A est une base choisie parmi l'adénine ou ses dérivés, notamment ceux de formule:

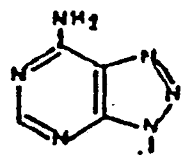
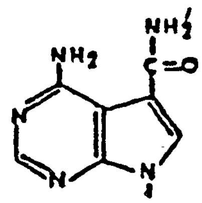
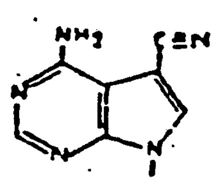

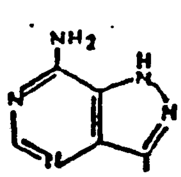
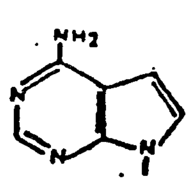
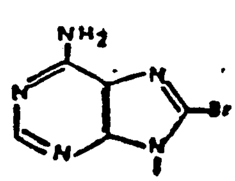

A l'intérieur de cette classe de composés de couplage, une classe préférée est constituée par ceux dans lesquels T représente l'oxygène.

Des composés de couplage particulièrement préférés selon l'invention ont pour formule:

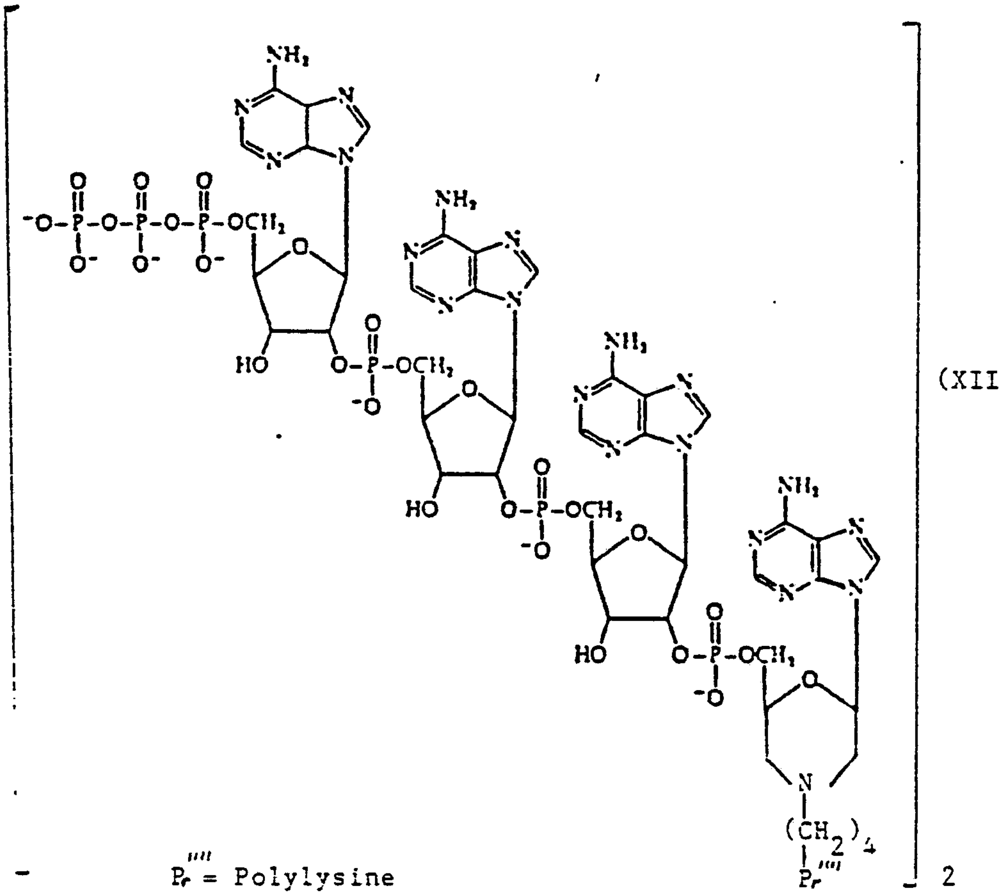

$P_r''''$ = Polylysine

(XIII)

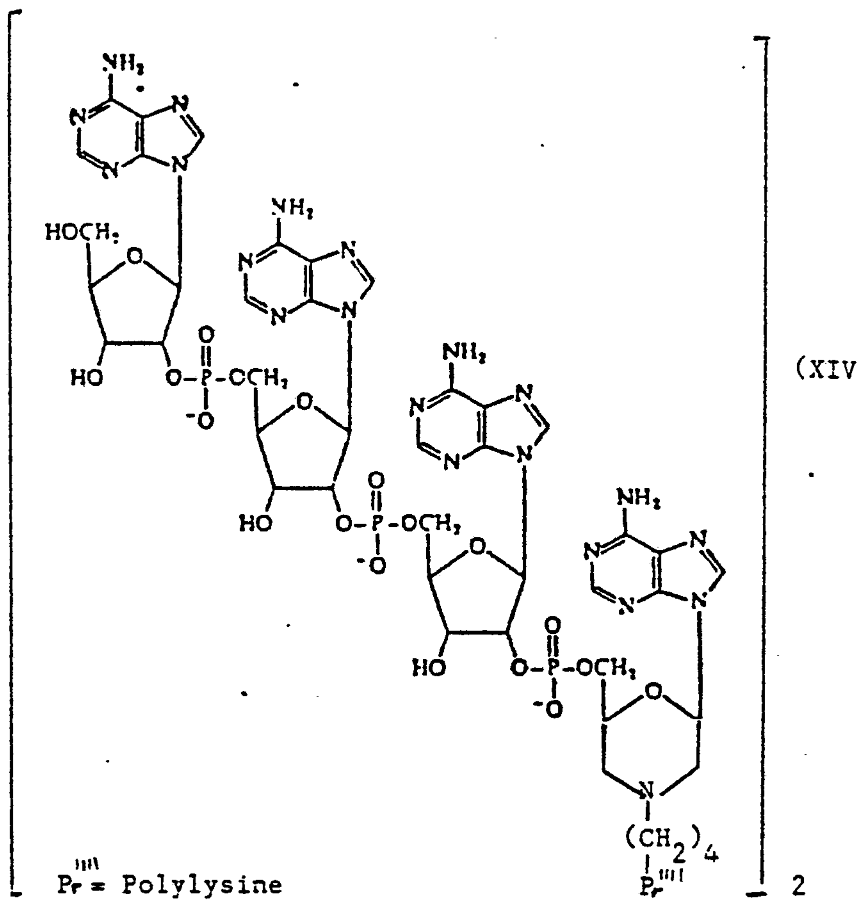

$P_r''''$ = Polylysine

(XIV)

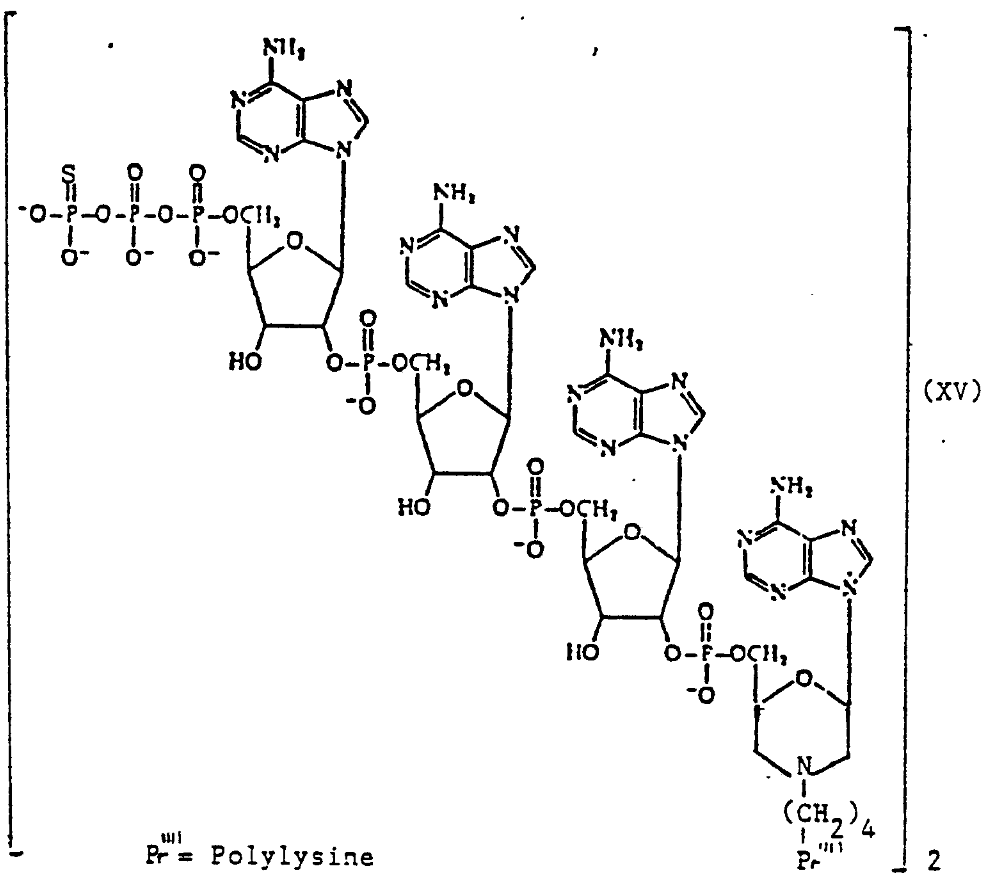

Pr''' = Polylysine

(XV)

L'invention concerne également les sels qui peuvent être obtenus par réaction des susdits oligonucléotides avec les bases appropriées, en particulier les sels d'ammoniums quaternaire, tels que le sel de triéthylammonium, et les sels minéraux, tels que le sel de sodium.

L'invention concerne également un procédé de preparation des composés de couplage.

Pour préparer les composés de couplage selon l'invention, on peut avoir recours au procédé décrit ci-après.

Ce procédé comprend:

- l'oxydation du composé de départ de formule (Ibis) suivante:

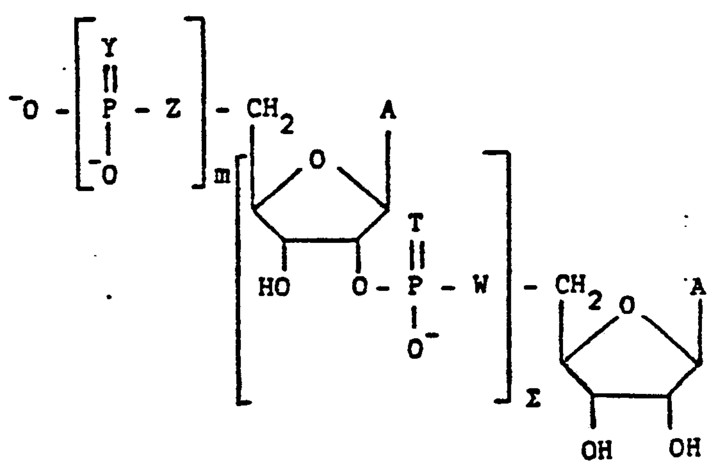

(Ibis)

dans laquelle Y, Z, T, W, A, n, Σ ont les significations indiquées ci-dessus;
pour introduire deux fonctions aldéhyde sur les carbones en 2' et 3' de la dernière unité nucléosidique et obtenir le composé de formule (Iter) suivante:

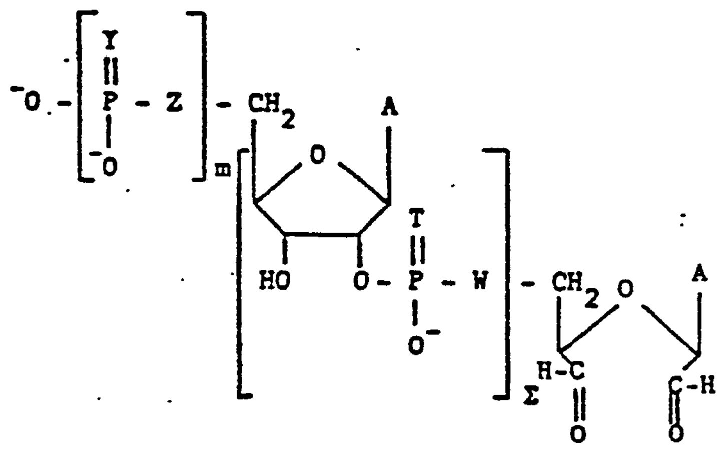

(Iter)

- l'alcoylation réductive du composé de formule (Iter) par un polypeptide Pr-$NH_2$ dont l'enchaînement comprend au moins une sequence latérale dont l'extrémité est un groupe $NH_2$, cette réaction ayant lieu entre les fonctions aldéhyde du composé (Iter) avec le groupe $NH_2$ du polypeptide pour obtenir le composé de formule (I):

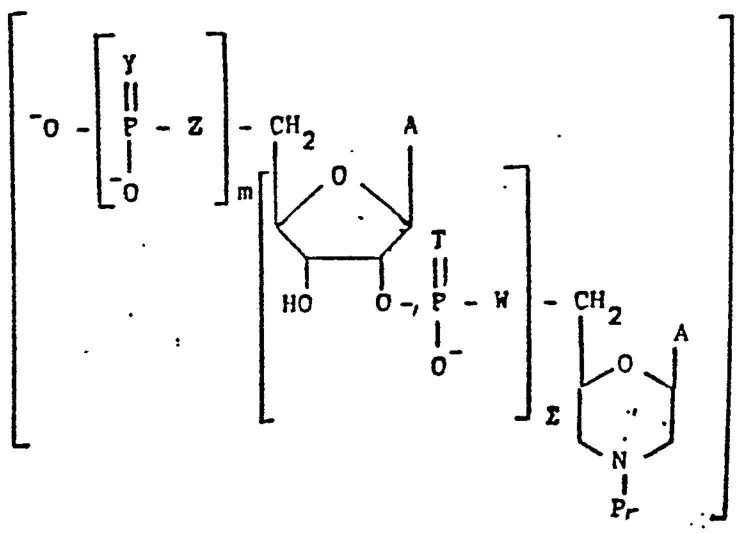

(I)

- le fractionnement entre les composés de couplage selon l'invention et les produits non couplés, notamment par filtration moléculaire.

L'oxydation se fait notamment par le périodate, notamment périodate de sodium, dans des conditions de pH rigoureusement contrôlées pour éviter une bêta élimination. L'expression "conditions de pH rigoureusement contrôlées" signifie le maintien du milieu réactionnel à pH 4,0, à 0 - 4°C et dans l'obscurité.

L'alcoylation réductive du composé de formule (Iter) se fait par des méthodes classiques telles que celles decrites dans Khym J. C., Biochemistry, 1963, 2, 344 - 350; Gray C., Arch. Biochem. Biophys., 1974, 163, 426 - 428; Lee R. T. et Lee Y. C., Methods in Enzymol., 1982, 83, 289 - 295; Imai J., Johnson M., Torrence P., J. Biol. Chem., 1982, 21, 12739 - 12741.

On peut utiliser notamment le borohydrure de sodium, ou de préférence le cyanoborohydrure de sodium, pour effectuer l'alcoylation réductive du composé (Iter).

Le nombre de molécules d'oligonucléotides dépend des quantités respectives du composés (Iter) par rapport à la quantité du polypeptide.

En ce qui concerne la synthese chimique des composés de formule (Ibis), on peut avoir recours soit à une synthèse chimique totale, soit à une synthèse enzymatique suivie de modifications chimiques.

En ce qui concerne la synthèse chimique, on peut se reporter au protocole décrit dans Methods of Enzymology, 79, 1981, 233 - 234.

En ce qui concerne la synthèse enzymatique des composé; de formule (Ibis), elle comprend:

- la polymérisation du composé de formule (Iquater) suivante:

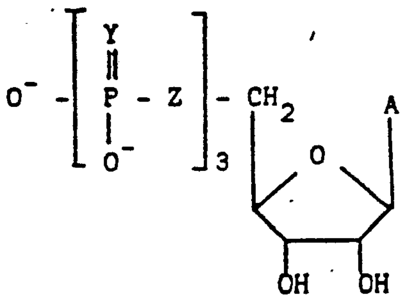

(Iquater)

dans laquelle:
- Y représente O, S, Se ou NH;
- Z représente O, S ou NH;
- l'un au moins des éléments Y et Z pouvant être différent de l'oxygène;

m est supérieur ou égal à 3;
- A a les significations indiquées ci-dessus;

pour obtenir un composé de formule suivante (Ibis):

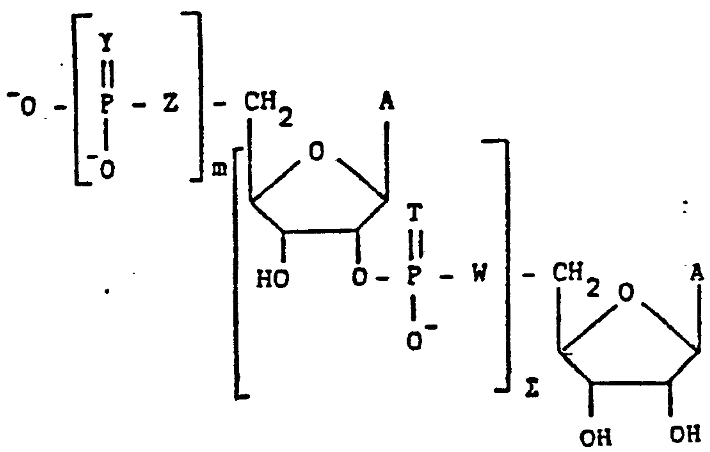

(Ibis)

dans laquelle:
- Y et T, identiques ou différents, représentent O, S, Se, NH;
- Z et W, identiques ou différents, représentent O, S, NH;

l'un au moins des éléments Y et Z pouvant être différent de l'oxygène;
- Σ est un nombre entier égal à n - 1, n étant supérieur ou égal à 3;
- m est un nombre entier supérieur ou égal à 1;
- A a les significations indiquées ci-dessus.

C'est ainsi que pour obtenir l'oligonucléotide de formule suivante (XVI):

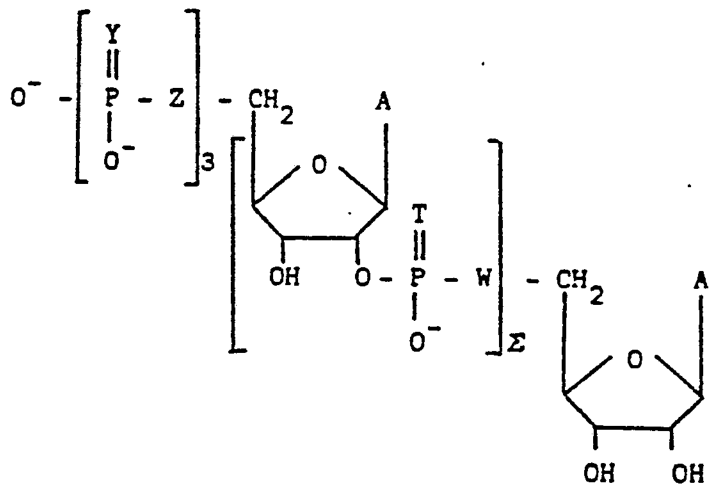

(XVI)

dans laquelle:
- Y et T, identiques ou différents, représentent O, S, Se, NH;
- Z et W, identiques ou différents, représentent O, S, NH;
- Y et Z ne représentent pas simultanément l'oxygène;
- $\Sigma$ est un nombre entier variant de 2 à 9;
- A est une base choisie parmi l'adénine ou ses dérivés, notamment ceux de formule:

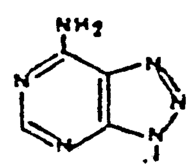
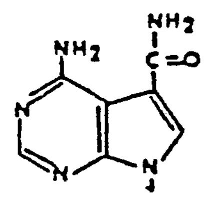
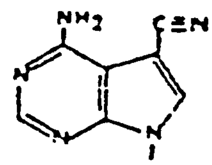
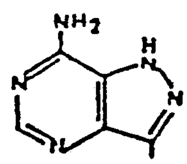
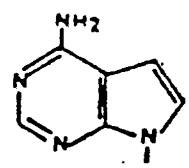
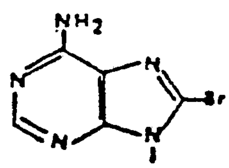

on effectue les étapes suivantes:
1. la polymérisation d'un composé de formule suivante (XVIbis):

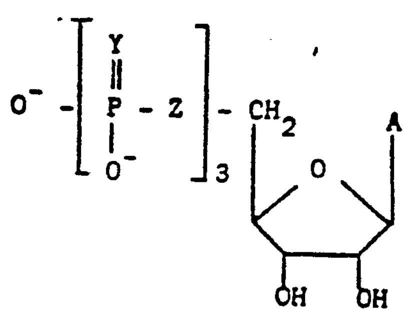

(XVIbis)

dans laquelle:
- Y représente O, S, Se ou NH;
- Z représente O, S ou NH;
- Y et Z ne représentent pas simultanément l'oxygène;
- A a les significations indiquées ci-dessus.

Parmi les composés de formule sont disponibles dans le commerce, ceux de formule suivante (XVIbis):

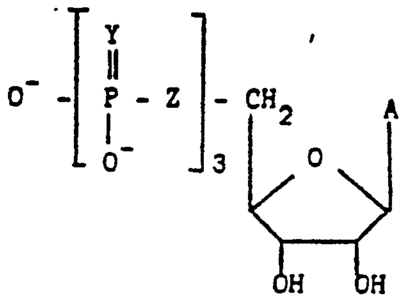

(XVIbis)

dans lesquels:
- Y représente le soufre et
- Z représente l'oxygène, ainsi que ceux dans lesquels:
- Y représente l'oxygene et
- Z représente le groupe NH,
- A représentant l'adénine ou un de ses dérivés comme définis ci-dessus.

Un procédé d'obtention des oligonucléotides de formule suivante (XVII):

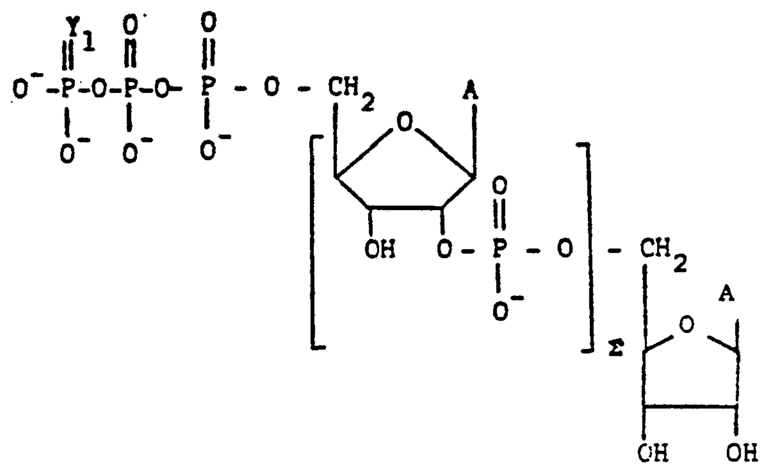

(XVII)

dans laquelle:
- $Y_1$ représente NH, Se, S;
- Σ est un nombre entier égal à n - 1, n variant de 3 à 10;
comprend la polymérisation du composé de formule suivante (XVIIbis):

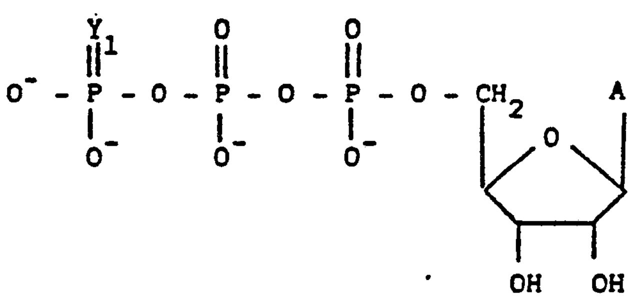

(XVIIbis)

Un procédé d'obtention des oligonucléotides de formule suivante (XVIII):

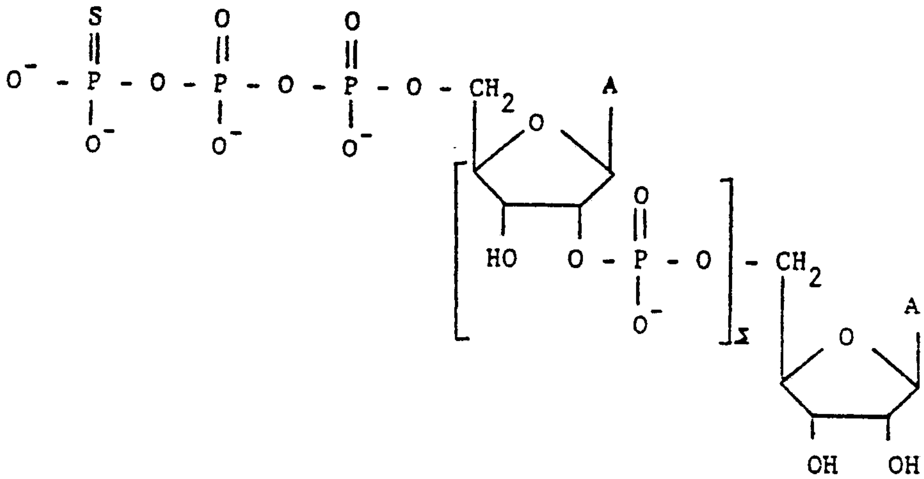

(XVIII)

dans laquelle:

- Σ a les significations précédemment indiquées;
- A a la signification précédemment indiquée, et de préférence représente l'adénine;

comprend la polymérisation d'un composé de formule suivante (XVIIIbis):

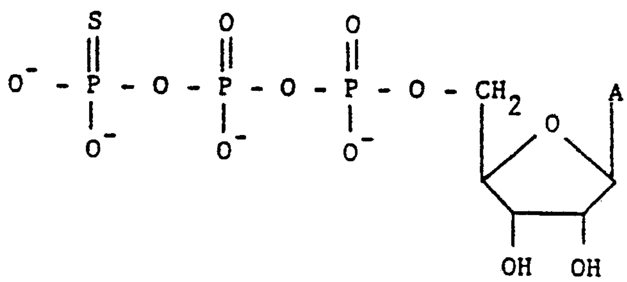

(XVIIIbis)

Un procedé d'obtention des oligonucléotides de formule suivante (XIX):

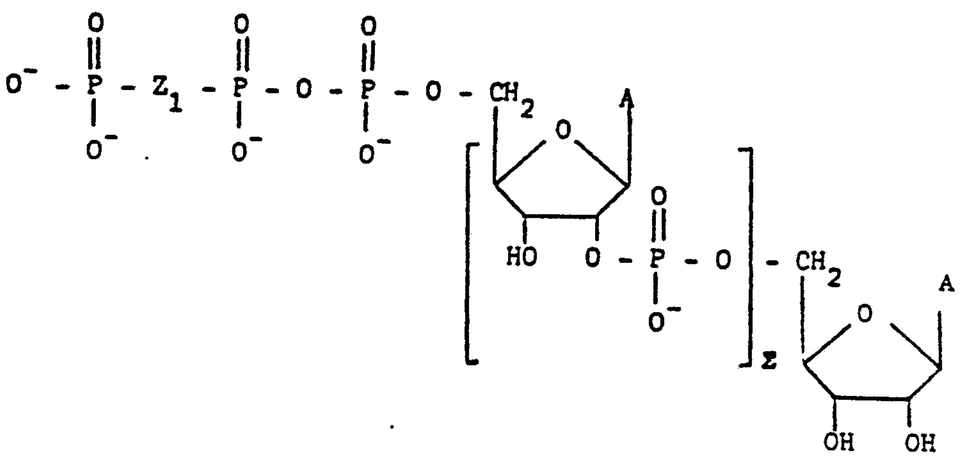

(XIX)

dans laquelle:

- $Z_1$ représente NH ou S;

Σ est un nombre entier égal à n - 1, n variant de 3 à 10;
comprend la polymérisation du composé de formule suivante (XIXbis):

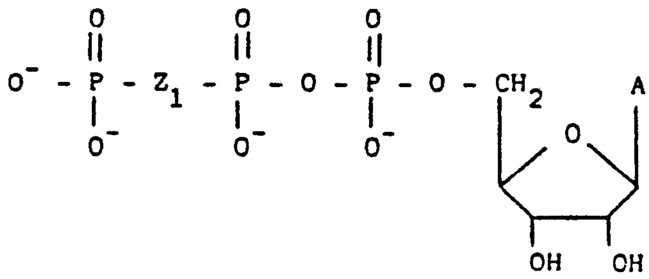

(XIXbis)

dans laquelle:

$Z_1$ représente S ou NH et

A a la signification indiquée ci-dessus.

Un procédé d'obtention d'oligonucléotides de formule suivante (XX):

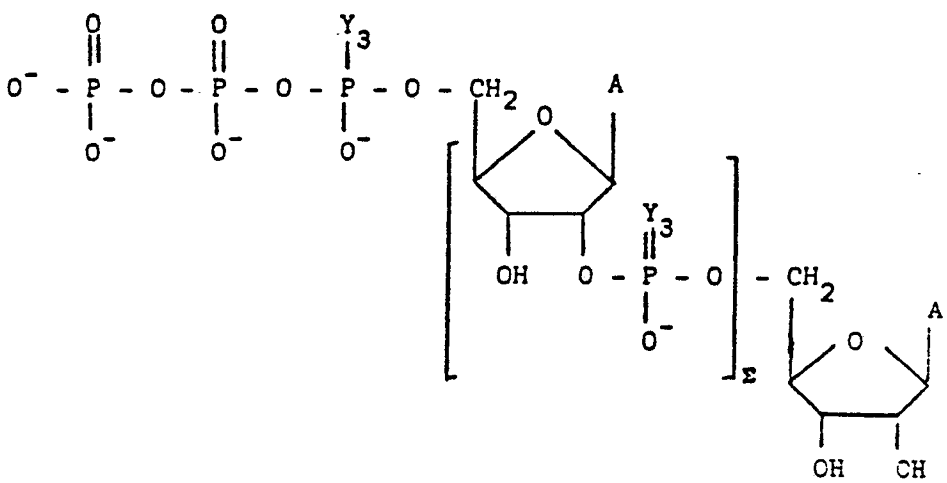

(XX)

dans laquelle:

- $Y_3$ représente NH, S ou Se;
- Σ et A ont les significations indiquées ci-dessus;

comprend la polymérisation du composé de formule suivante (XXbis):

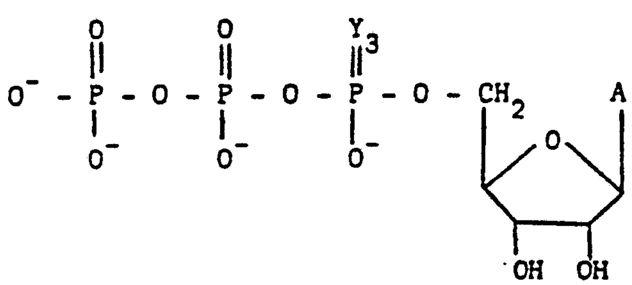

(XXbis)

dans laquelle:

- $Y_3$ représente S, Se, NH;
- A a les significations indiquées ci-dessus.

**Exemple 1**

Cet exemple concerne la préparation d'oligonucléotides (2'-5')$(A)_n$ synthétisés par voie enzymatique et modifiés chimiquement, que l'on désigne ci-après par γS-(2'-5')$A_n$ et qui peut être représenté par la formule suivante:

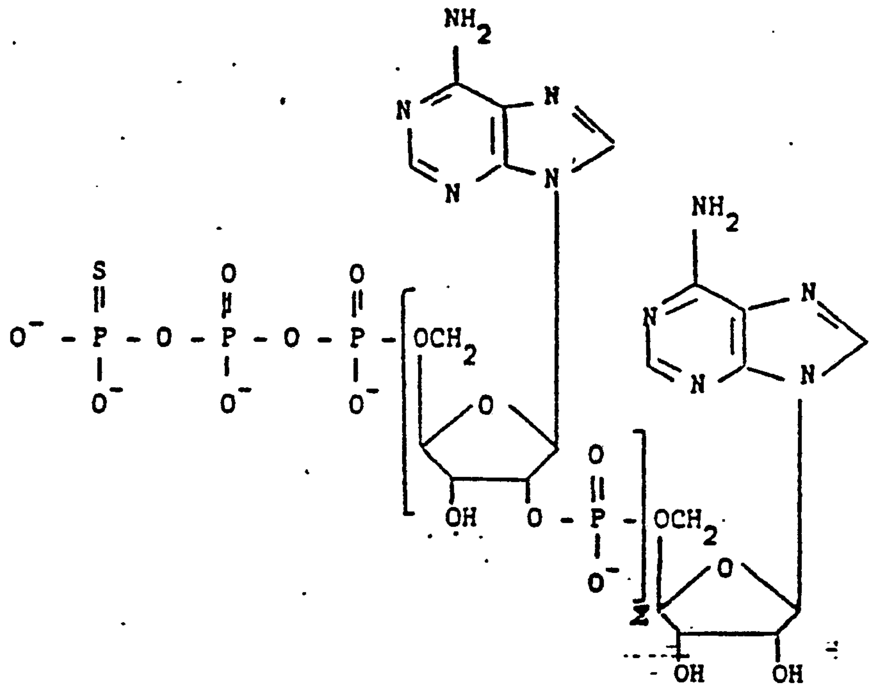

dans laquelle Σ est un nombre entier égal ou supérieur à 2.

Le produit de départ est l'adénosine 5'-0-(3-thiotriphosphate) (ci-après nommé γS ATP) de formule:

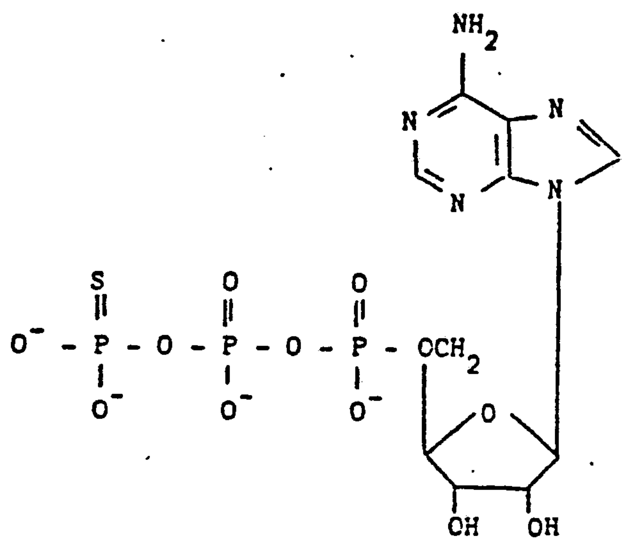

que l'on polymérise par exemple par voie enzymatique à l'aide d'une préparation partiellement purifiée de 2-5A synthétase, pour obtenir le composé de formule ci-dessus désignée par γS-(2'-5')$(A)_n$.

**Exemple 2**

Cet exemple concerne la préparation du composé de couplage entre un composé de formule ci-après représenté et désigné par (2'-5')$(A)_n$ et la polylysine pour donner le composé de formule:

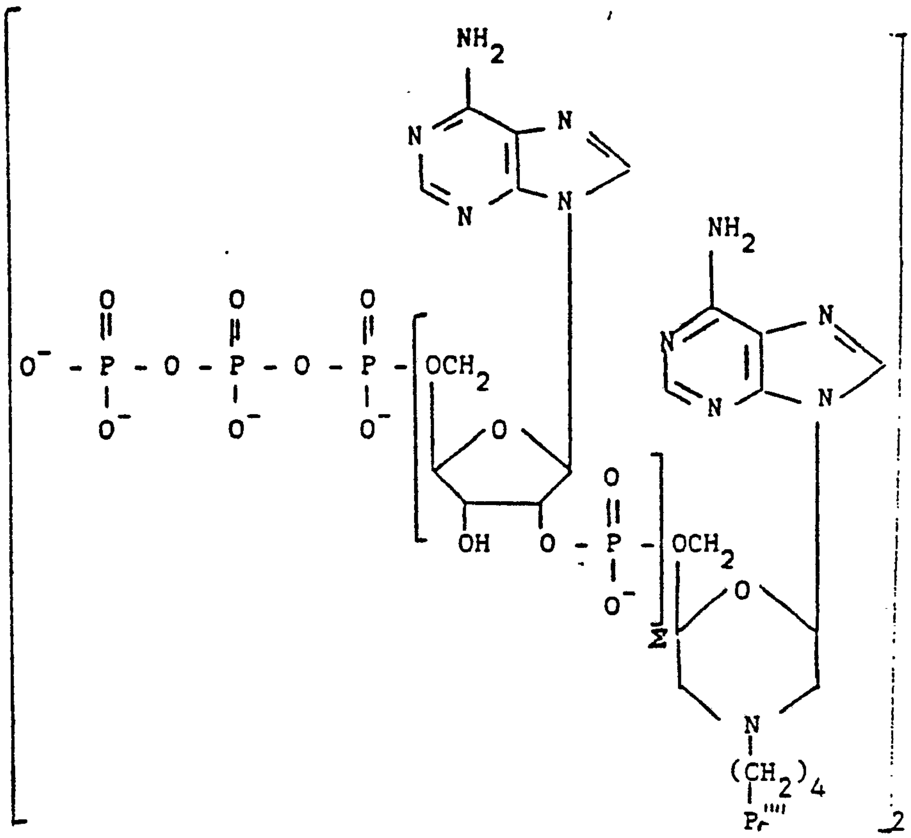

dans laquelle Σ est supérieur ou égal à 2 et Pr'''' représente la polylysine.

Ce compose de couplage est obtenu à partir du composé (2'-5')$(A)_n$ de formule suivante:

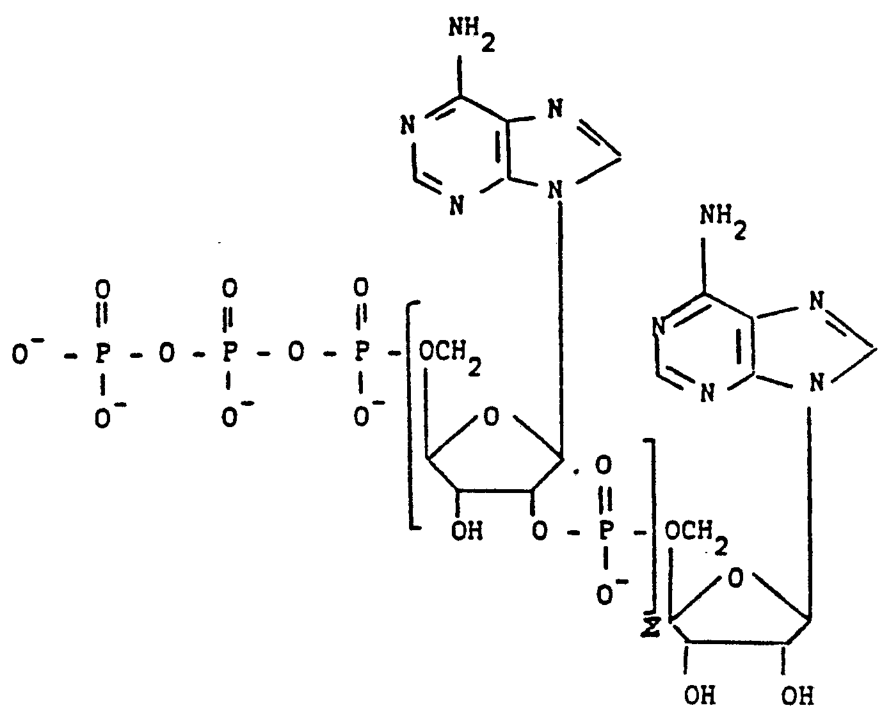

et de la polylysine.

La réaction se fait entre les fonctions aldéhyde obtenue à partir de l'oxydation de la dernière unité nucléosidique du compose representé ci-dessus et les groupes $NH_2$ en epsilon des résidus de la lysine.

On fait agir 0,6 µmoles de $(2'-5')(A)_n$ dans 400 µl sur 0,6 µmoles de $IO_4Na$ dans 4 µl de tampon acétate de sodium 0,1 M à pH 4,75.

La réaction d'oxydation est effectuée à l'abri de la lumière, à 0 - 4°C, pendant une heure.

Dans le produit obtenu, il y a eu oxydation à 100 % du glycol du $(2'-5')(A)_n$.

On effectue ensuite l'alcoylation réductive par le cyanoborohydrure de sodium.

Pour ce faire, on additionne au produit qui vient d'être obtenu ci-dessus 300 µl de cyanoborohydrure de sodium (2 µmoles) dans du tampon phosphate de sodium 0,2 M, à pH 8,0, ainsi que 400 µl de polylysine de poids moléculaire moyen de 14 000 (0,14 µmoles). La réaction se déroule à 20 - 22°C pendant environ une heure.

On purifie ensuite par exemple sur une colonne de gel commercialisé sous le nom de Sephadex G-25. On repère le composé de couplage de l'invention avec la méthode de dosage de Lowry à une densité optique de 260 nm.

Le composé de couplage obtenu contient deux molecules de $(2'-5')A_n$ pour une molécule de polylysine.

Le couplage entre les molécules de $(2'-5')(A)_n$ et la polylysine peut se faire par tout autre méthode de couplage connue, telle que celle décrite à propos du couplage entre le méthotrexate et la polylysine dans Proc. Nat. Acad. Sci. USA, vol. 75, n° 8, p. 3867 - 3870 August, 1978, Cell Biology.

**Exemple 3**

Cet exemple concerne la préparation d'un composé de couplage entre un composé $(2'-5')(A)_n$ et une asialoglycoprotéine.

On rappelle que les glycoprotéines comportent des séquences latérales dont l'extrémité est généralement constituée par une galactose et un acide sialique terminal.

Par traitement approprie, on peut eliminer l'acide sialique terminal, obtenant ainsi une asialoglycoprotéine comportant des séquences latérales dont les extrémités sont des galactoses.

Ces asialoglycoprotéines sont internalisées très efficacement par les hépatocytes après interaction spécifique avec les récepteurs au galactose sur les membranes des cellules d'hépatocyte.

Cet exemple concerne plus particulièrement le couplage entre l'asialofétuine et un composé de $(2'-5')(A)_n$.

Le composé de couplage est obtenu de la façon suivante.

L'asialofétuine est disponible dans le commerce, distribuée notamment par la Société SIGMA.

On a prealablement transformé les groupes COOH des résidus aspartyle et glutamyle de l'asialofétuine en groupe

```
C-NH-NH2,
||
O
```

à l'aide d'hydrazine, pour augmenter le nombre de groupes $NH_2$ disponibles.

L'asialofétuine ainsi modifiée est appelée asialofétuine hydrazide.

Pour ce faire, on ajoute 7 µl d'hydrazine pure distillée à 40 mg d'asialofétuine dans 400 µl d'eau, le pH de la solution ayant été ajusté à 4,75 à l'aide d'acide, notamment d'HCl 0,1 N.

On verse sur cette solution de la carbodiimide (62 mg dans 100 µl d'eau) goutte à goutte, et on ajuste constamment le pH à 4,75 avec HCl 1N; la carbodiimide est utilisée comme catalyseur pour fixer l'hydrazine sur le carboxyle de l'aspartyle et du glutamyle et pour obtenir des aspertate hydrazide et des glutamate hydrazide.

Le composé obtenu est de l'asialofétuine hydrazide que l'on purifie par gel filtration sur une colonne de Sephadex G-50, tampon acétate de sodium 0,1 M, pH 4,75.

A la sortie de la colonne, on récupère l'asialoétuine hydrazide, et on effectue le couplage avec le composé de $(2'-5')(A)_n$.

Pour ce faire, on ajoute 1 µmole de $(2'-5')A_n$ ont le dernier nucléoside a été préalablement oxydé par 2 µl d'acide périodique dans les conditions suivantes: solution à 21,4 mg/ml, tampon acétate de sodium 0,1 M, H 4,75, à l'obscurité, pendant une heure à 4°C.

Après la réaction de couplage, on passe le composé obtenu sur une colonne de gel filtration G-50 pour éliminer le $(2'-5')(A)_n$ non fixé à l'asialofétuine.

Le composé de couplage obtenu comporte deux molécules de $(2'-5')A_n$ pour une molécule asialofétuine.

D'autres composés de couplage peuvent être obtenus entre notamment à partir d'un composé $(2'-5')A_n$ de formule indiquée ci-dessus et de tout polypeptide naturel ou synthétique susceptible d'interagir avec les récepteurs de la surface cellulaire, par l'intermédiaire de leurs groupes $NH_2$ situes à l'extremite de sequences latérales et/ou des groupes COOH modifiés par l'hydrazine.

On peut également obtenir des composés de couplage entre un composé de $(2'-5')(A)_n$ et une néoglycoprotéine, telle qu'une néoglycoprotéine à mannose, par exemple le mannose-polylysine.

Le composé de couplage obtenu est susceptible d'être internalisé spécifiquement par les cellules possédant des récepteurs au mannose, à savoir les macrophages.

Les composes de couplage de ce type permettent ainsi de cibler le $(2'-5')A_n$ sur les macrophages.

On peut également obtenir des composés de couplage entre un composé de (2'-5')$A_n$ et des anticorps (mono ou polyclonaux) dirigés contre des antigénes de la surface cellulaire.

**Activité pharmacologique**

1. Matériaux

Les milieux proviennent d'Eurobio (Paris) et les sérums des Laboratoires Flow.

2. Cellules et virus

On maintient des cellules leucémiques murines L1210 en suspension dans un milieu commercialisé sous la désignation RPMI 1640, notamment par les Laboratoires Eurobio, Paris, complété par du sérum de veau foetal à 10 % (v/v), 50 UI/ml de pénicilline et 50 µg/ml de streptomycine. On fait croître des cellules L929 dans un milieu minimal essentiel additionné de 5 % (v/v) de sérum de cheval donneur, de 3 g/l de bouillon de phosphate bacto-tryptose, 3,4 g/l de glucose et d'antibiotiques comme mentionné ci-dessus. On utilise la souche Indiana du virus de stomatite vésiculaire (VSV) et on la fait pousser dans des cellules L929.

2. Introduction du composé de couplage dans les cellules.

On laisse incuber les cellules avec les composés de couplage selon l'invention.

3. Essai de l'activité antivirale

Les cellules sont infectées à des temps indiqués, avec du virus de stomatite vésiculaire (VSV) à une multiplicité de 10 pendant une heure à 37°C dans un milieu RPMI 1640 additionné de sérum de veau foetal 5 % (v/v). Les virus non adsorbés sont soigneusement éliminés par trois lavages avec du RPMI contenant du sérum de veau foetal à 10 % (v/v).

On détermine le titre de virus produit 18 heures plus tard selon les méthodes connues (Stewart W. E., 1970, J. Virol., 6, 795 - 799). Pour résumer $10^6$ cellules L929 sont mises dans des boîtes de Petri destinées à la culture de tissus (2 cm de diamètre). 24 heures après l'incubation, on étend 0,05 ml des suspensions de virus diluée (facteur de dilution 50) soigneusement sur la monocouche des cellules. Une heure plus tard, la suspension virale est éliminée par succion et on étend 2 ml d'agarose fondu à 1,6 % (v/v) dans un milieu essentiel minimum complété par du sérum de veau foetal à 2 % (v/v) sur la monocouche des cellules. Les plaques sont incubées pendant 18 heures dans un incubateur à $CO_2$. Les plaques sont ensuite révélées par une solution à 1 % (v/v) de rouge neutre dans une solution saline tamponnée isotonique de phosphate.

**Résultats concernant la synthèse des protéines**

On a déterminé l'effet de l'inhibition de la synthèse des protéines cellulaires par des composés de couplage selon l'invention (en fonction de la dose) après incorporation de méthionine $^{35}S$ dans les cellules L1210, servant à repérer les protéines synthétisées.

On a aussi comparé les effets dose réponse relatifs aux composés suivants:

- mélange de deux composés non couplés entre eux: polylysine et de (2'-5')$A_4$Ox Red de formule suivante:

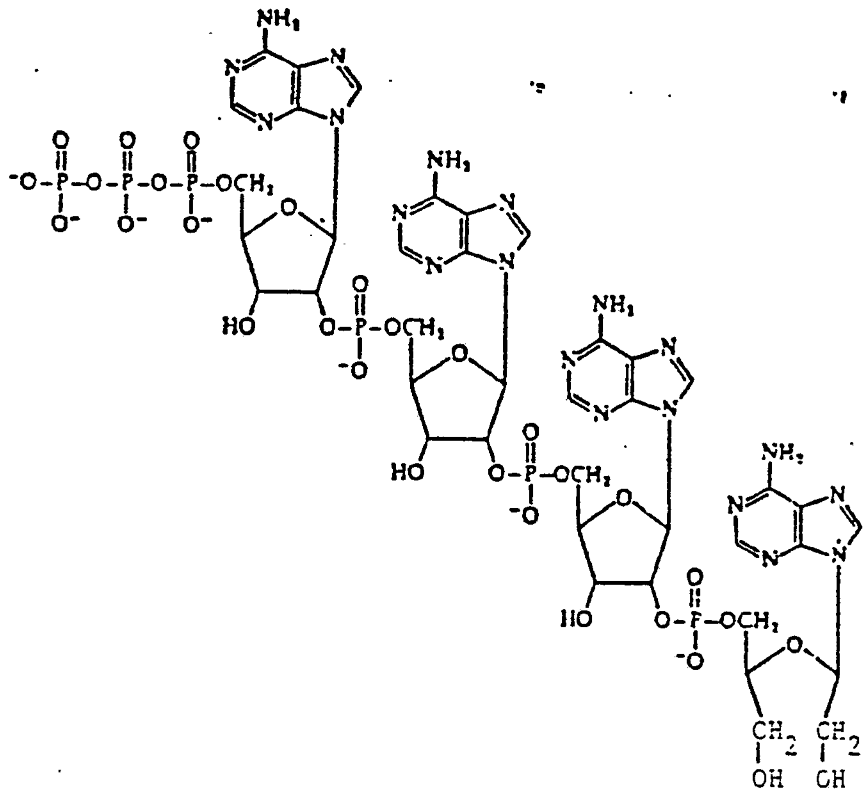

- (2'-5')$A_3$-polylysine selon l'invention (deux molécules de (2'-5')$A_3$ par molécule de polylysine);

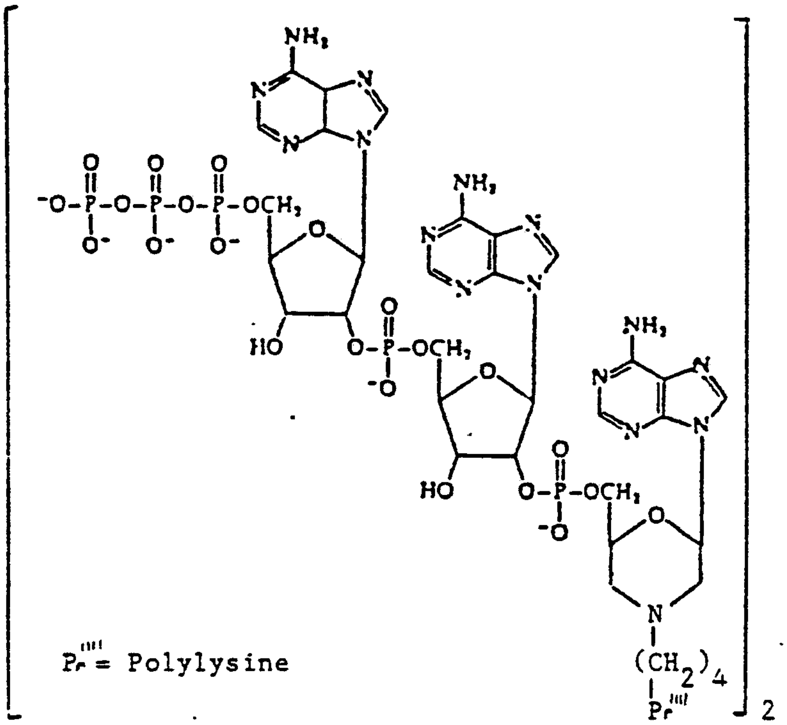

- (2'-5')$A_4$-polylysine selon l'invention (deux molécules de (2'-5')$A_4$ par molécule de lysine)

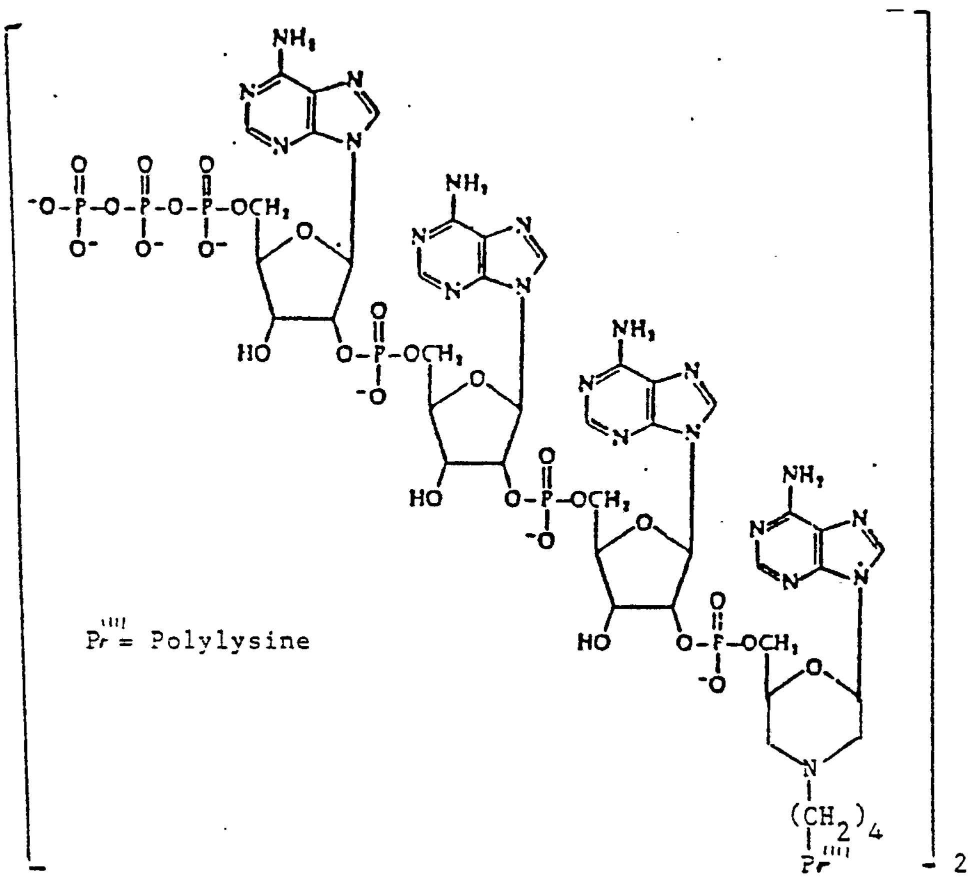

Pr = Polylysine

Pour ce faire, les cellules sont incubées en présence d'un mélange de polylysine et de (2'-5')$A_4$Ox Red non couplé, du composé (2'-5')$A_4$-polylysine selon l'invention et du composé (2'-5')$A_3$-polylysine.

L'incorporation de méthionine $^{35}S$ est suivie par le dosage de la radioactivité incorporée après un marquage de 30 minutes.

Ces résultats apparaissent sur la figure 1 sur laquelle on a représenté en abscisses la quantité en nM de composés testés et en ordonnées, exprimée en $10^{-4}$ cpm la quantité de protéine synthétisée.

Le melange de polylysine et de (2'-5')$A_4$Ox Red non couplé est représenté par la courbe en pointillés qui porte les ronds noirs.

Le composé de couplage (2'-5')$A_4$-polylysine est représenté par la courbe en traits pleins qui porte les ronds blancs.

Le compose de couplage (2'-(4)$A_3$-polylysine est représenté par la courbe en traits pleins qui porte les triangles noirs.

On constate que le composé (2'-5')$A_4$Ox Red ne modifie sensiblement pas la synthèse des protéines dans les cellules L1210, tandis que les deux composes de couplage selon l'invention sont actifs vis-à-vis de l'inhibition de la synthèse de protéines.

**Activité antivirale des composés de couplage (2'-5')Anpolylysine selon l'invention**

Pour tester l'activité biologique des composés de couplage conformes à l'invention, dans les cellules intactes, on fait incuber les cellules avec les composés de couplage selon l'invention ainsi qu'avec des composés non couplés, pour faire une étude comparative.

Le tableau I ci-après est relatif à l'activité antivirale:

- du composé de couplage (2'-5')$A_4$-polylysine dont la formule a été représentée ci-dessus;
- du composé de couplage entre le (2'-5')$A_4$ déphosphorylé et la polylysine, ci-après dénommée "core (2'-5')$A_4$-polylysine" de formule:

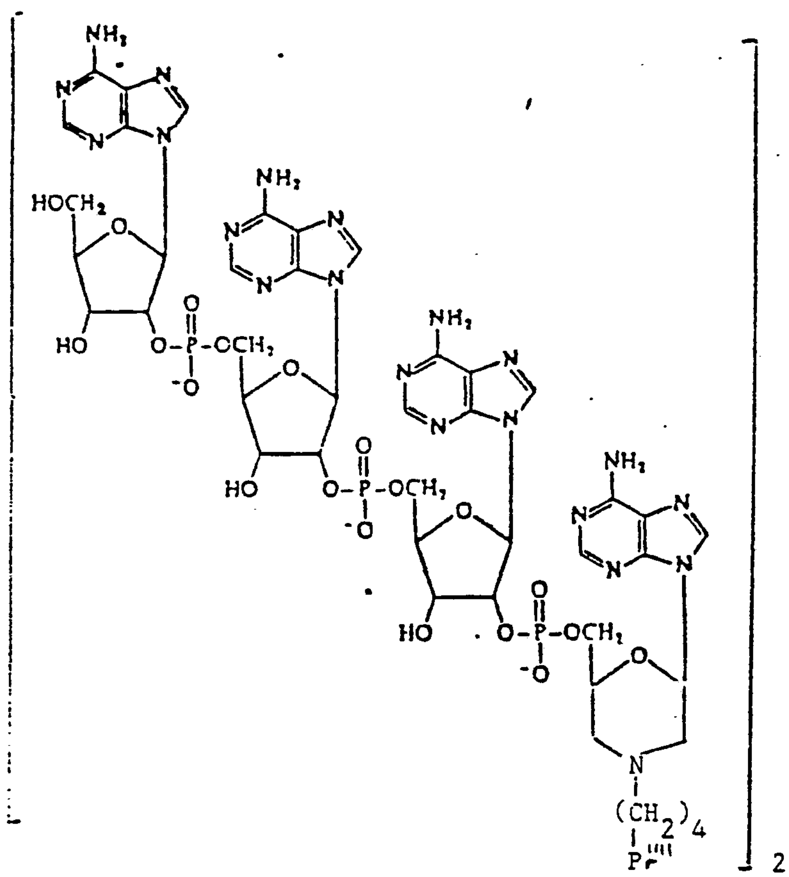

- du mélange de deux composés suivants: (2'-5')$A_4$ et polylysine;
- du mélange de deux composés suivants: (2'-5')$A_4$Ox Red dont la formule a été ci-dessus représentée et polylysine.

**Tableau I**

Effet antiviral de (2'-5')$(A)_n$ couplé à la polylysine comparé à des mélanges de polylysine et de (2'-5')$(A)_n$ non couple

| Traitement des cellules avec | Concentration en polylysine (nM) | Titre en VSV (% du contrôle) |
|---|---|---|
| Poly lysine | 500 | 100 |
| Poly lysine + (2'-5')$A_n$Ox Red | 500 | 100 |
| Poly lysine + (2'-5')$A_4$ | 500 | 100 |
| Core (2'-5')$A_4$ poly lysine | 500 | 19 |
| (2'-5')$A_4$ poly lysine | 500 | 0,01 |
| (2'-5')$A_4$ poly lysine | 180 | 0,6 |
| (2'-5')$A_4$ poly lysine | 100 | 5,0 |
| (2'-5')$A_4$ poly lysine | 36 | 100 |

Les essais dont les résultats ont été rassemblés dans le tableau I ont été effectués comme suit.

Des cellules L1210 en suspension sont incubées avec les differents composés aux concentrations indiquées dans le tableau.

Une heure après, les cellules sont infestées avec du virus de stomatite vésiculaire (multiplicité de l'infection = 10) et le rendement du virus est déterminé 18 heures après, par essai de plaques dans des cellules L929.

Comme le montre le tableau I, le mélange de polylysine et de composé (2'-5')$A_4$ non couplé ainsi que le mélange de polylysine et de composé (2'-5')$A_4$Ox Red non couplé n'affectent pas la production de virus de stomatite vésiculaire.

Au contraire, à partir d'une concentration de 100 nM de composé de couplage (2'-5')$A_4$-polylysine de l'invention (qui comporte deux molécules de (2'-5')$A_4$ pour une molécule de polylysine), la croissance du virus est reduite de 20 fois, environ 200 fois, 10 000 fois pour des concentrations respectives de 100, 180 et 500 nM de composé de couplage (2'-5')$A_4$-polylysine.

**Exemple 4**

Cet exemple concerne la préparation d'un composé de couplage entre un composé $(2'-5')(A)_n$ et l'asialofétuine hydrazide.

Les modifications chimiques sont celles décrites à l'exemple 3 et les conditions expérimentales sont celles décrites dans la partie intitulée "activité pharmacologique" avec la modification suivante:

- Les cellules L1210 sont remplacées par les hépatocytes tranformés, lignées de cellules répondant au nom de Hep G2 caractérisées par B. Knowles du Wistar Institute, Philadelphie. Elles sont cultivées dans un milieu essentiel minimum tel que défini ci-dessus et complémenté par 10 % (v/v) de sérum de veau foetal.

**Résultats concernant la synthèse des protéines**

Le compose de couplage possède un effet d'inhibition de la synthèse des protéines. Pour ce faire, les cellules sont incubées en présence de (2'5')A-asialofétuine hydrazide (rapport molaire 5 : 1) à suivie par l'incorporation de méthionine [$^{35}$S] et le dosage de la radioactivité incorporée dans les protéines est estimé après marquage des cellules pendant 30 minutes.

Les resultats sont illustrés par la figure 2 sur laquelle on a représenté en ordonnée le pourcentage d'incorporation de la radioactivité (% du contrôle non traité), en abcisse les concentrations micromolaires en (2'-5')A couplé (exprimé en concentration d'asialofétuine hydrazide).

Le mélange asialofétuine hydrazide et de $(2'-5')A_4$Ox Red non couplé est représenté par la courbe en pointillé.

Le composé de couplage $(2'-5')A_4$-asialofétuine hydrazide est représenté par la courbe en trait plein.

On constate que les composés non couplés sont sans effet sur la synthèse des protéines alors que le composé résultant du couplage est actif vis-à-vis des l'inhibition de la synthèse des protéines.

**Activité antivirale du composé de couplage $(2'-5')A_4$-asialofétuine hydrazide**

Pour tester l'activité antivirale du composé de couplage dans les cellules Hep G2, on fait incuber les cellules avec le composé de couplage ainsi qu'avec les composés non couplés.

Le tableau II ci-après est relatif à l'activité antivirale de ces composés.

Dans le tableau II, on a également indiqué les résultats obtenus avec un mélange de composé (2'-5')A Ox Red et d'asialofétuine hydrazide (non couplé) dans le rapport molaire 5 : 1.

**Tableau II**

Effet antiviral de $(2'-5')(A)_n$ couplé à l'asialofétuine

| composés | concentration (μM) | titre de virus titre en VSV<br>VSV (unités infectieuses/ml) | (% du contrôle) |
|---|---|---|---|
| / | | 1.70 x $10^9$ | 100 |
| asialofétuine hydrazide +(2'-5')A Ox Red | 1 | 1.7 x $10^9$ | 100 |
| asialofétuine hydrazide +(2'-5')A Ox Red | 2 | 1.3 x $10^9$ | 76 |
| $(2'-5')A_4$-asialofétuine hydrazide | 0.5 | 4.2 x $10^8$ | 24 |
| | 1 | 4.2 x $10^8$ | 24 |
| | 2 | 7.8 x $10^5$ | 0.45 |
| | 3 | 2.3 x $10^4$ | 0.01 |

Les cellules Hep G2 en suspension sont incubées avec les composés couplés ou non couplés aux concentrations indiquées dans le tableau II.

Une heure après, les cellules sont infectées avec le virus de la stomatite vésiculaire (VSV) et le rendement en virus est déterminé 18 heures après par essai de plaques dans les cellules L929.

Comme l'illustre le tableau II, le melange d'asialofétuine hydrazide et le composé (2'-5')$A_4$Ox Red non couplé n'affectent pas la production du virus et les cellules meurent par infection virale après 20 heures.

En effet, la différence de résultats obtenus avec $10^{-6}$ M de mélange d'asialofétuine hydrazide et de (2'-5')A Ox Red et 2 x $10^{-6}$ M de mélange l'asialofétuine hydrazide et de (2'-5')A Ox Red n'est pas significative.

Par contre, les concentrations de 2 et 3 micromoles de composé de couplage (2'-5')$A_4$-asialofétuine hydrazide protégent complétement les cellules qui restent viables au-delà de 20 heures d'incubation.

Le compose de couplage est sans effet sur d'autres types cellulaires comme les L1210 ou L929 qui ne posèdent pas les récepteurs appropriés. Ce qui démontre bien que ce produit ne peut être que ciblé sur des cellules portant à leur surface le récepteur membranaire approprié comme les hépatocytes.

Ce composé est capable d'activer l'endoribonucléase L et de dégrader l'ARN viral en empéchant ainsi le virus de se développer. Le choix de la molécule de transport ou vecteur permet de véhiculer la molécule active le (2'-5')$A_n$ très spécifiquement sur un seul type cellulaire d'hépatocyte.

## Conclusion

Les composés de couplage selon l'invention obtenus à partir du couplage de (2'-5')$A_n$, naturel ou modifie et d'un polypeptide porteur approprie, sont des composés actifs vis-à-vis de l'endoribonucléase L, qui peuvent être internalisés par la cellule et présentent une activité antivirale efficace dans les cellules.

L'invention concerne également les sels que les composes de couplage ci-dessus peuvent former avec les bases en particulier les base minérales ou organiques. Parmi les sels de base minérale, on préfère les sels de sodium et de potassium. Parmi les sels organiques, on préfère les sels d'amine, d'alcoylamine et d'arylamine, en particulier ceux d'amines secondaires, tels que la diéthylamine, la pipérazine, ou d'amines tertiaires, tels que la méthylamine, la pyridine, la méthylpipérazine etc. Parmi tous ceux-ci, les sels physiologiquement acceptables sont préférés. Les sels peuvent être lyophilisés. Les composés selon l'invention ont des propriétés biologiquement intéressantes, en particulier des propriétés du type de l'interféron, et plus particulièrement une activite antivirale.

Les composés selon l'invention sont capables d'activer l'endoribonucléase L, de dégrader l'ARN viral, en empéchant ainsi la synthèse des protéines virales dans les cellules infectées par un virus.

Les oligonucléotides selon l'invention sont donc des substituts appropriés de l'interféron et de ses applications connues. Ils peuvent être préparés reproductiblement sous forme hautement purifiée, comme réactifs biologiques, en particulier comme référence de comparaison dans les essais qualitatifs et quantitatifs, dans les cultures cellulaires, des composés de l'interféron ou d'autres substances semblables à l'interféron.

L'invention concerne également les sels pharmaceutiquement acceptables des composés de couplage définis ci-dessus en particulier ceux appropriés pour l'administration in vivo.

L'invention concerne également les compositions pharmaceutiques associant les susdits composés de couplage, de préférence sous la forme de sels pharmaceutiqiement acceptables, avec un véhicule pharmaceutique.

L'invention fournit ainsi des composition, pharmaceutiques ayant une activité semblable à celle de l'interféron en utilisant un composé chimique déterminé sous forme de haute pureté, ne présentant pas de toxicité, étant stable et facile à manipuler.

La composition selon l'invention peut être sous forme de préparations administrables par voie orale ou rectale, en utilisant des solides ou des liquides appropriés pour un tel type d'administration ou sous forme de préparations injectables stériles contenant au moins l'un quelconque des composés de couplage en association avec des véhicules liquides appropriés stériles, de préférence isotoniques.

D'autres formes appropriées de préparations consistant en pommades dans lesquelles les composés de couplage de l'invention sont associés avec des véhicules en pommade.

L'une quelconque des techniques de préparation classiquement utilisées pour associer l'interféron avec les supports pharmaceutioues peut être utilisée pour préparer les compositions pharmaceutiques selon l'invention.

Les compositions de l'invention ont des propriétés antivirales et sont en particulier susceptibles d'inhiver les maladies virales susceptibles d'être suivies de troubles tumoraux, par exemple les maladies induites par les virus de l'hépatite B ou les différentes formes de virus de l'herpès.

Plus généralement, les compositions de l'invention sont utiles pour le traitement et la prévention de maladies virales, et pour les traitements antitumoraux vis-à-vis des tumeurs susceptibles d'être également délimitées par les traitements à l'interféron.

On notera que les doses auxquelles les compositions sont utilisées sont déterminées selon la nature de la maladie qui afflige le patient et les conditions particulières de santé.

Les dosages appropriés sont déterminés par le médecin, comme la pratique l'exige dans ces domaines d'application.

**Revendications** pour les états contractants: BE, CH, DE, FR, GB, IT, LI, LU, NL, SE

1. Composes de couplage representes par la formule (I) définie ci-dessous:

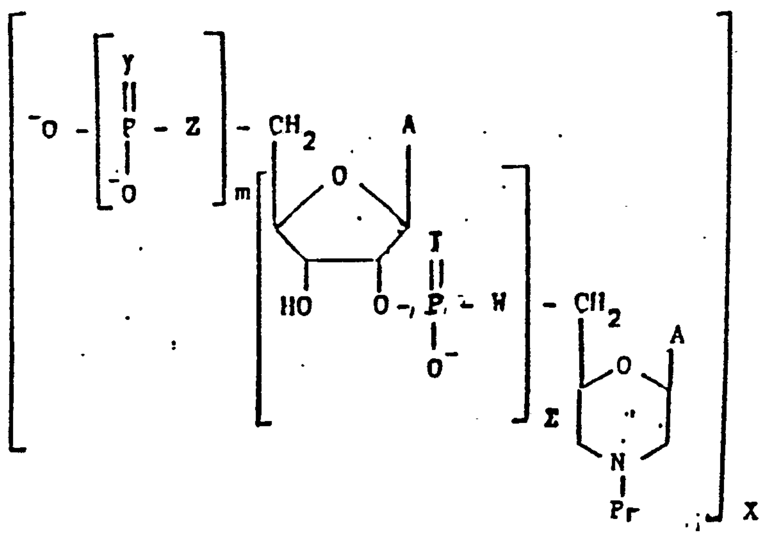

(I)

dans laquelle:
- Y et T, identiques ou différents, représentent indépendamment l'un de l'autre O, S, Se ou NH;
- Z et W, identiques ou différents, représentent indépendamment l'un de l'autre O, S ou NH;
- A représente l'adénine ou l'un de ses dérivés, notamment ceux de formule:

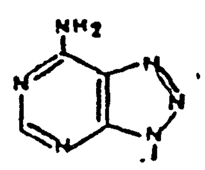
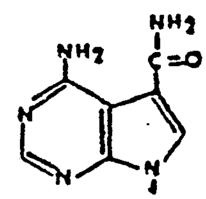
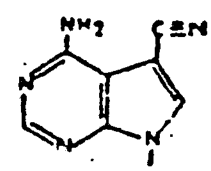
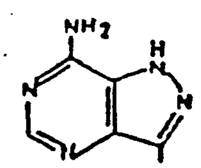
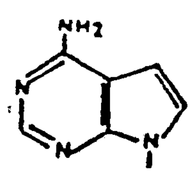
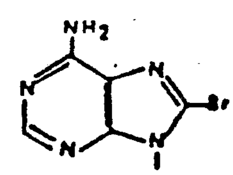

- Σ est un nombre entier égal à n - 1, n étant un nombre entier supérieur ou égal à 3;
- m est un nombre entier supérieur ou égal à 0 et de préference supérieur ou égal à 2;
- X est un nombre entier supérieur ou égal à 1, de préference compris de 2 à 10;
- Pr est un enchaînement polypeptidique comportant au moins 5 acides aminés, et l'atome d'azote appartenant au cycle:

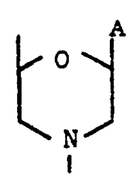

appartenant également à l'extrémité d'une séquence latérale du polypeptide Pr et/ou appartenant à un groupe

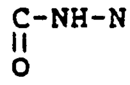

provenant d'un groupe COOH modifié par l'hydrazine.

2. Composés de couplage selon la revendication 1 de formule:

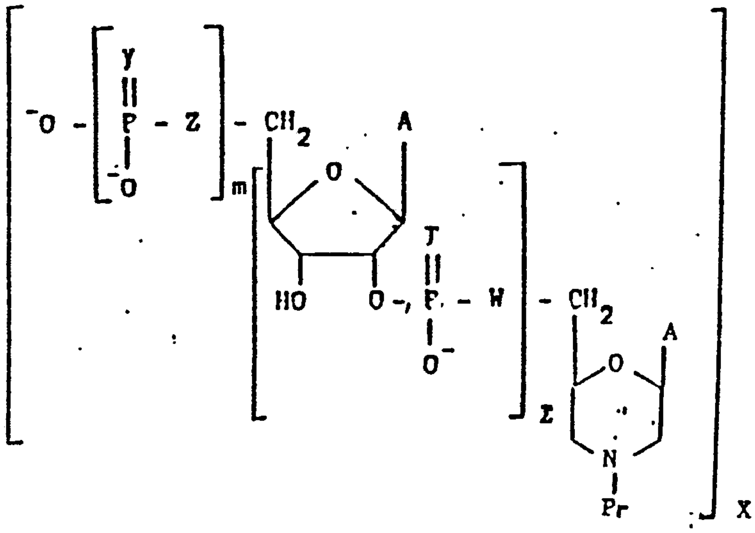

(I)

dans laquelle:

- Y et T, identiques ou différents, représentent indépendamment l'un de l'autre O, S, Se ou NH;
- Z et W, identiques ou différents, représentent indépendamment l'un de l'autre O, S ou NH;
- A représente l'adénine ou l'un de ses dérivés selon la revendication 1;
- Σ est un nombre entier égal à n - 1, n étant un nombre entier supérieur ou égal à 3;
- m est un nombre entier supérieur ou égal à 0 et de préférence supérieur ou égal à 2;
- X est un nombre entier supérieur ou égal à 1, de préférence compris de 2 à 10,
- Pr est un enchaînement polypeptidique comportant au moins 5 acides aminés, et l'atome d'azote appartenant au cycle;

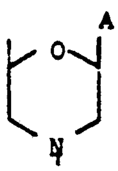

appartenant également à l'extrémité d'une séquence latérale du polypeptide Pr.

3. Composés de couplage selon la revendication 1 de formule (II):

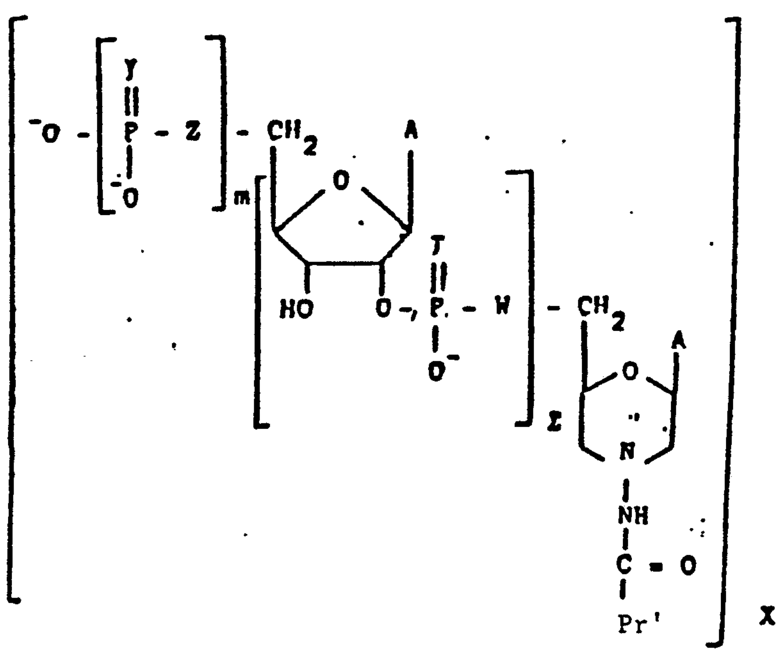

(II)

dans laquelle Y, Z, T, W, Σ, X, m et A ont les significations ci-dessus définies, Pr' représente un enchaînement polypeptidique comportant au moins 5 acides aminés, et dans lequel le groupe

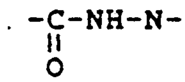

représenté sur la figure provient du groupe

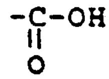

terminal du polypeptide et/ou d'un groupe

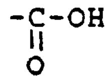

libre des résidus aspartyle et/ou glutamyle.

4. Composés de couplage selon la revendication 1 de formule (III):

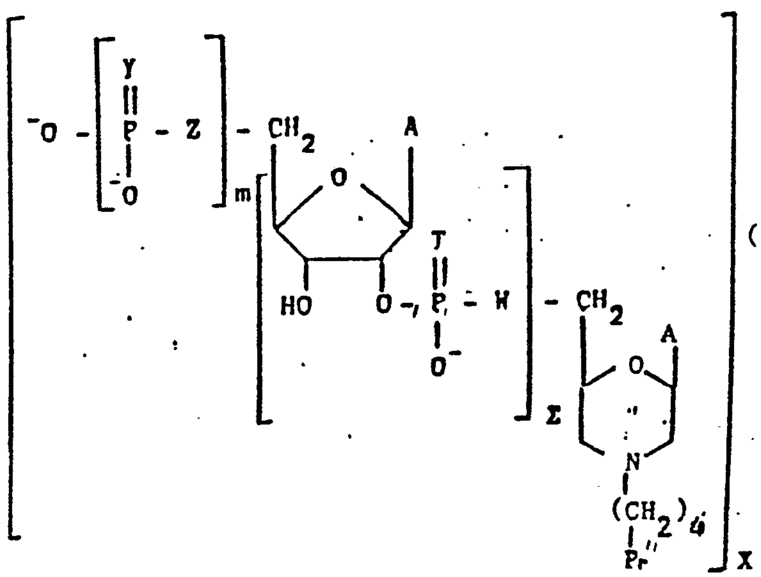

(III)

dans laquelle:

- Y et T identiques ou différents, représentent indépendamment l'un de l'autre O, S, Se ou NH;
- Z et W, identiques ou différents, représentent indépendamment l'un de l'autre O, S, ou NH;
- A représente l'adénine ou l'une de ses dérivés selon la revendication 1;
- Σ est un nombre entier égal à n - 1, n étant un nombre entier supérieur ou égal à 3;
- X est un nombre entier égal ou supérieur à 1, de préférence compris de 2 à 10;
- m est un nombre entier supérieur ou égal à 0 et de préférence supérieur ou égal à 2;
- Pr'' est un enchaînement polypeptidique comportant au moins 5 acides aminés et dans laquelle le groupe $-(CH_2)_4-$ représenté dans la formule appartient à la séquence latérale d'un résidu lysyle entrant dans la constitution de l'enchaînement polypeptidique Pr''.

5. Composés de couplage selon la revendication 1 de formule (IV):

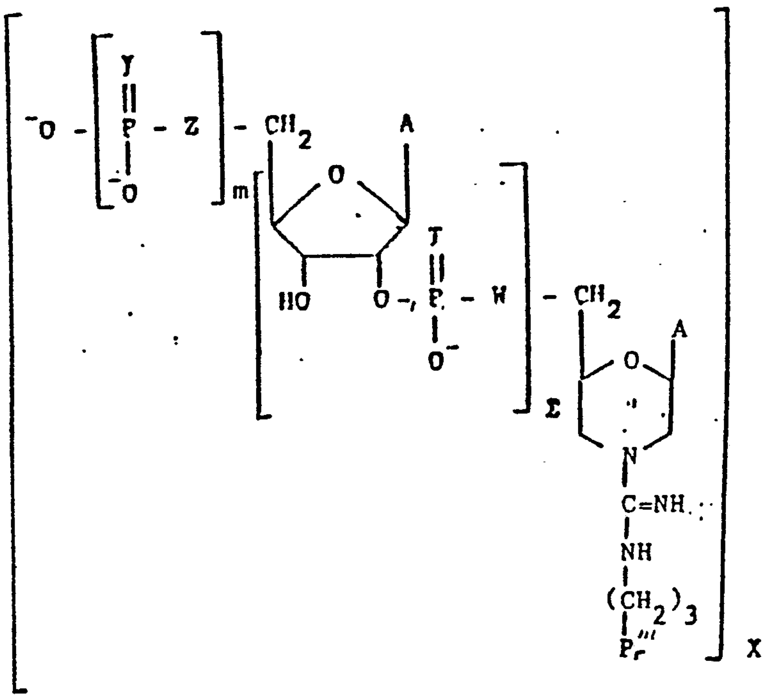

(IV)

dans laquelle Y, T, Z, W, A, Σ, n, X, m ont les significations indiquées à la revendication 1, Pr''' est un enchaînement peptidique comportant au moins 5 acides aminés et l'atome d'azote appartenant au cycle:

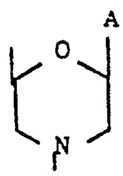

appartient également à l'extrémité de la séquence latérale d'un résidu d'arginyle entrant dans la constitution de l'enchaînement polypeptidique Pr''.

6. Composés de couplage selon la revendication 1 de formule (V):

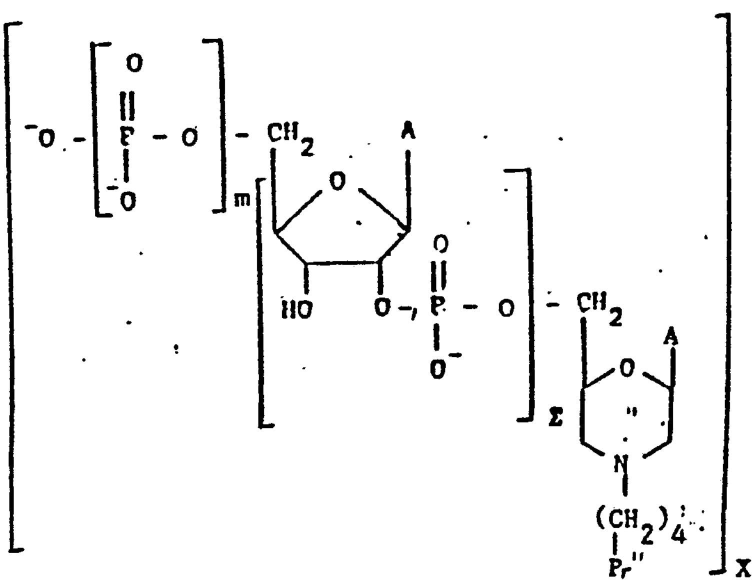

(V)

dans laquelle:
- A représente l'adénine ou l'un de ses dérivés selon la revendication 1;
- Σ est un nombre entier égal à n - 1, n étant un nombre entier supérieur ou égal à 3;
- m est un nombre entier supérieur ou égal à 0 et de préférence supérieur à 2;
- X est un nombre entier égal à 1 ou, de preference compris de 2 à 10;
- Pr'' est un enchaînement peptidique comportant au moins 5 acides aminés et dans laquelle le groupe $-(CH_2)_4-$ représenté dans la formule appartient à la séquence latérale d'un residu lysyle entrant dans la constitution de l'enchaînement polypeptidique Pr''.

7. Composés de couplage selon la revendication 1 de formule suivante:

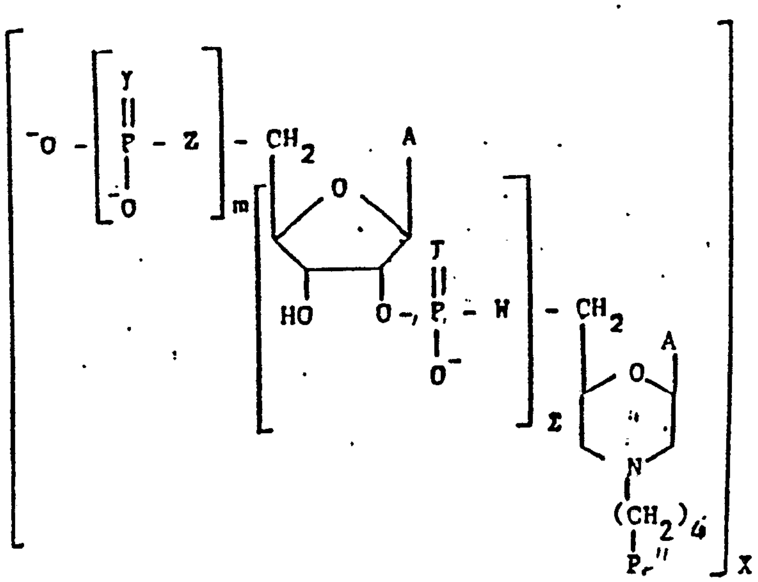

(III)

dans laquelle:
- Y et T, identiques ou différents, représentent indépendamment l'un de l'autre O, S, Se ou NH;
- Z et W, identiques ou différents, représentent indépendamment l'un de l'autre O, S ou NH;
- A représente l'adénine ou l'un de ses dérivés selon la revendication 2;
- Σ est un nombre entier égal à n - 1, n étant un nombre entier supérieur ou égal à 3;
- m est un nombre entier supérieur ou égal à 0 et de préférence supérieur ou egal à 1;
- X est un nombre entier égal à 1, ou de préférence compris de 2 à 10;
- Pr'' est un enchaînement peptidique comportant au moins 5 acides aminés et dans lequel le groupe $(CH_2)_4$- représenté dans la formule appartient à la séquence latérale d'un résidu lysyle entrant dans la constitution de l'enchaînement polypeptidique Pr'';

sous réserve que l'un au moins des éléments Y ou Z est différent de l'oxygène.

8. Composés de couplage selon la revendication 1 de formulas suivantes:

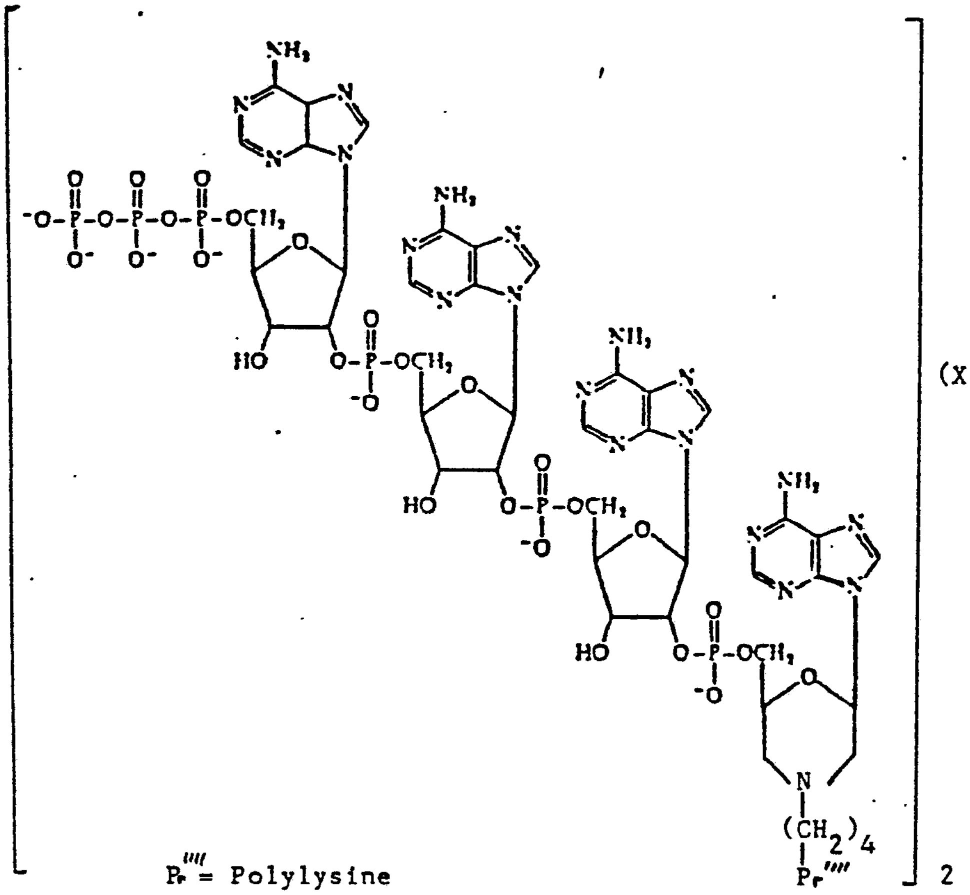

Pr'''' = Polylysine

(XIII)

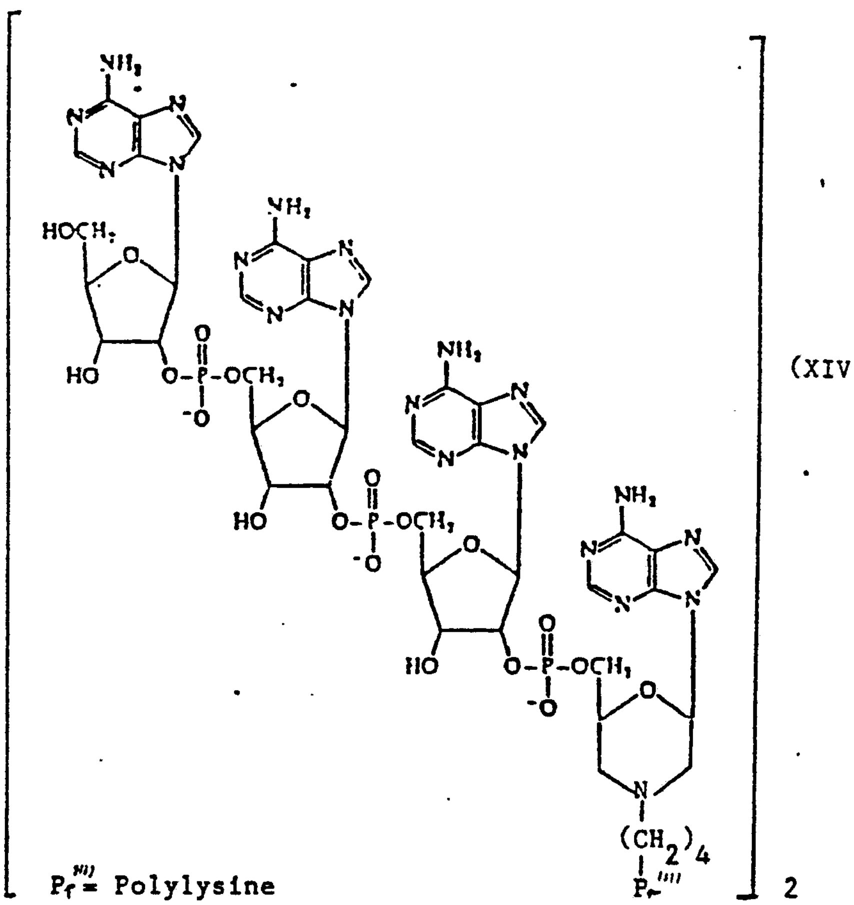

Pr'''' = Polylysine

(XIV)

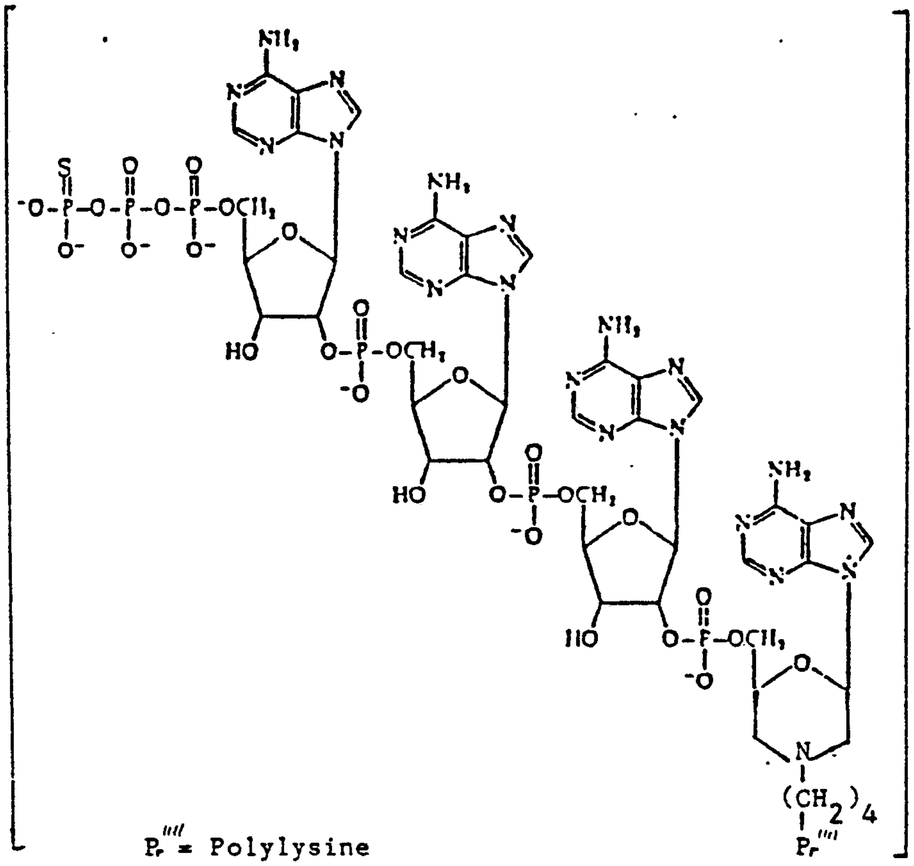

P'''' = Polylysine

(XV)

9. Procédé de préparation des composés de couplage selon l'une quelconque des revendications précédentes, caractérisé en ce qu'il comprend:

- l'oxydation du composé de départ de formule (Ibis) suivante:

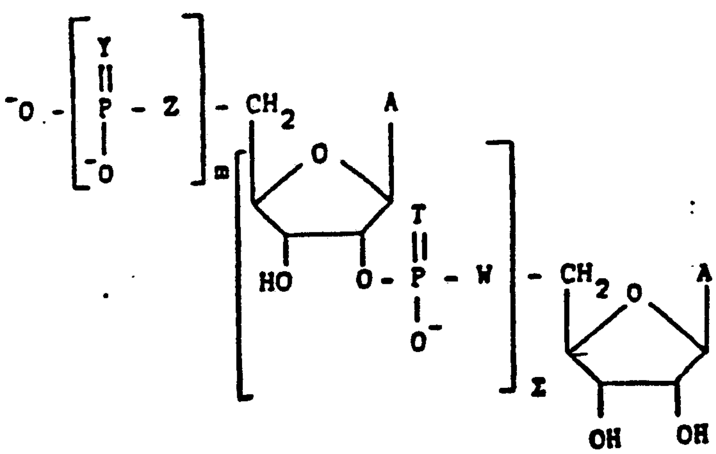

(Ibis)

dans laquelle Y, Z, T, W, A, m, Σ ont les significations indiquées à l'une des revendications 1 à 8;

pour introduire deux fonctions aldéhyde sur en 2' et 3' de la dernière unité nucléosidique et obtenir le composé de formule (Iter) suivante:

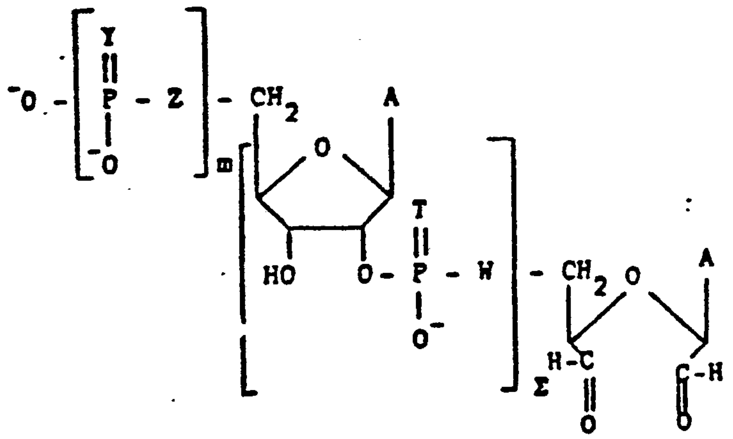

(Iter)

- l'alcoylation réductive du composé de formule (Iter) par un polypeptide Pr-$NH_2$ dont l'enchaînement comprend au moins une séquence latérale dont l'extrémité est un groupe aminé, notamment $NH_2$, cette réaction ayant lieu entre les fonctions aldéhyde du composé (Iter) avec le groupe $NH_2$ du polypeptide pour obtenir le composé de formule (I):

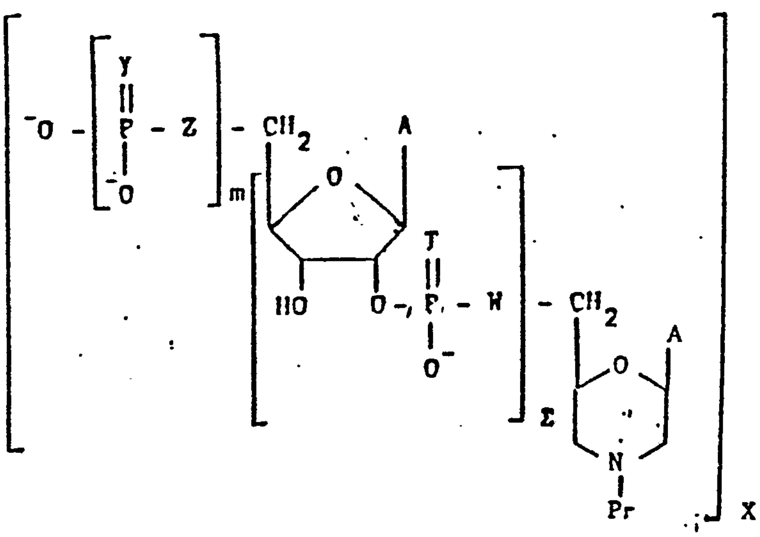

(I)

- le fractionnement entre les composés de couplage selon l'invention et les produits non couplés, notamment par filtration moléculaire.

**Revendications** pour l'état contractant: AT

1. Procéde de preparation des composés de couplage de formule I:

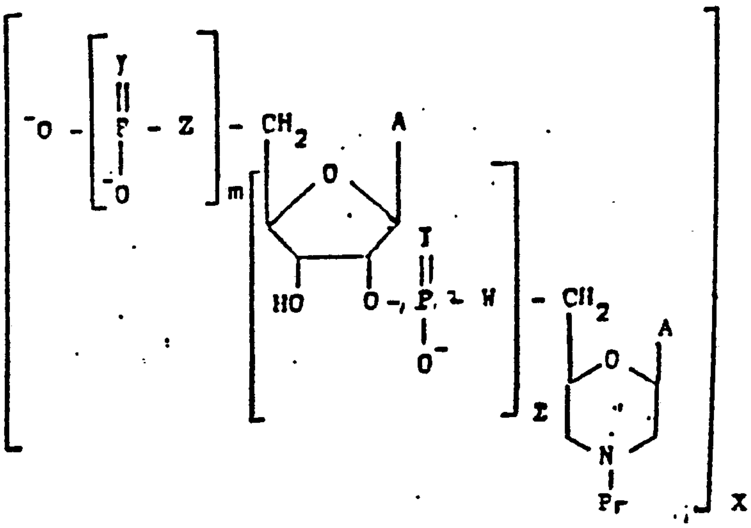

(I)

dans laquelle:
- Y et T, identiques ou différents, représentent indépandamment l'un de l'autre O, S, Se ou NH;
- Z et W, identiques ou différents, représentent indépendamment l'un de l'autre O, S, ou NH;

A représente l'adénine ou l'une de ses dérivés, notamment ceux de formule:

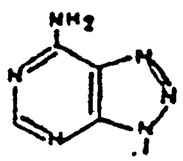
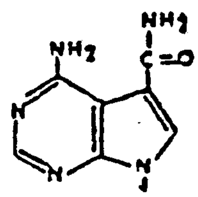
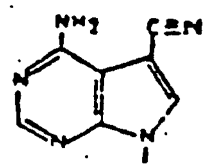
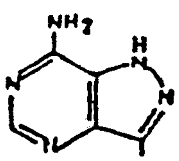
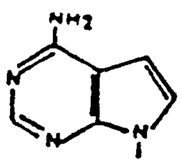
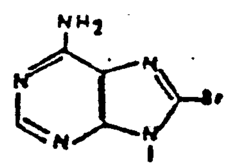

- Σ est un nombre entier égal à n - 1, étant un nombre entier superieur ou égal à 3;
- m est un nombre entier supérieur ou égal à 0 et de préférence supérieur ou égal à 2;
- X est un nombre entier supérieur ou égal à 1, de préference compris de 2 à 10;
- Pr est un enchaînement polypeptidique comportant au moins 5 acides aminés, et l'atome d'azote appartenant au cycle:

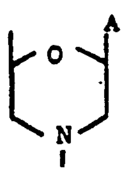

appartenant également à l'extrémité d'une séquence latérale du polypeptide Pr et/ou appartenant à un groupe

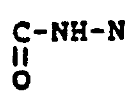

provenant d'un groupe COOH modifié par l'hydrazine
caractérisé en ce qu'il comprend:
- l'oxydation du composé de départ de formule (Ibis) suivante:

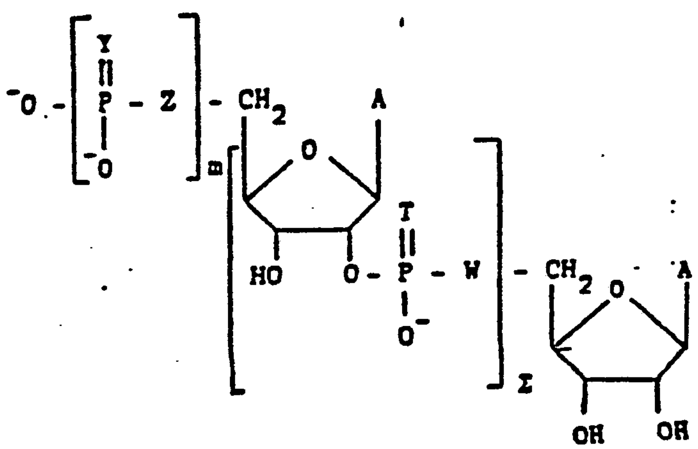

(Ibis)

dans laquelle Y, Z, T, W, A, m, Σ ont les significations indiquées précédemment;
pour introduire deux fonctions aldéhyde sur les carbones en 2' et 3' de la dernière unité nucléosidique et obtenir le composé de formule (Iter) suivante:

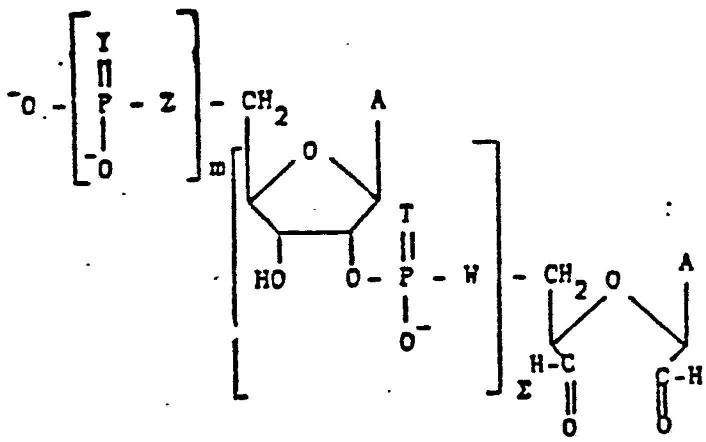

(Iter)

- l'alcoylation réductive du composé de formule (Iter) par un polypeptide Pr-$NH_2$ dont l'enchaînement comprend au moins une séquence latérale dont l'extrémité est un groupe amine, notamment $NH_2$, cette réaction ayant lieu entre les fonctions aldéhyde du composé (Iter) avec le groupe $NH_2$ du polypeptide pour obtenir le composé de formule (I):

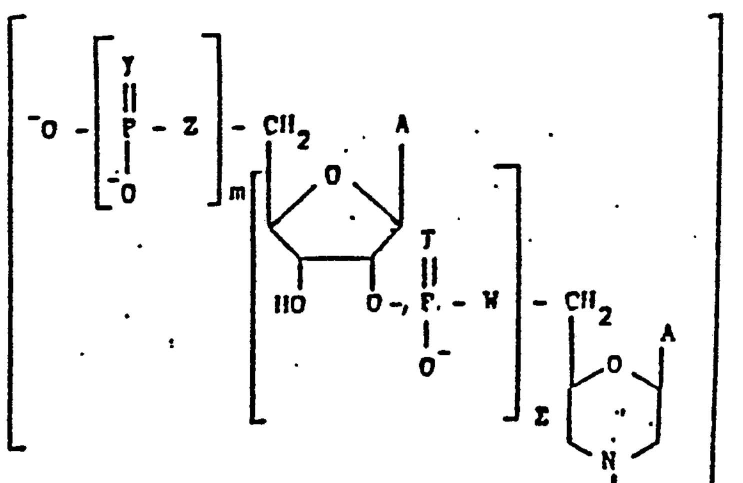

(I)

- le fractionnement entre les composés de couplage selon l'invention et les produits non couplés, notamment par filtration moléculaire.

2. Procédé de préparation selon la revendication 1 de composés de couplage de formule:

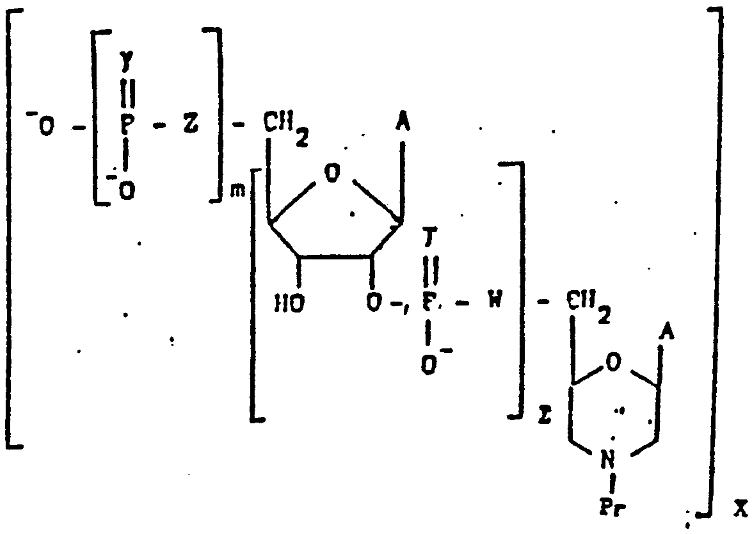

(I)

dans laquelle.

- Y et T, identiques ou différents, représentent indépendamment l'un de l'autre O, S, Se ou NH;
- Z et W, identiques ou différents, représentent indépendamment l'un de l'autre O, S ou NH;
- A représente l'adénine ou l'un de ses dérivés selon la revendication 1;
- Σ est un nombre entier égal à n - 1, n étant un nombre entier supérieur ou égal à 3;
- m est un nombre entier égal ou supérieur à 0 et de préférence supérieur ou égal à 2;
- X est un nombre entier supérieur ou égal à 1, de preférence compris de 2 à 10;
- Pr est un enchaînement polypeptidique comportant au moins 5 acides aminés, et l'atome d'azote appartenant au cycle:

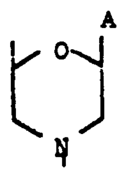

appartenant également à l'extrémité d'une séquence latérale du polypeptide Pr.

3. Procédé de préparation selon la revendication 1 de composés de couplage de formule (II):

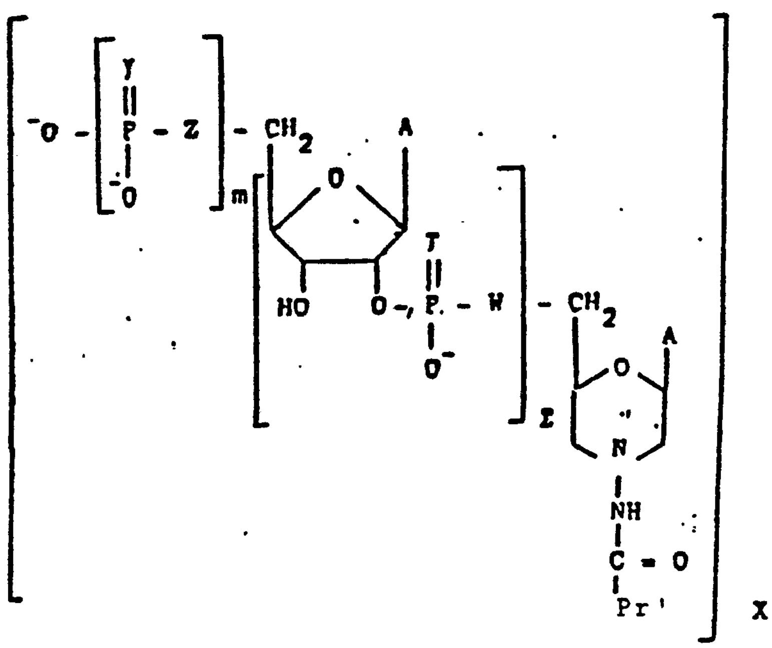

(II)

dans laquelle Y, Z, T, W, Σ, X m et A ont les significations ci-dessus définies, Pr' représente un enchaînement polypeptidique comportant au moins 5 acides aminés, et dans lequel le groupe

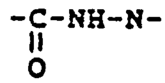

représenté sur la figure provient du groupe

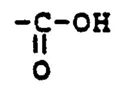

terminal du polypeptide et/ou d'un groupe

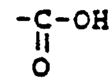

libre des résidus aspartyle et/ou glutamyle.

4. Procédé de préparation selon 1, revendication 1 de composés de formule (III):

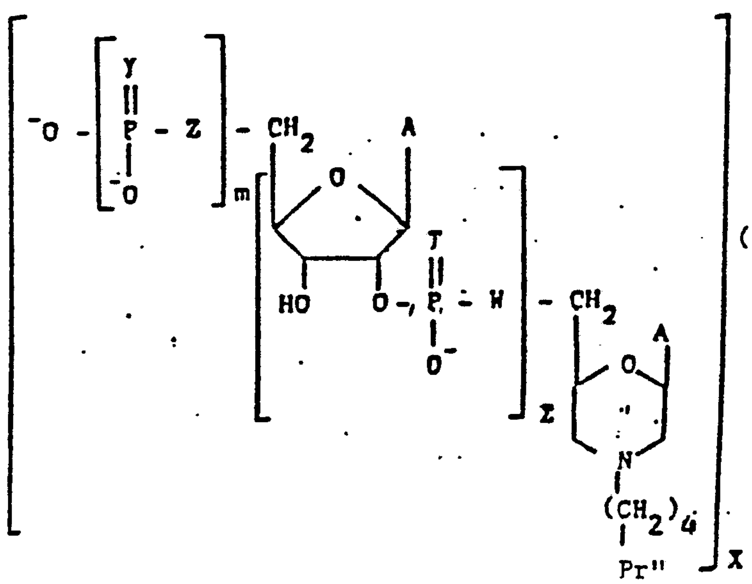

(III)

dans laquelle:
- Y et T identiques ou différents, représentent indépendamment l'un de l'autre O, S, Se ou NH;
- Z et W, identiques ou différents, représentent indépendamment l'un de l'autre O, S, ou NH;
- A représente l'adénine ou l'un de ses dérivés selon la revendication 1;
- Σ est un nombre entier égal à n - 1, n étant un nombre entier supérieur ou égal à 3;
- X est un nombre entier égal ou supérieur à 1, de préference compris de 2 à 10;
- m est un nombre entier supérieur ou égal à 0 et de préférence supérieur ou égal 2;
- Pr'' est un enchaînement polypeptidique comportant au moins 5 acides amines et dans laquelle le groupe $-(CH_2)_4-$ représenté dans la formule appartient à la séquence latérale d'un résidu lysyle entrant dans la constitution de l'enchaînement polypepridique Pr''.

5. Procédé de préparation selon la revendication 1 de composés de formule (IV):

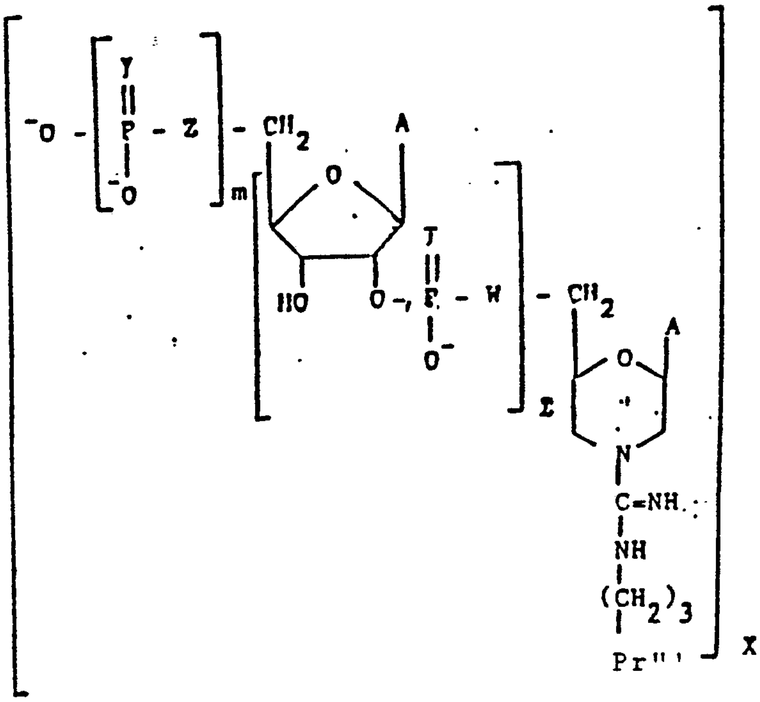

(IV)

dans laquelle T, Z, W, A, Σ, n, X, m ont les significations indiquées à la revendication 1 Pr''' est un enchaînement peptidique comportant au moins 5 acides aminés et l'atome d'azote appartenant au cycle:

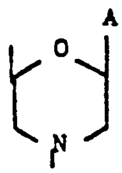

appartient également à l'extrémité de la séquence latérale d'un résidu d'arginyle entrant dans la constitution de l'enchaînement polypeptidique Pr''.

6. Procédé de préparation selon la revendication 1 de composés de formule (V):

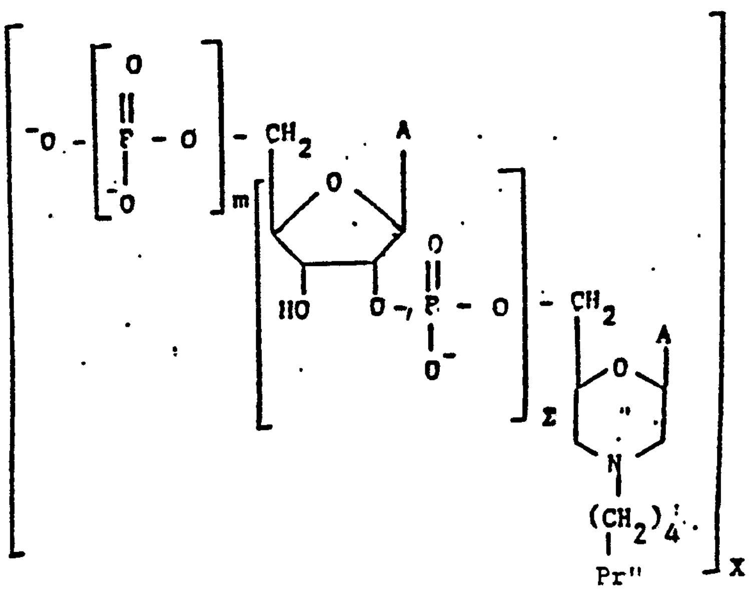

(V)

dans laquelle:

- A représente l'adénine ou l'un de ses dérivés selon la revendication 1;
- Σ est un nombre entier égal à n - 1, n étant un nombre entier supérieur ou égal à 3;
- m est un nombre entier supérieur ou égal à 0 et de préférence supérieur ou égal à 1;
- X est un nombre entier égal à 1 ou, de préférence compris de 2 à 10;
- Pr'' est un enchaînement peptidique comportant au moins 5 acides aminés et dans laquelle le groupe $-(CE_2)_4-$ représenté dans la formule appartient à la séquence latérale d'un résidu lysyle entrant dans la constitution de l'enchaînement polypeptidique Pr''.

7. Procédé de préparation selon la revendication 1 de composés de formule suivante:

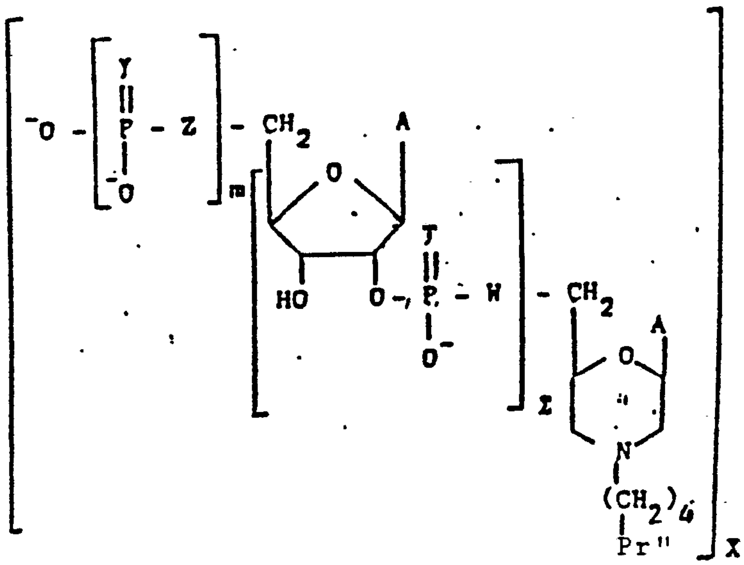

(III)

dans laquelle:
- Y et T, identiques ou différents, représentent indépendamment l'un de l'autre O, S, Se ou NH;
- Z et W, identiques ou différents, représentent indépendamment l'un de l'autre O, S ou NH;
- A représente l'adénine ou l'un de ses dérivés selon la revendication 2;
- Σ est un nombre entier égal à n - 1, n étant un nombre entier supérieur ou égal à 3;
- m est un nombre entier supérieur ou égal à 0 et de préférence supérieur ou égal à 3;
- X est un nombre entier égal à 1, ou de préférence compris de 2 à 10;
- Pr'' est un enchaînement peptidique comportant au moins 5 acides aminés et dans lequel le groupe $(CH_2)_4$- représenté dans la formule appartient à la séquence latérale d'un résidu lysyle entrant dans la constitution de l'enchaînement polypeptidique Pr'';

sous réserve que l'un au moins des éléments Y ou Z est différent de l'oxygène.

8. Procédé de préparation selon la revendication 1 de composés de couplage de formules siuvantes:

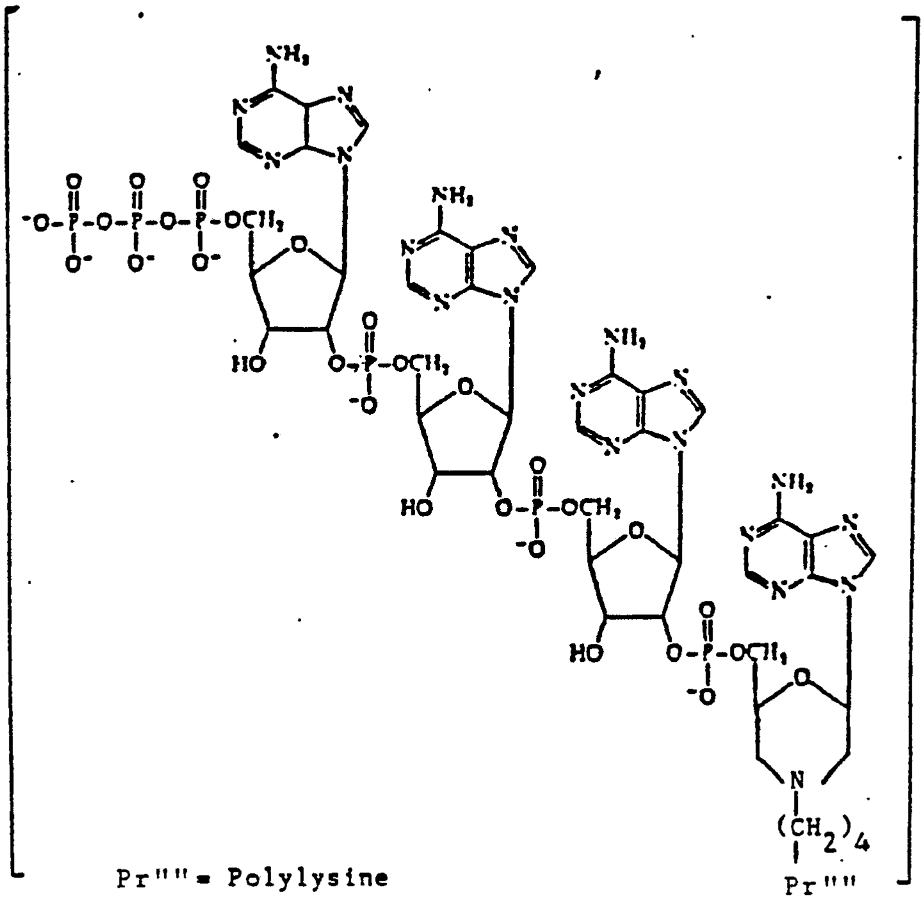

Pr'''' = Polylysine

(XIII)

2

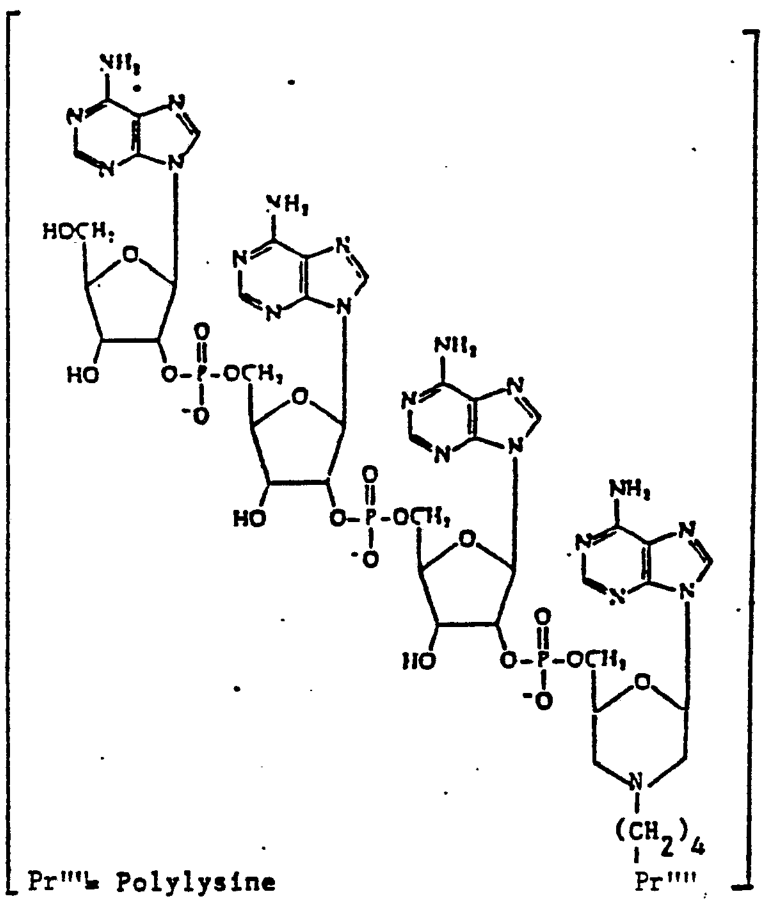

Pr'''' = Polylysine

(XIV)

2

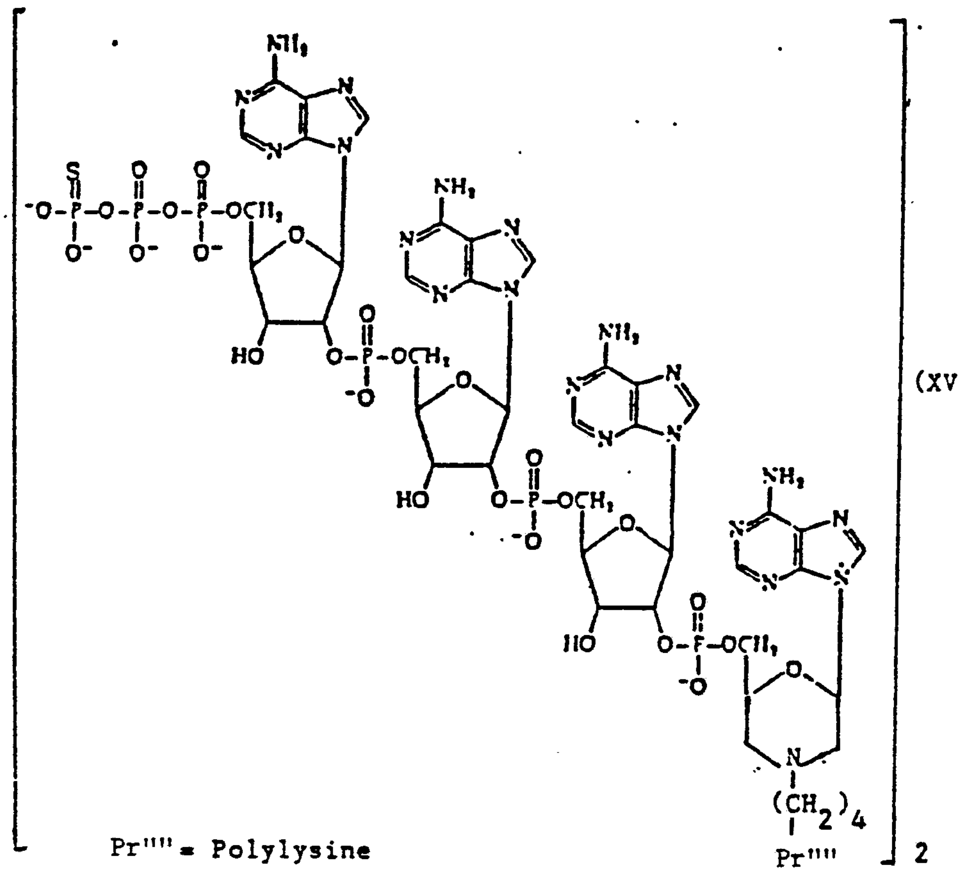

(XV)

Pr'''' = Polylysine

9. Procédé de préparation d'oligonucléotides selon les revendications 1 à 8, caractérisé en ce que la synthèse enzymatique des composés de formule (Ibis), comprend:
- la polymérisation du composé de formule (Iquater) suivante:

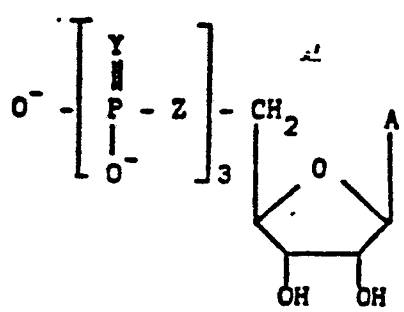

(Iquater)

dans laquelle:
- Y représente O, S Se ou NH;
- Z représente O, S ou NH;
  l'un au moins des éléments Y et Z pouvant être différent de l'oxygène;
- A a les significations indiquées aux revendications 1 à 7.

10. Procédé de préparation d'oligonucléotides selon les revendications 1 à 8, caractérisé en ce qu'il comprend la polymérisation d'un composé de formule suivante (XVIbis):

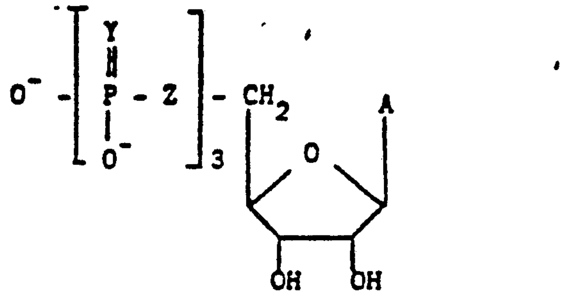

(XVIbis)

dans laquelle:
- Y représente O, S, Se ou NH;
- Z représente O, S ou NH;
- Y et Z ne représentent pas simultanément l'oxygène;
- A a les significations indiquées aux revendications précédentes.
  pour obtenir les composés de formule suivante (XVI):

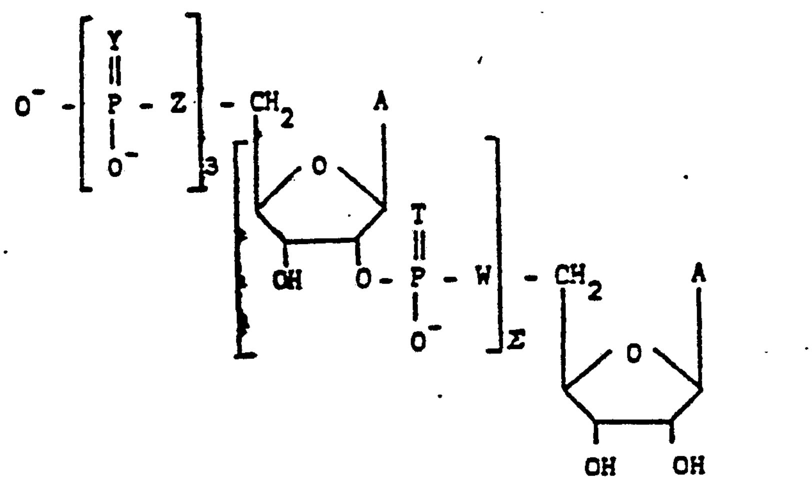

(XVI)

dans laquelle:
- Y et T, identiques ou différents, représentent O, S, Se, NH;
- Z et W, identiques ou différents, représentent O, S, NH;
- Y et Z ne représentent pas simultanément l'oxygène;
- Σ est un nombre entier variant de 2 à 9;
- A est une base choisie parmi l'adénine ou ses dérivés, notamment ceux de formule:

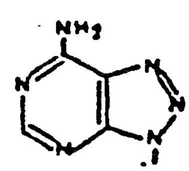
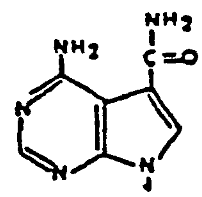
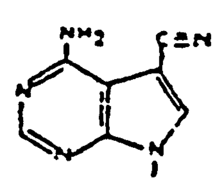
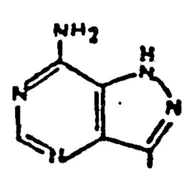
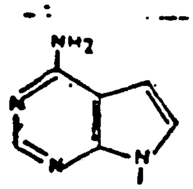
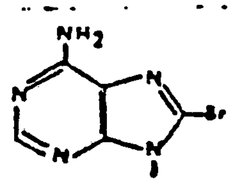

11. Procédé de préparation d'oligonucléotides selon les revendications 1 à 8, caractérisé en ce qu'i comprend la polymérisation du composé de formule suivante (XVIIbis):

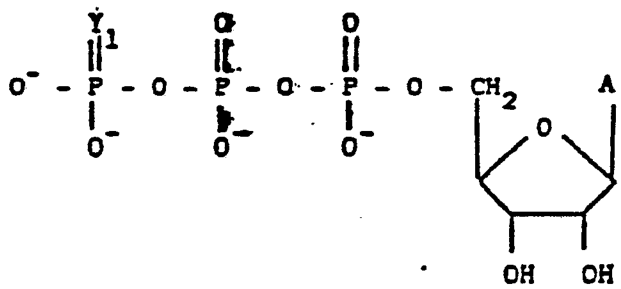

(XVIIbis)

dans laquelle:
- $Y_1$ représente NH, Se, S;
- A a la signification indiquée aux revendications précédentes,
pour obtenir les composés de formule suivante (XVII):

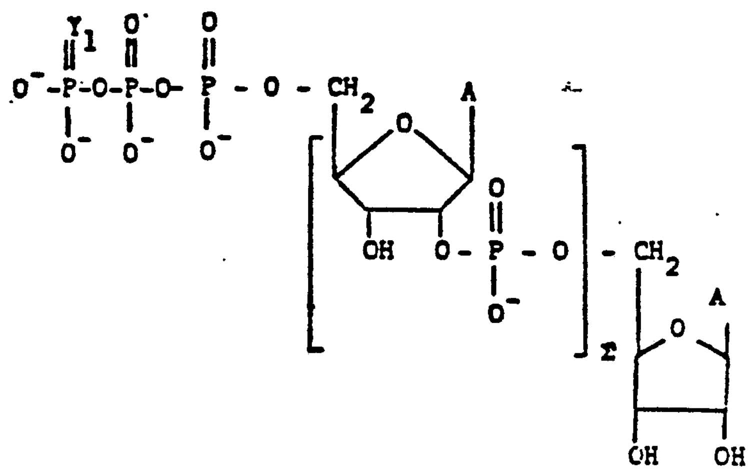

(XVII)

dans laquelle:
- $Y_1$ représente NH, Se, S;
- Σ est un nombre entier égal à n - 1, n variant de 3 à 10;
- A a, la signification indiquée aux revendications précédentes.

12. Procédé de préparation d'oligonucléotides selon les revendications 1 à 8, caractérisé en ce qu'il comprend:
la polymérisation d'un composé de formule suivante (XVIIIbis):

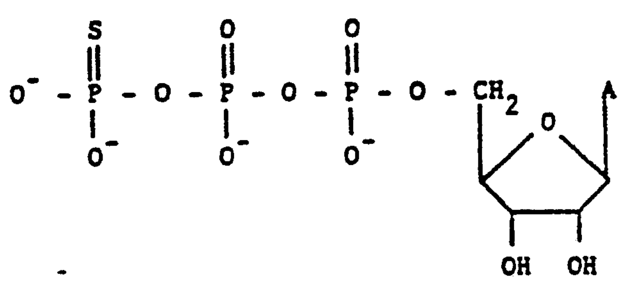

(XVIIIbis)

dans laquelle
- A a la signification indiquée aux revendications précédentes,
pour obtenir les composés de formule suivante (XVIII):

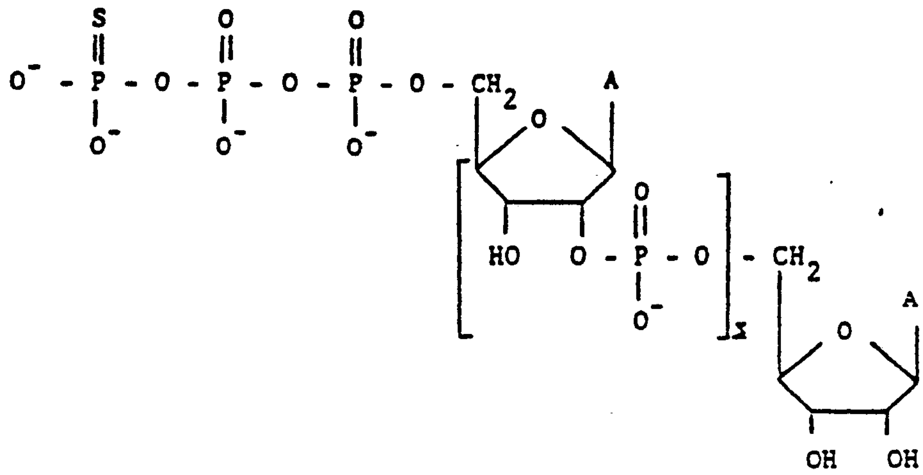

(XVIII)

dans laquelle:
- Σ a les significations précedemment indiquées;
- A a la signification précédemment indiquée, et de préférence représente l'adénine.

13. Procédé de préparation d'oligonucléotides caractérisé en ce qu'il comprend:
la polymérisation du composé de formule suivante (XIXbis):

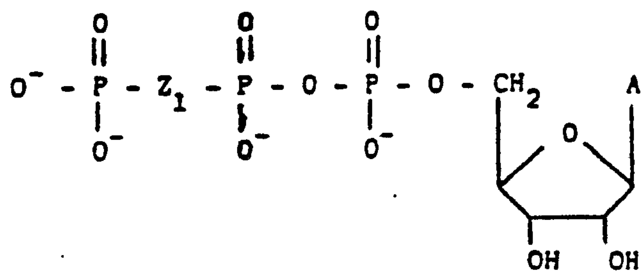

(XIXbis)

dans laquelle:
$Z_1$ représente S ou NH et
A a la signification indiquée aux revendications précédentes
pour obtenir les composés de formule suivante (XIX):

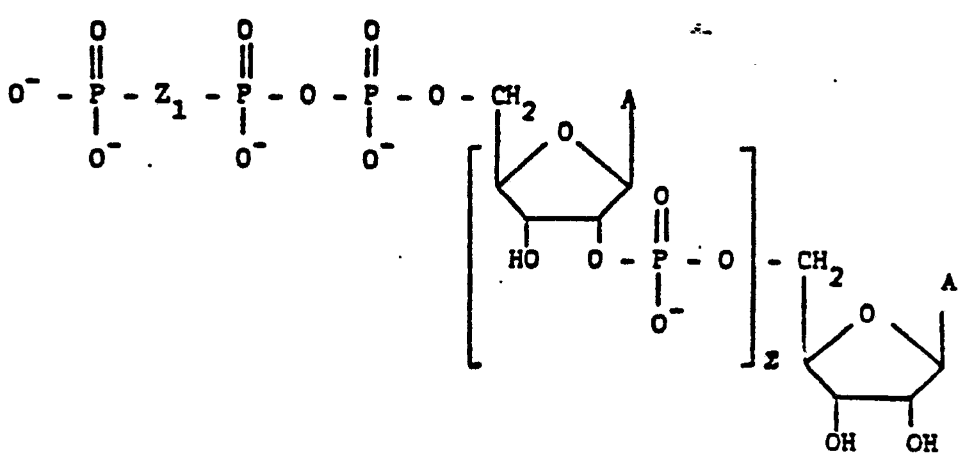

, (XIX)

dans laquelle:
- $Z_1$ représente NH ou S;
- Σ est un nombre entier égal à n - 1, n variant de 3 à 10;
- A a le signification indiquée aux revendications précédentes.

14. Procédé de préparation d'oligonucléotides selon les revendications 1 à 8, caractérisé en ce qu'il comprend:
la polymérisation du composé de formule suivante (XXbis):

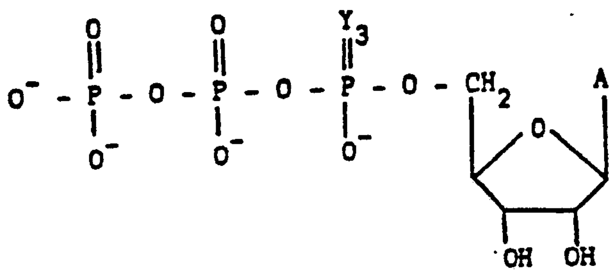

(XXbis)

dans laquelle:
- $Y_3$ représente S, Se, NH;
- A a les signifactions indiquées aux revendications précédentes,
  pour obtenir les composés de formule suivante (XX):

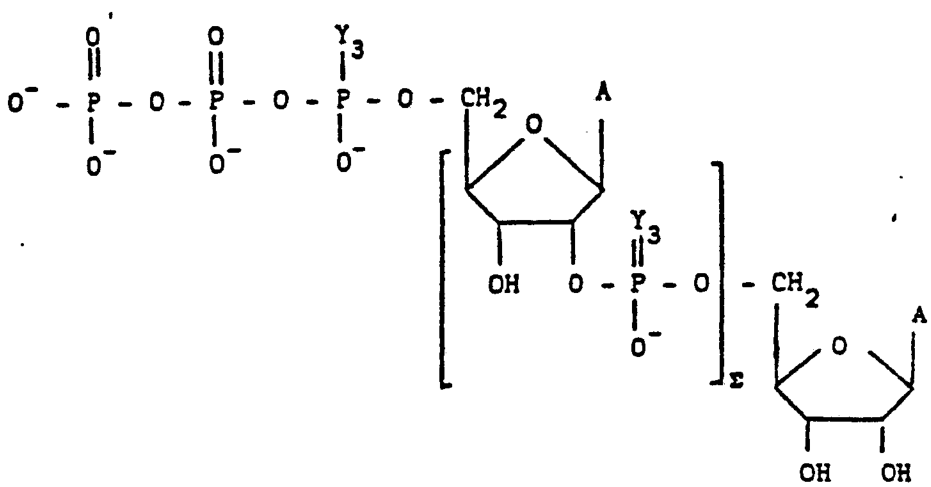

(XX)

dans laquelle:
- $Y_3$ représente NH, S ou Se;
- Σ et A ont les significations indiquées ci-dessus.

**Patentansprüche** für die Vertragsstaaten: BE, CH, DE, FR, GB, IT, LI, LU, NL, SE

1. Kupplungsverbindungen, die durch die nachstehend definierte Formel (I) repräsentiert werden:

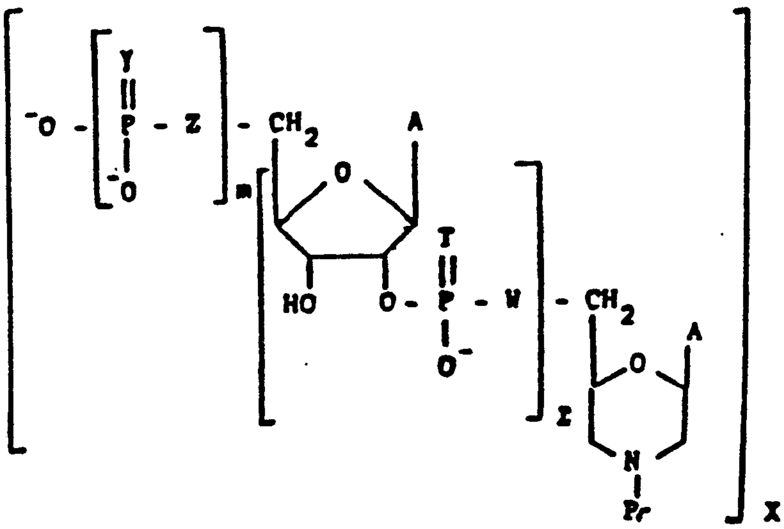

(I) ,

in welcher:
- Y und T, die gleich oder verschieden sind, unabhängig voneinander O, S, Se oder NH bedeuten;
- Z und W, die gleich oder verschieden sind, unabhängig voneinander O, S oder NH bedeuten;

A Adenin oder eines seiner Derivate, insbesondere solche der Formel:

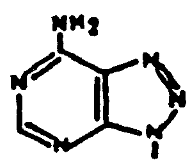
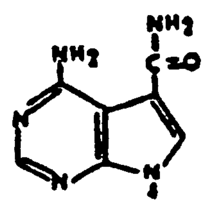
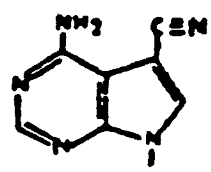
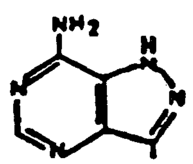
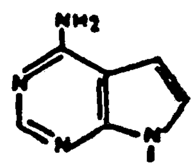
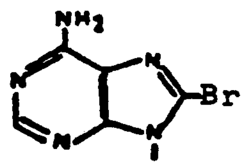

bedeutet;
- Σ eine ganze Zahl gleich n - 1 ist wobei n eine ganze Zahl größer oder gleich 3 ist;
- m eine ganze Zahl größer oder gleich 0, und vorzugsweise größer oder gleich 2 ist;
- X eine ganze Zahl größer oder gleich 1, vorzugsweise 2 bis 10, ist;
- Pr eine wenigstens 5 Aminosäuren enthaltende Polypeptidkette ist, und das dem Ring:

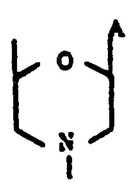

angehörende Stickstoffatom auch dem Ende einer Seitensequenz des Polypeptids Pr angehört, und/oder einer Gruppe

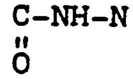

angehört, die von einer durch Hydrazin modifizierten COOH-Gruppe stammt.

2. Kupplungsverbindungen nach Anspruch 1 der Formel:

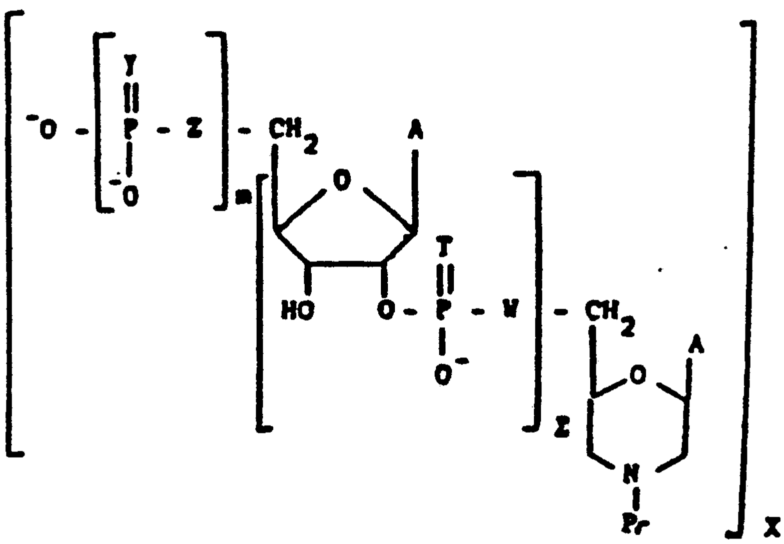

(I) ,

in welcher:

- Y und T, die gleich oder verschieden sind, unabhängig voneinander O, S, Se oder NH bedeuten;
- Z und W, die gleich oder verschieden sind, unabhängig voneinander O, S oder NH bedeuten;
- A Adenin oder eines seiner Derivate gemäß Anspruch 1 bedeutet;
- Σ eine ganze Zahl gleich n - 1 ist wobei n eine ganze Zähl größer oder gleich 3 ist;
- m eine ganze Zahl größer oder gleich 0, und vorzugsweise größer oder gleich 2 ist;
- X eine ganze Zahl größer oder gleich 1, vorzugsweise 2 bis 10, ist;
- Pr eine wenigstens 5 Aminosäuren enthaltende Polypeptidkette ist, und das dem Ring:

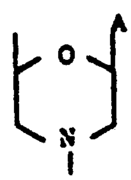

angehörende Stickstoffatom auch dem Ende einer Seitensequenz des Polypeptids Pr angehört.

3. Kupplungsverbindungen nach Anspruch 1 der Formel (II):

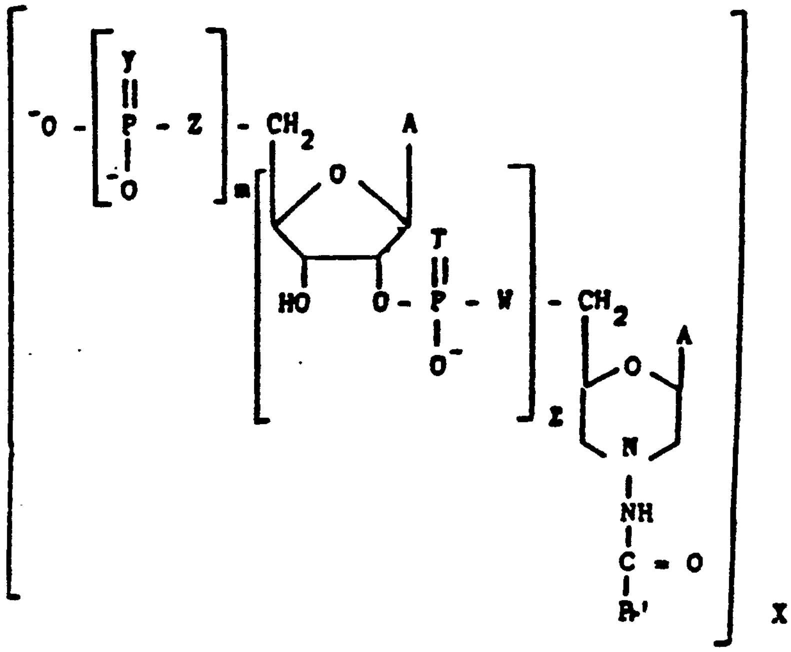 (II) ,

in welcher Y, Z, T, W, A, m, Σ und X die oben definierten Bedeutungen haben, Pr' eine wenigstens 5 Aminosäuren enthaltende Polypeptidkette bedeutet, und in welcher die Gruppe

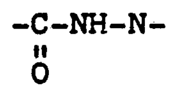

im Formelbild von den endständigen

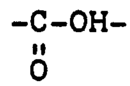

Gruppe des Polypeptids und/oder einer freien

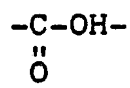

Gruppe von Aspartyl- und/oder Glutamylresten stammt.

4. Kupplungsverbindungen nach Anspruch 1 der Formel (III):

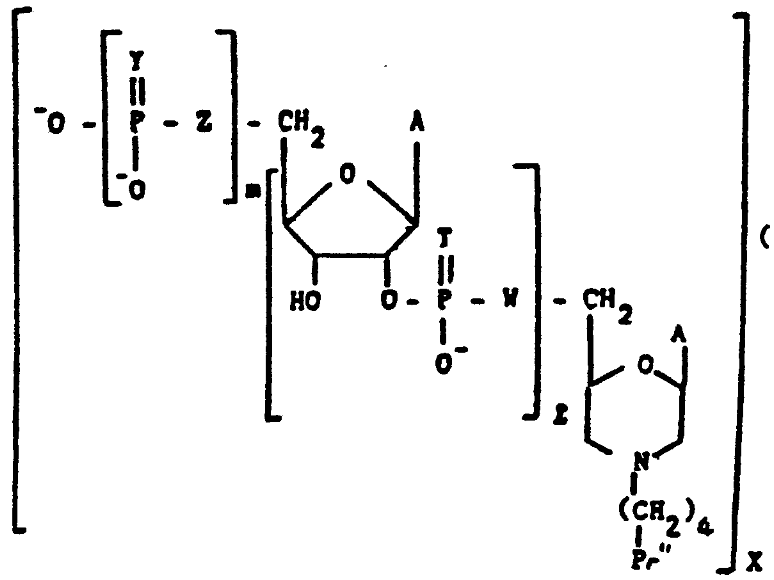

(III) ,

in welcher:

- Y und T die gleich oder verschieden sind, unabhängig voneinander O, S, Se oder NH bedeuten;
- Z und W, die gleich oder verschieden sind, unabhängig voneinander O, S oder NH bedeuten;
- A Adenin oder eines seiner Derivate nach Anspruch 1 bedeutet;
- Σ eine ganze Zahl gleich n - 1 ist, wobei n eine ganze Zahl größer oder gleich 3 ist;
- m eine ganze Zahl großer oder gleich 0, und vorzugsweise größer oder gleich 2 ist;
- X eine ganze Zahl größer oder gleich 1, vorzugsweise 2 bis 10, ist;
- Pr'' eine wenigstens 5 Aminosäuren enthaltende Polypeptidkette ist; und in welcher die in der Formel aufscheinende Gruppe $-(CH_2)_4-$ der Seitensequenz eines Lysylrestes angehört, der in der Konstitution der Polypeptidkette Pr'' vorliegt.

5. Kupplungsverbindungen nach Anspruch 1 der Formel (IV):

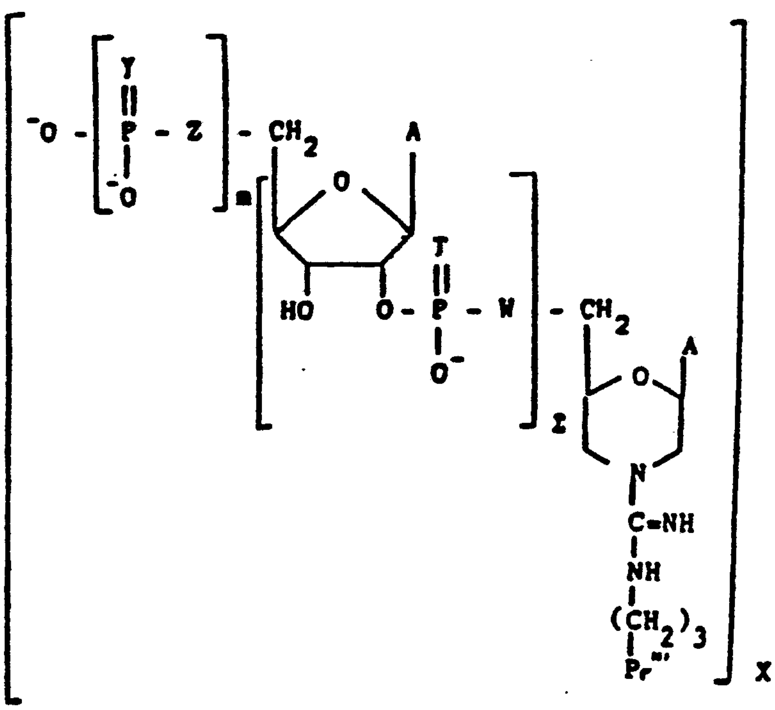

(IV) ,

in welcher Y, T, Z, W, A, Σ, n, X und m die in Anspruch 1 angegebene Bedeutung haben, Pr'' eine wenigstens 5 Aminosäuren enthaltende Peptidkette ist, und das dem Ring:

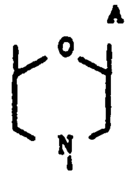

angehörende Stickstoffatom auch dem Ende der Seitensequenz eines Arginylrestes angehört, der in der Konstitution der Polypeptidkette Pr'' vorliegt.

6. Kupplungsverbindungen nach Anspruch 1 der Formel (V):

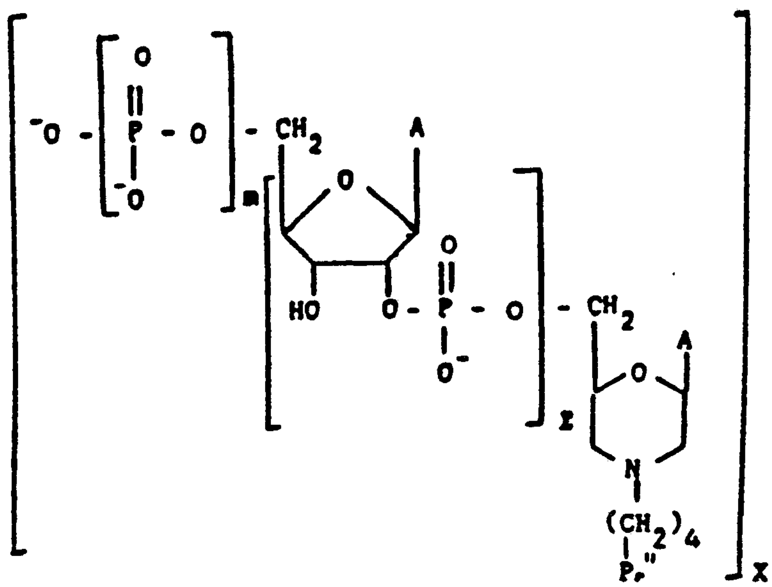

(V) ,

in welcher:
- A Adenin oder eines seiner Derivate gemäß Anspruch 1 bedeutet:
- Σ eine ganze Zahl gleich n - 1 ist, wobei n eine ganze Zahl großer oder gleich 3 ist;
- m eine ganze Zahl größer oder gleich 0, und vorzugsweise größer als 2 ist;
- X eine ganze Zahl gleich 1, oder vorzugsweise 2 bis 10, ist;
- Pr'' eine wenigstens 5 Aminosäuren enthaltende Peptidkette ist; und in welcher die in der Formel aufscheinende Gruppe $-(CH_2)_4-$ der Seitensequenz eines Lysylrestes angehört, welcher in der Konstitution der Polypeptidkette Pr'' vorliegt.

7. Kupplungsverbindungen nach Anspruch 1 der folgenden Formel:

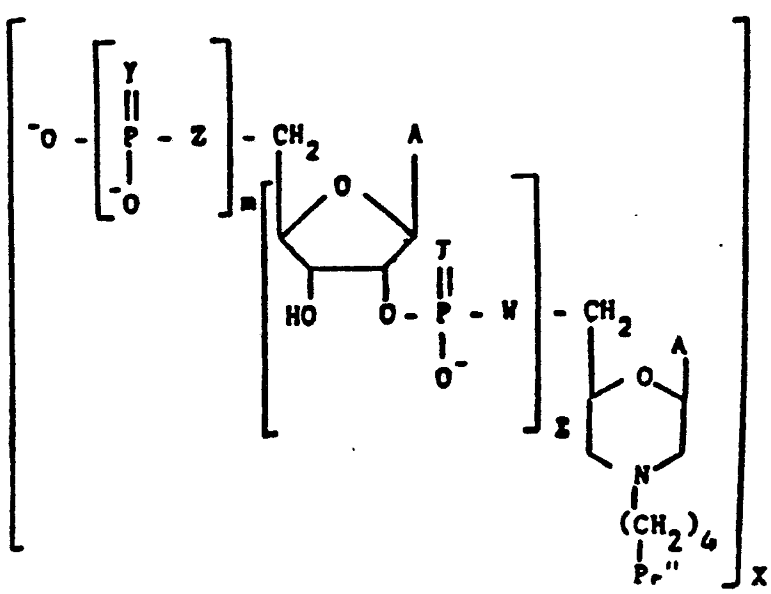

(III),

in welcher:

- Y und T, die gleich oder verschieden sind, unabhängig voneinander O, S, Se oder NH bedeuten;
- Z und W, die gleich oder verschieden sind, unabhängig voneinander O, S oder NH bedeuten;
- A Adenin oder eines seiner Derivate gemäß Anspruch 2 bedeutet;
- Σ eine ganze Zahl gleich n - 1 ist, wobei n eine ganze Zahl größer oder gleich 3 ist;
- m eine ganze Zahl größer oder gleich 0, und vorzugsweise großer oder gleich 1 ist;
- X eine ganze Zahl gleich 1, oder vorzugsweise 2 bis 10, ist;
- Pr'' eine wenigstens 5 Aminosäuren enthaltende Peptidkette ist; und in welcher die in der Formel aufscheinende Gruppe $(CH_2)_4$- der Seitensequenz eines in der Konstitution der Polypeptidkette Pr'' vorliegenden Lysylrestes angehört; mit der Maßgabe, daß wenigstens einer der Reste Y und Z von Sauerstoff verschieden ist.

8. Kupplungsverbindungen nach Anspruch 1 der folgenden Formeln:

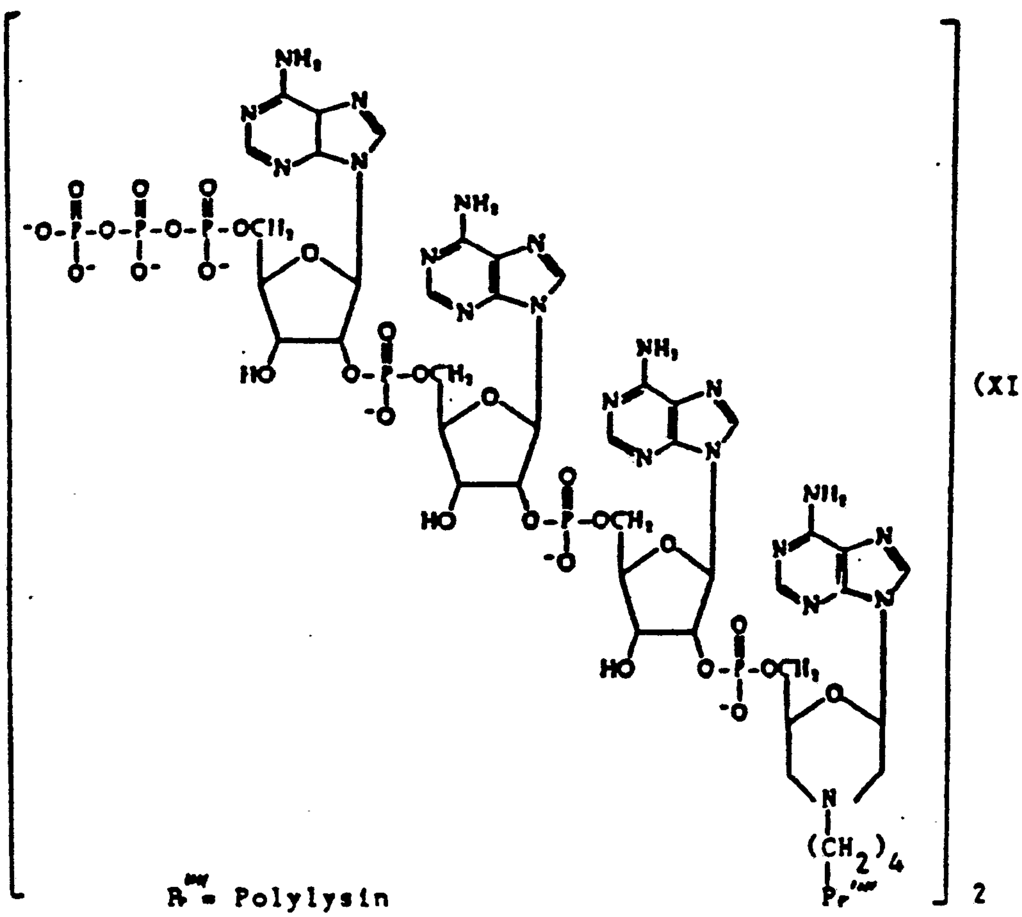

(XIII)

R''' = Polylysin

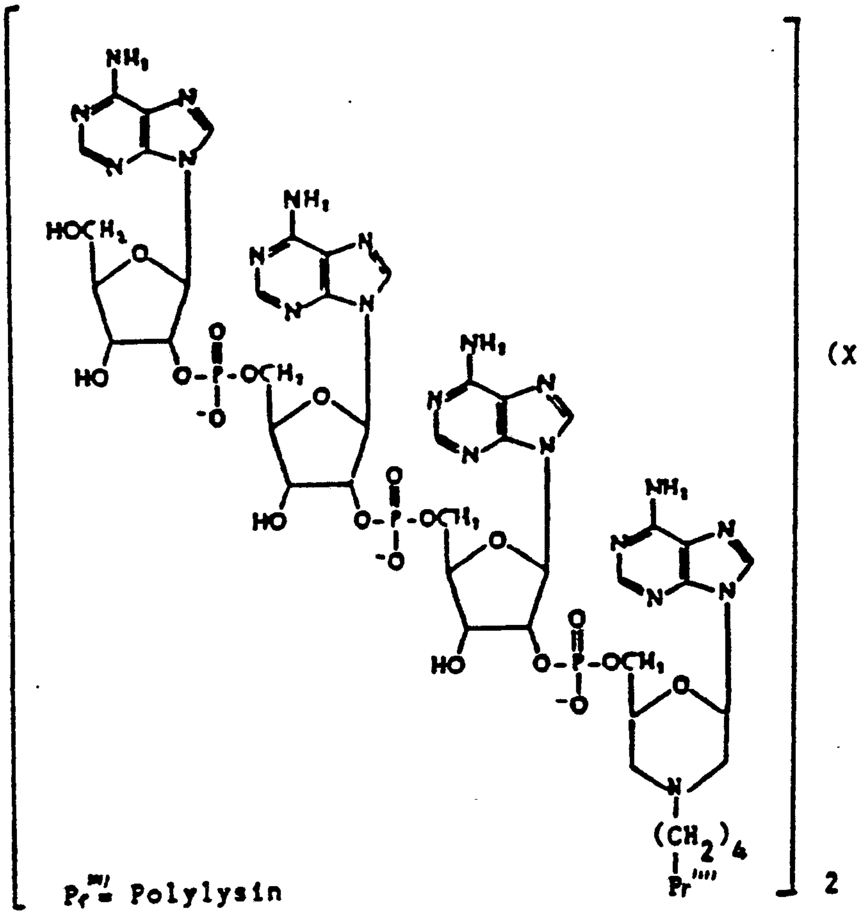

(XIV)

Pr'''' = Polylysin

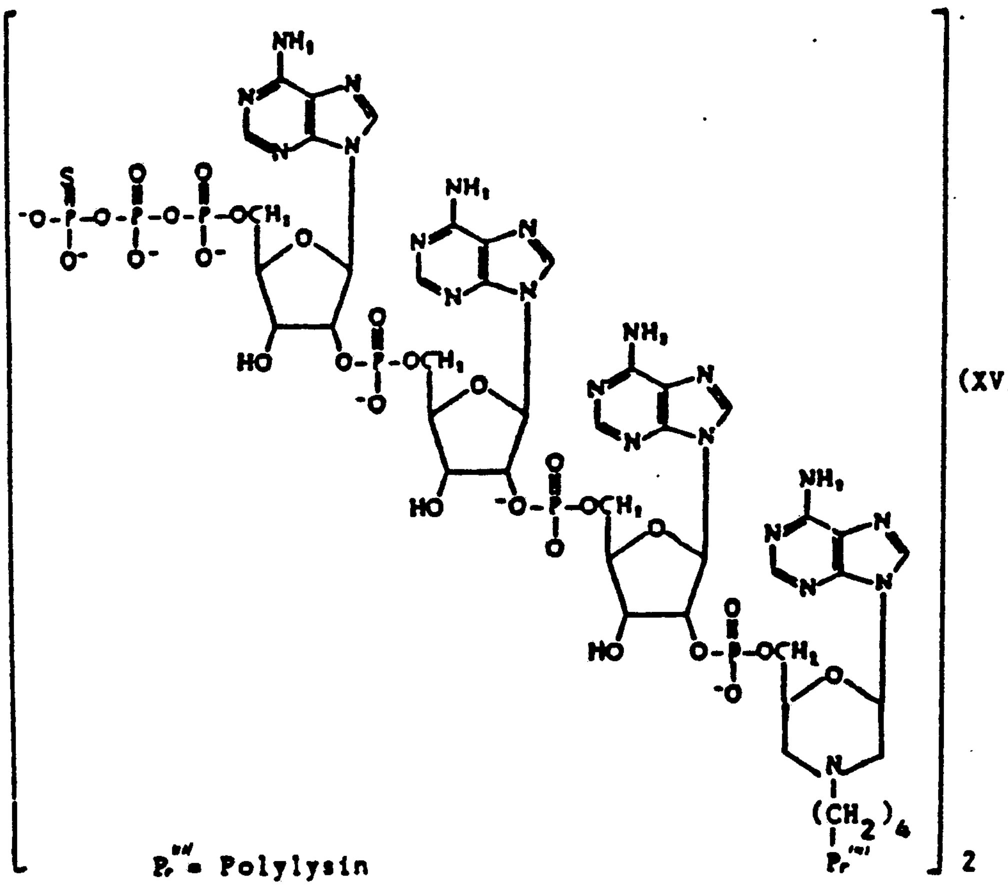

(XV)

Pr'''' = Polylysin

9. Verfahren zur Herstellung von Kupplungsverbindungen nach einem der vorhergehenden Ansprüche, dadurch gekennzeichnet, daß es umfaßt:
- die Oxydation der Ausgangsverbindung der folgenden Formel (Ibis):

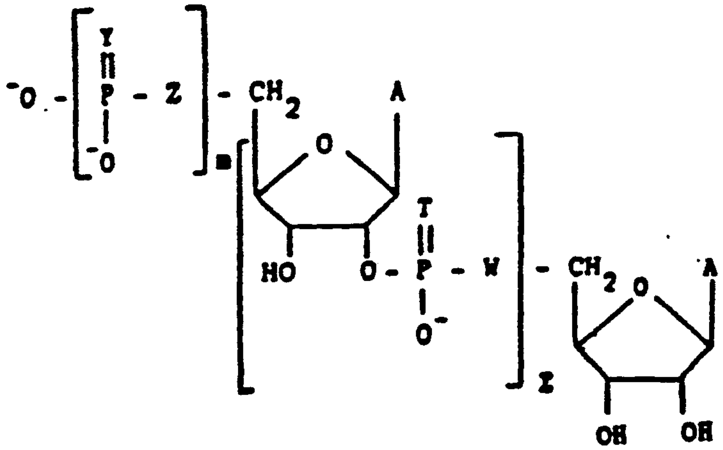

(Ibis),

in welcher Y, Z, T, W, A, m und Σ die in einem der Ansprüche 1 bis 8 angegebenen Bedeutungen haben;
zur Einführung von zwei Aldehydfunktionen an den Kohlenstoffen 2' und 3' der letzten Nukleosideinheit, wobei die Verbindung der folgenden Formel (Iter) erhalten wird:

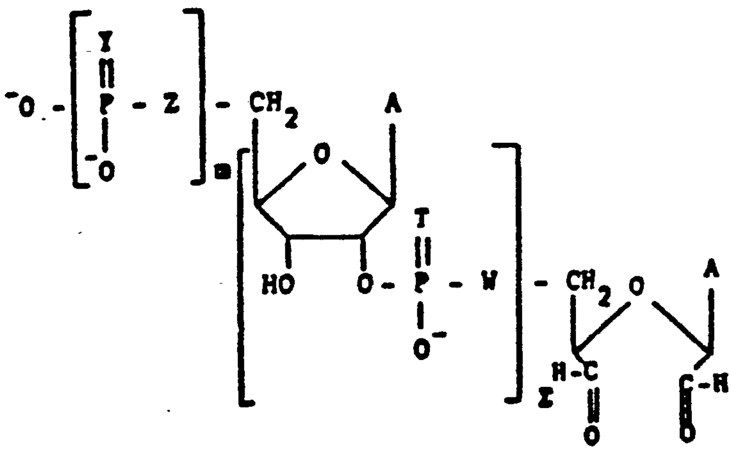

(Iter)

- die reduktive Alkylierung der Verbindung der Formel (Iter) mit einem Polypeptid $Pr\text{-}NH_2$, dessen Kette wenigstens eine Seitensequenz mit endständiger Aminogruppe, insbesondere $NH_2$, enthalt, wobei diese Reaktion zwischen den Aldehydfunktionen von Verbindung (Iter) und der $NH_2$-Gruppe des Polypeptids erfolgt, und die Verbindung der Formel (I):

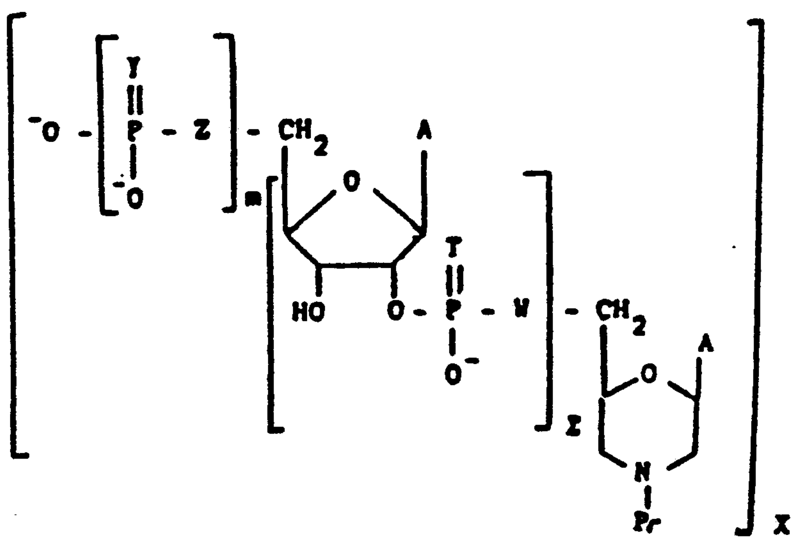

(I)

erhalten wird, und

- die Fraktionierung der Kupplungsverbindungen gemäß der Erfindung und der nicht gekuppelten Produkte, insbesondere durch Molekularfiltration.

**Patentansprüche** für den Vertragsstaat: AT

1. Verfahren zur Herstellung von Kupplungsverbindungen der Formel I:

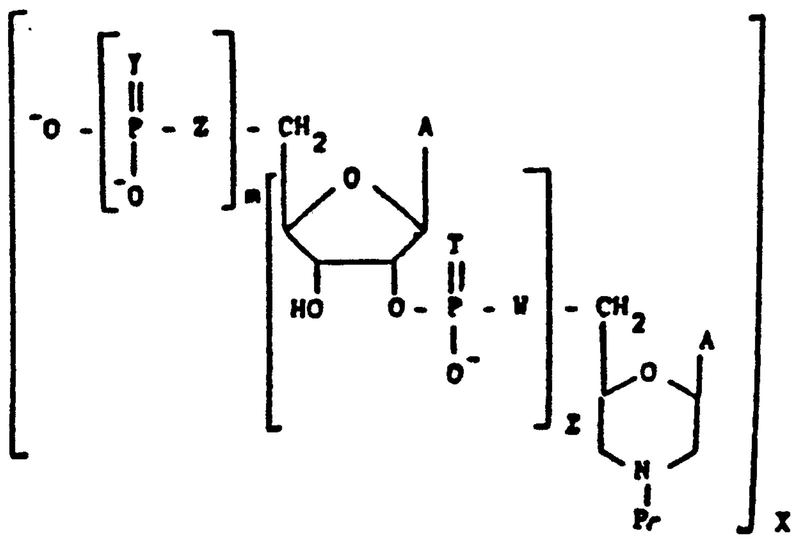

(I) ,

in welcher:

- Y und T, die gleich oder verschieden sind, unabhängig voneinander O, S, Se oder NH bedeuten;
- Z und W, die gleich oder verschieden sind, unabhängig voneinander O, S oder NH bedeuten;
- A Adenin oder eines seiner Derivate, insbesondere solche der Formel:

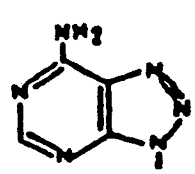
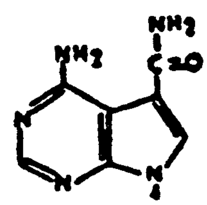
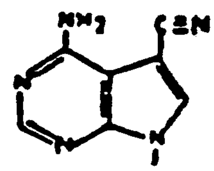
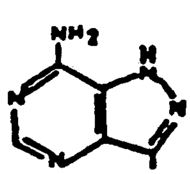
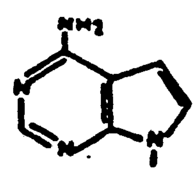
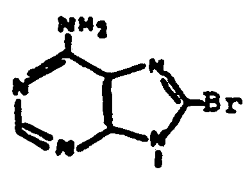

bedeutet;

- Σ eine ganze Zahl gleich n - 1 ist, wobei n eine ganze Zahl größer oder gleich 3 ist;
- m eine ganze Zahl größer oder gleich 0, und vorzugsweise größer oder gleich 2 ist;
- X eine ganze Zahl größer oder gleich 1, vorzugsweise 2 bis 10, ist;
- Pr eine wenigstens 5 Aminosäuren enthaltende Polypeptidkette ist, und das dem Ring:

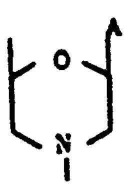

angehörende Stickstoffatom auch dem Ende einer Seitensequenz des Polypeptids Pr angehört, und/oder einer Gruppe

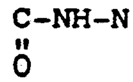

angehört, die von einer durch Hydrazin modifizierten COOH-Gruppe stammt, dadurch gekennzeichnet, daß es umfaßt:

- die Oxydation der Ausgangsverbindung der folgenden Formel (Ibis):

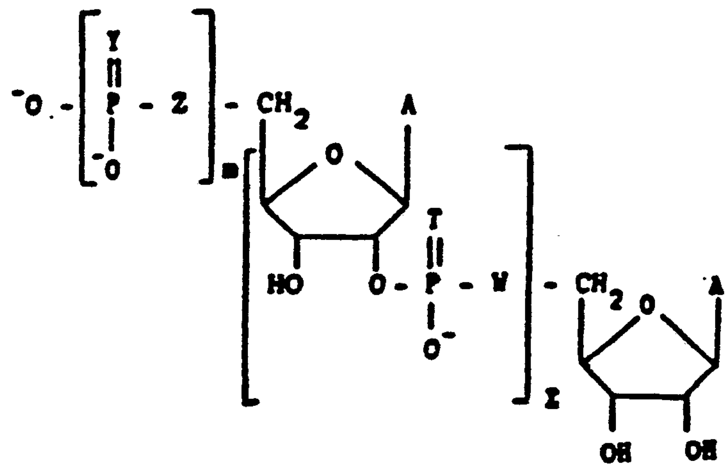

(Ibis),

in welcher Y, Z, T, W, A, m und Σ die oben angegebenen Bedeutungen haben;
zur Einführung von zwei Aldehydfunktionen an den Kohlenstoffen 2' und 3' der letzten Nukleosideinheit, wobei die Verbindung der folgenden Formel (Iter) erhalten wird:

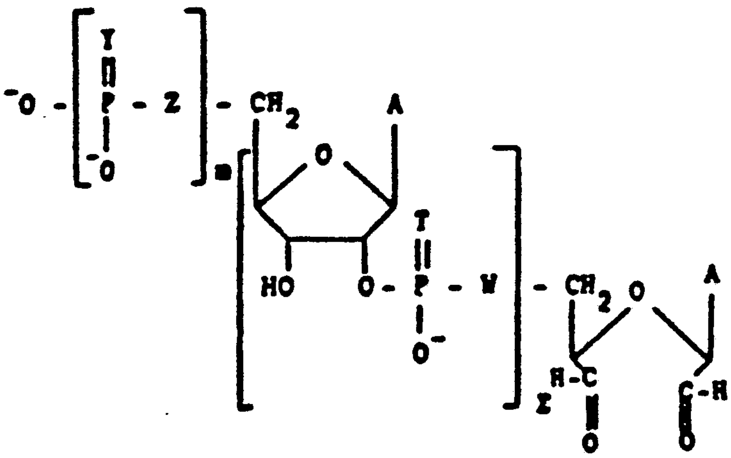

(Iter)

- die reduktive Alkylierung der Verbindung der Formel (Iter) mit einem Polypeptid Pr-$NH_2$, dessen Kette wenigstens eine Seitensequenz mit endständiger Aminogruppe, insbesondere $NH_2$, enthält, wobei diese Reaktion zwischen den Aldehydfunktionen von Verbindung (Iter) und der $NH_2$-Gruppe des Polypeptids erfolgt, und die Verbindung der Formel (I):

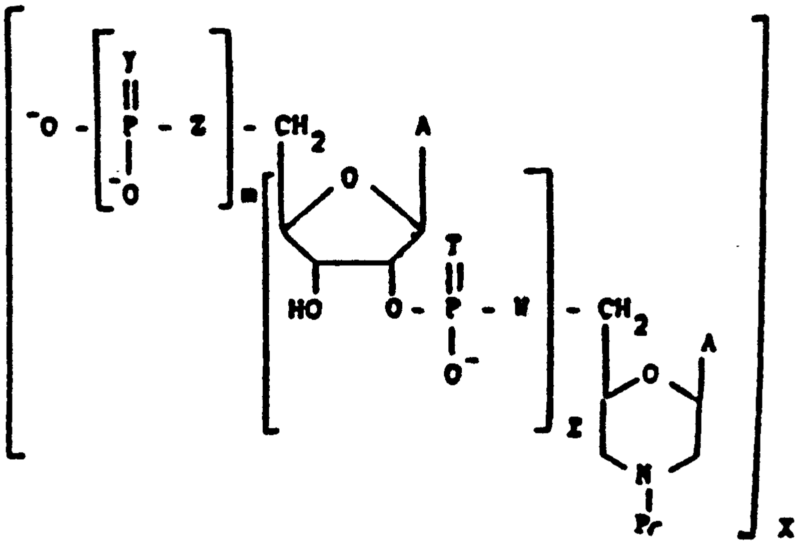

(I)

erhalten wird, und

- die Fraktionierung der Kupplungsverbindungen gemäß der Erfindung und der nicht gekuppelten Produkte, insbesondere durch Molekularfiltration.

2. Verfahren nach Anspruch 1 zur Herstellung von Kupplungsverbindungen der Formel:

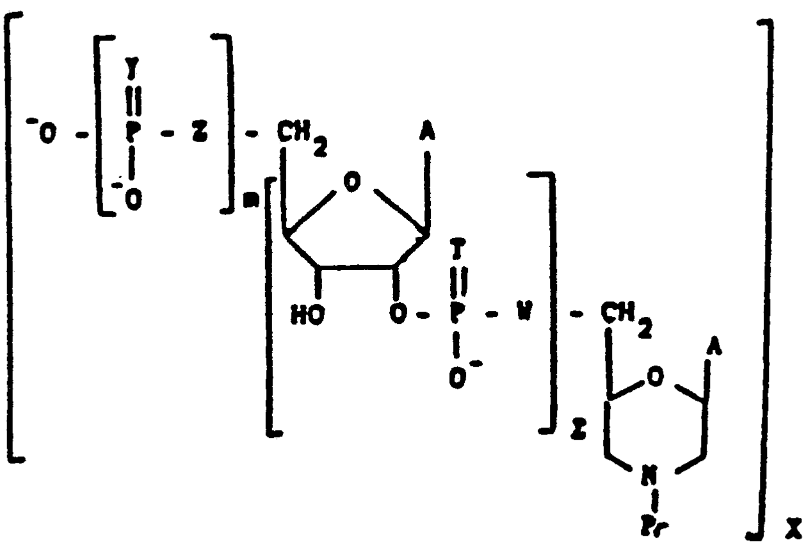

(I) ,

in welcher:

- Y und T, die gleich oder verschieden sind, unabhängig voneinander O, S, Se oder NH bedeuten;
- Z und W, die gleich oder verschieden sind, unabhängig voneinander O, S oder NH bedeuten;
- A Adenin oder eines seiner Derivate gemäß Anspruch 1 bedeutet;
- Σ eine ganze Zahl gleich n - 1 ist, wobei n eine ganze Zahl größer oder gleich 3 ist;
- m eine ganze Zahl großer oder gleich 0, und vorzugsweise größer oder gleich 2 ist;
- X eine ganze Zahl größer oder gleich 1, vorzugsweise 2 bis 10, ist;
- Pr eine wenigstens 5 Aminosäuren enthaltende Polypeptidkette ist, und das dem Ring:

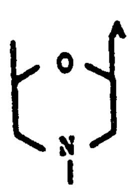

angehörende Stickstoffatom auch dem Ende einer Seitensequenz des Polypeptids Pr angehört.

3. Verfahren nach Anspruch 1 zur Herstellung von Kupplungsverbindungen der Formel (II):

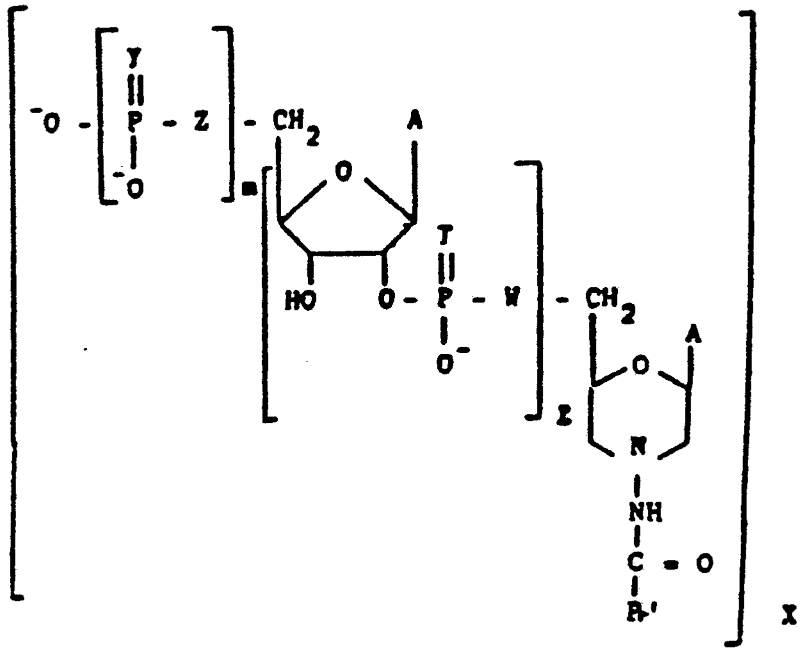

(II) ,

in welcher Y, Z, T, W, A, m, Σ und X die oben definierten Bedeutungen haben, Pr' eine wenigstens 5 Aminosäuren enthaltende Polypeptidkette bedeutet, und in welcher die Gruppe

$$-\underset{\underset{O}{\|}}{C}-NH-N-$$

im Formelbild von den endständigen

$$-\underset{\underset{O}{\|}}{C}-OH-$$

Gruppe des Polypeptids und/oder einer freien

$$-\underset{\underset{O}{\|}}{C}-OH-$$

Gruppe von Aspartyl- und/oder Glutamylresten stammt.

4. Verfahren nach Anspruch 1 zur Herstellung von Kupplungsverbindungen der Formel (III):

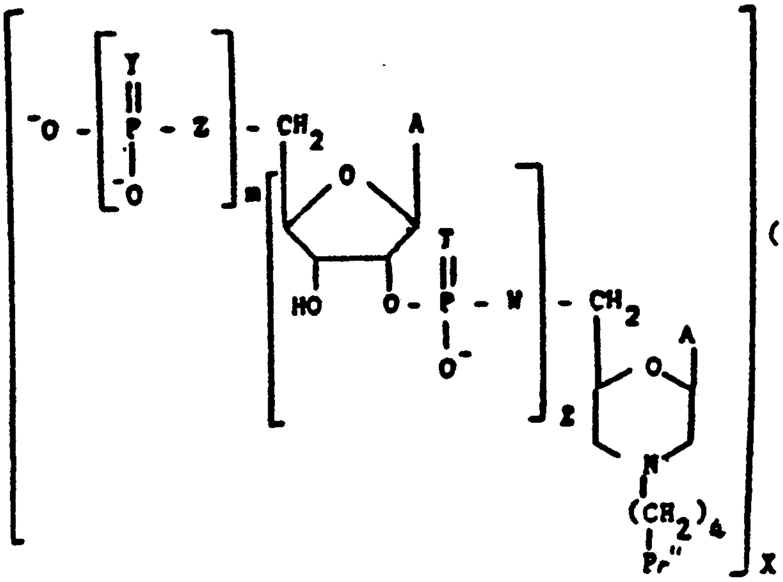

(III) ,

in welcher:

- Y und T, die gleich oder verschieden sind, unabhängig voneinander O, S, Se oder NH bedeuten;
- Z und W, die gleich oder verschieden sind, unabhängig voneinander O, S oder NH bedeuten;
- A Adenin oder eines seiner Derivate nach Anspruch 1 bedeutet;
- Σ eine ganze Zahl gleich n - 1 ist, wobei n eine ganze Zahl größer oder gleich 3 ist;
- m eine ganze Zahl größer oder gleich 0, und vorzugsweise großer oder gleich 2 ist;
- X eine ganze Zahl größer oder gleich 1, vorzugsweise 2 bis 10, ist;
- Pr'' eine wenigstens 5 Aminosäuren enthaltende Polypeptidkette ist; und in welcher die in der Formel aufscheinende Gruppe $-(CH_2)_4-$ der in der Konstitution der Polypeptidkette Pr'' vorliegt.

5. Verfahren nach Anspruch 1 zur Herstellung von Kupplungsverbindungen der Formel (IV):

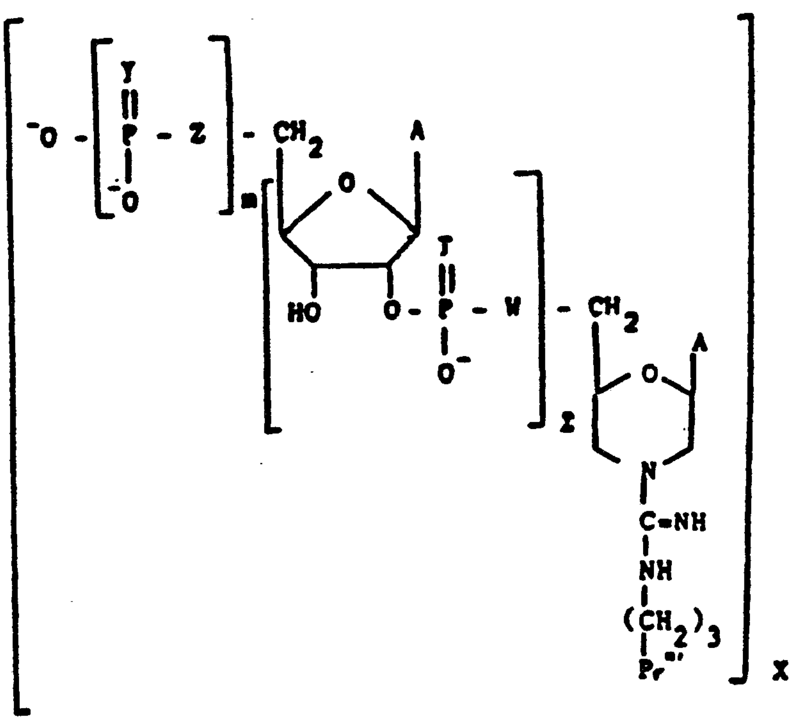

(IV) ,

in welcher Y, T, Z, W, A, Σ, n, X und m die in Anspruch 1 angegebene Bedeutung haben, Pr'' eine wenigstens 5 Aminosäuren enthaltende Peptidkette ist, und das dem Ring:

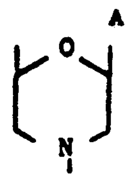

angehörende Stickstoffatom auch dem Ende der Seitensequenz eines Arginylrestes angehört, der in der Konstitution der Polypeptidkette Pr''' vorliegt.

6. Verfahren nach Anspruch 1 zur Herstellung von Kupplungsverbindungen der Formel (V):

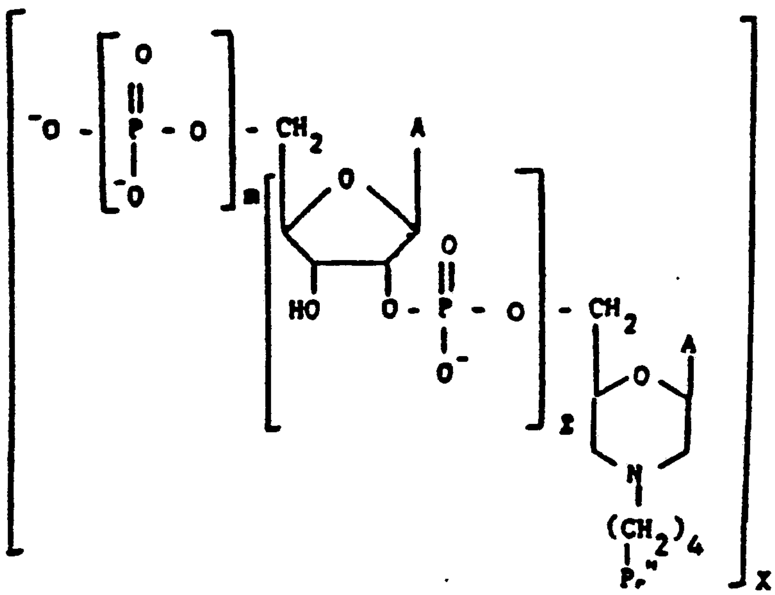

(V) ,

in welcher:

- A Adenin oder eines seiner Derivate gemäß Anspruch 1 bedeutet:
- Σ eine ganze Zahl gleich n - 1 ist, wobei n eine ganze Zahl größer oder gleich 3 ist;
- m eine ganze Zahl größer oder gleich 0, und vorzugsweise größer oder gleich 1, ist;
- X eine ganze Zahl gleich 1, oder vorzugsweise 2 bis 10, ist;
- Pr'' eine wenigstens 5 Aminosäuren enthaltende Peptidkette ist; und in welcher die in der Formel aufscheinende Gruppe $-(CH_2)_4-$ der Seitensequenz eines Lysylrestes angehört, welcher in der Konstitution der Polypeptidkette Pr'' vorliegt.

7. Verfahren nach Anspruch 1 zur Herstellung von Kupplungsverbindungen der folgenden Formel:

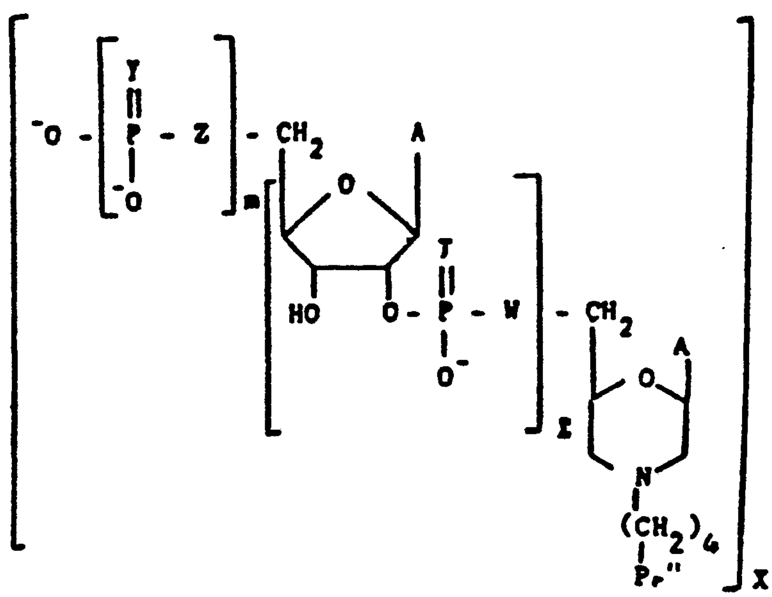

(III),

in welcher:

- Y und T, die gleich oder verschieden sind, unabhängig voneinander O, S, Se oder NH bedeuten;
- Z und W die gleich oder verschieden sind unabhängig voneinander O, S oder NH bedeuten;
- A Adenin oder eines seiner Derivate gemäß Anspruch 2 bedeutet;
- Σ eine ganze Zahl gleich n - 1 ist, wobei n eine ganze Zahl größer oder gleich 3 ist;
- m eine ganze Zahl größer oder gleich 0, und vorzugsweise größer oder gleich 1 ist;
- X eine ganze Zahl gleich 1, oder vorzugsweise 2 bis 10, ist;
- Pr'' eine wenigstens 5 Aminosäuren enthaltende Peptidkette ist; und in welcher die in der Formel aufscheinende Gruppe $(CH_2)_4$- der Seitensequenz eines in der Konstitution der Polypeptidkette Pr'' vorliegenden Lysylrestes angehört;

mit der Maßgabe, daß wenigstens einer der Reste Y und Z von Sauerstoff verschieden ist.

8. Verfahren nach Anspruch 1 zur Herstellung von Kupplungsverbindungen der folgenden Formeln:

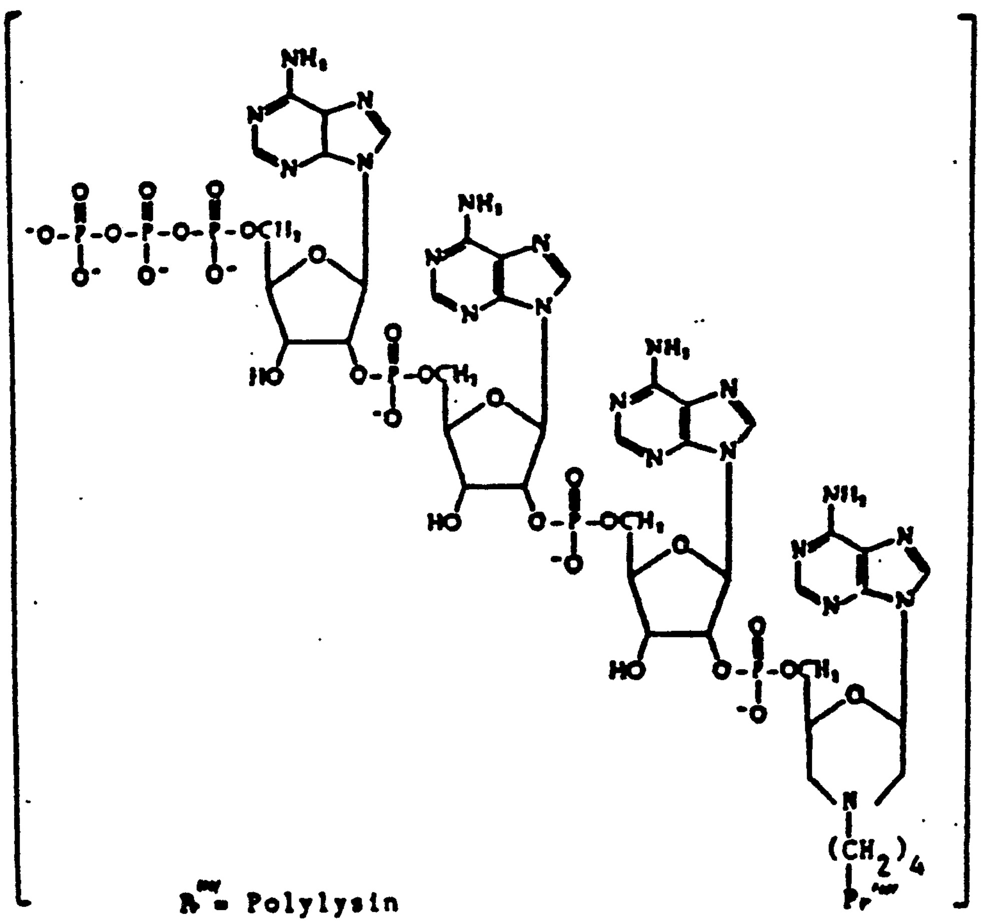

Pr''' = Polylysin

(XIII)

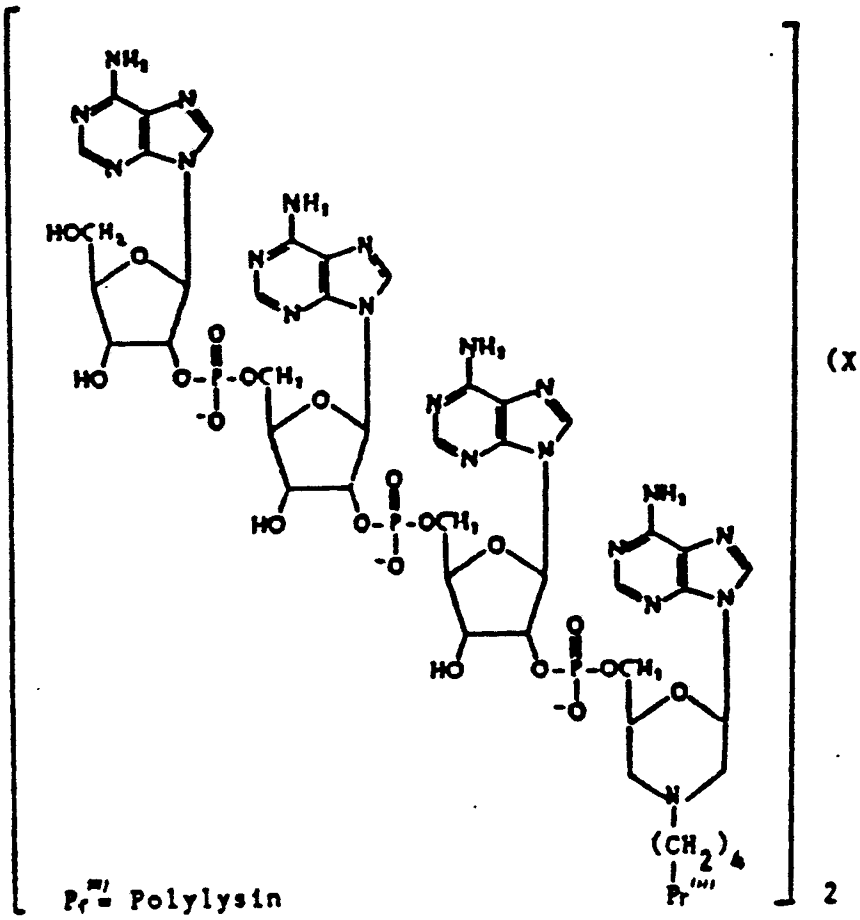

P_r''' = Polylysin

(XIV)

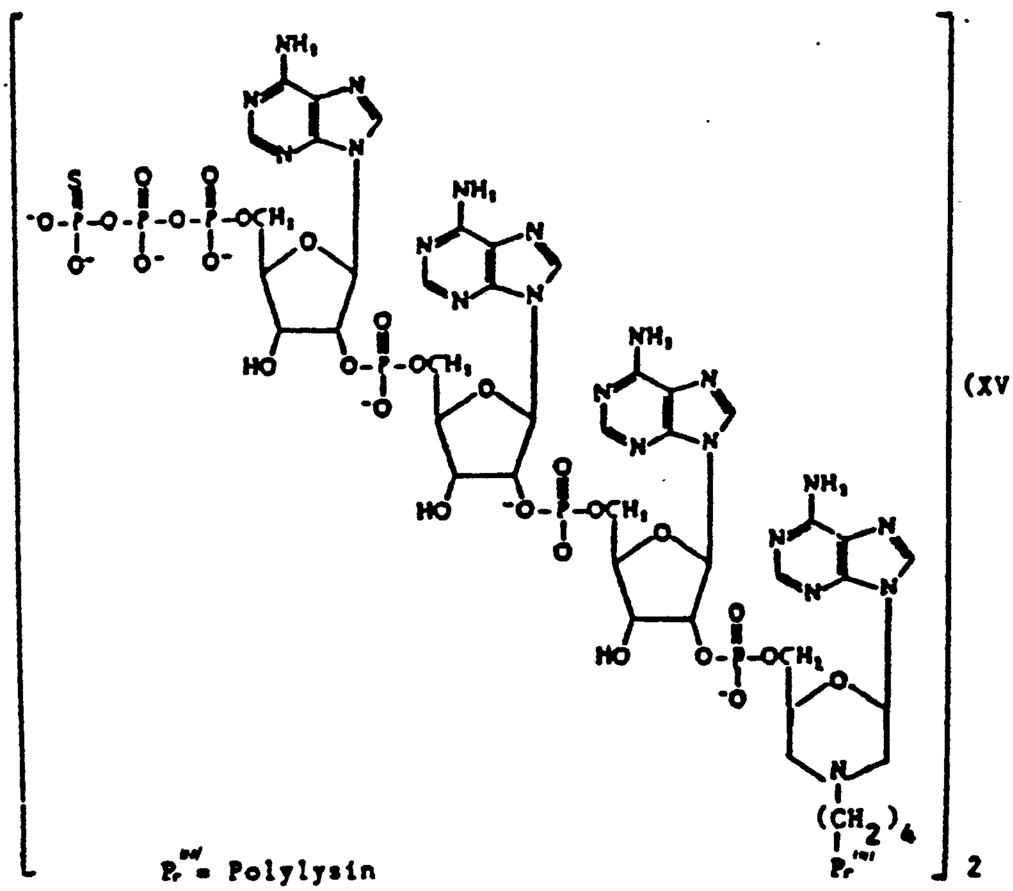

P_r''' = Polylysin

(XV)

9. Verfahren zur Herstellung von Oligonukleotiden nach den Ansprüchen 1 bis 8, dadurch gekennzeichnet, daß die enzymatische Synthese der Verbindungen der Formel (Ibis) umfaßt:
- die Polymerisation einer Verbindung der folgenden Formel (Iquater):

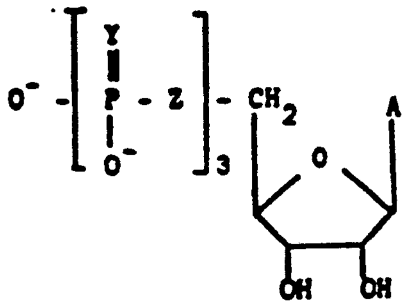

(Iquater),

in welcher:
- Y für O, S, Se oder NH steht;
- Z für O, S oder NH steht;
wobei wenigstens einer der Reste Y und Z von Sauerstoff verschieden sein kann; und
- A die in den Ansprüchen 1 bis 7 angegebenen Bedeutungen hat.

10. Verfahren zur Herstellung von Oligonukleotiden nach den Ansprüchen 1 bis 8, dadurch gekennzeichnet, daß es umfaßt:
die Polymerisation einer Verbindung der folgenden Formel (XVIbis):

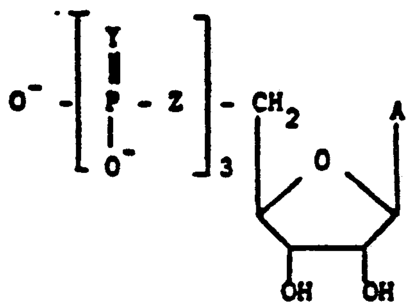

(XVIbis) ,

in welcher:
- Y für O, S, Se oder NH steht;
- Z für O, S oder NH steht;
- Y und Z nicht gleichzeitig Sauerstoff bedeuten; und
- A die in den vorhergehenden Ansprüchen angegebenen Bedeutungen hat,
zur Gewinnung der Verbindungen der folgenden Formel (XVI):

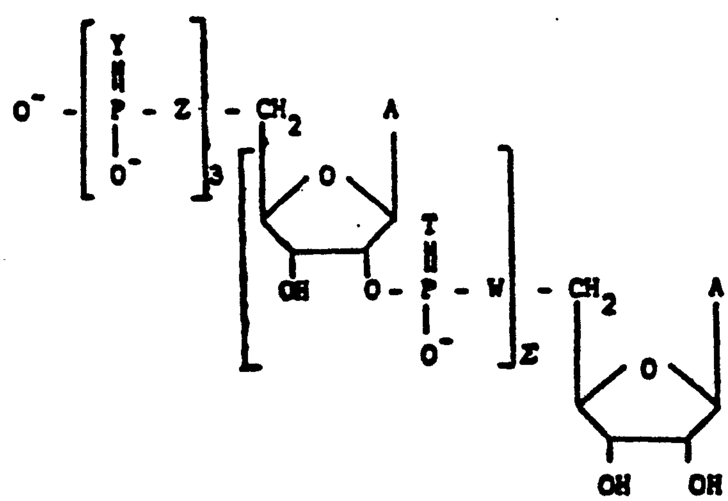

(XVI) ,

in welcher:

- Y und T, die gleich oder verschieden sind, O, S, Se oder NH bedeuten;
- Z und W, die gleich oder verschieden sind, O, S oder NH bedeuten;
- Y und Z nicht gleichzeitig Sauerstoff sind;
- Σ eine Zahl von 2 bis 9 ist; und
- A eine aus Adenin oder seinen Derivaten, insbesondere solchen der Formel:

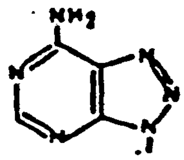
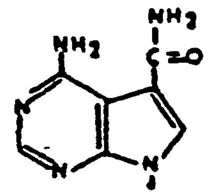
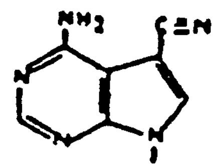
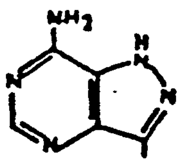
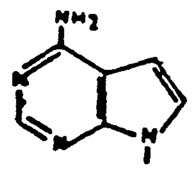
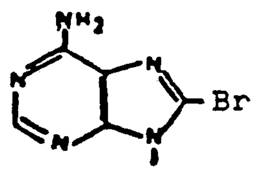
,

ausgewählte Base ist.

11. Verfahren zur Herstellung von Oligonukleotiden nach den Ansprüchen 1 bis 8, dadurch gekennzeichnet, daß es umfaßt:
die Polymerisation einer Verbindung der folgenden Formel (XVIIbis):

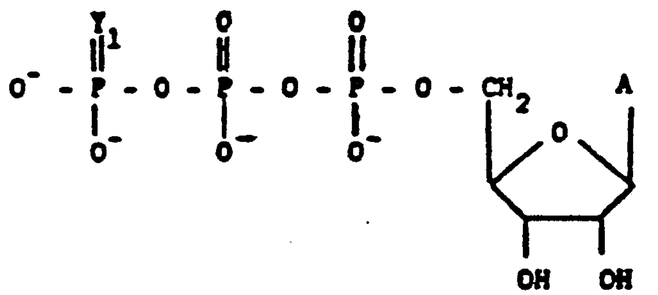
(XVIIbis) ,

in welcher
$Y_1$ für NH, Se oder S steht, und
A die in den vorhergehenden Ansprüchen angegebene Bedeutung hat, zur Gewinnung der Verbindungen der folgenden Formel (XVII):

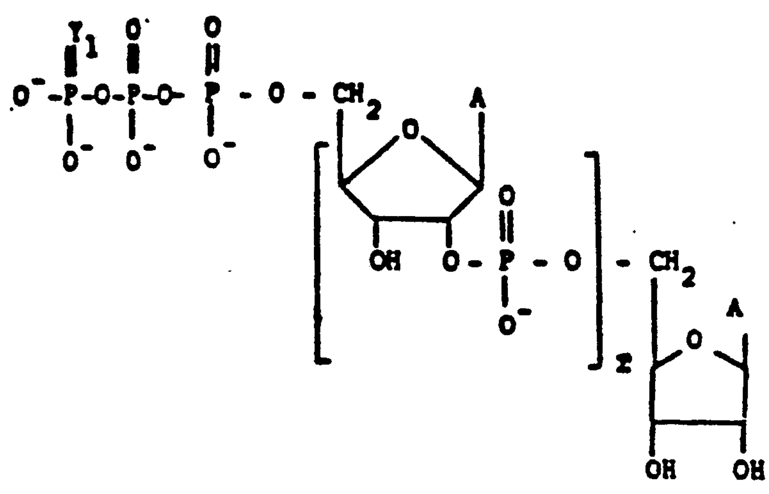
(XVII),

in welcher:
- $Y_1$ für NH, Se oder S steht;
- Σ eine ganze Zahl gleich n-1 ist, n 3 bis 10 beträgt, und
- A die in den vorhergehenden Ansprüchen angegebene Bedeutung hat.

12. Verfahren zur Herstellung von Oligonukleotiden nach den Ansprüchen 1 bis 8, dadurch gekennzeichnet, daß es umfaßt: die Polymerisation einer Verbindung der folgenden Formel (XVIIIbis):

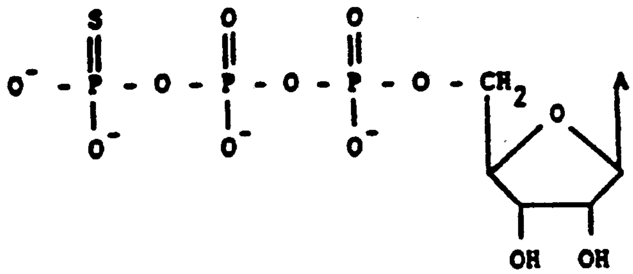

(XVIIIbis),

in welcher:
- A die in den vorhergehenden Ansprüchen angegebene Bedeutung hat,

zur Gewinnung von Verbindungen der folgenden Formel (XVIII):

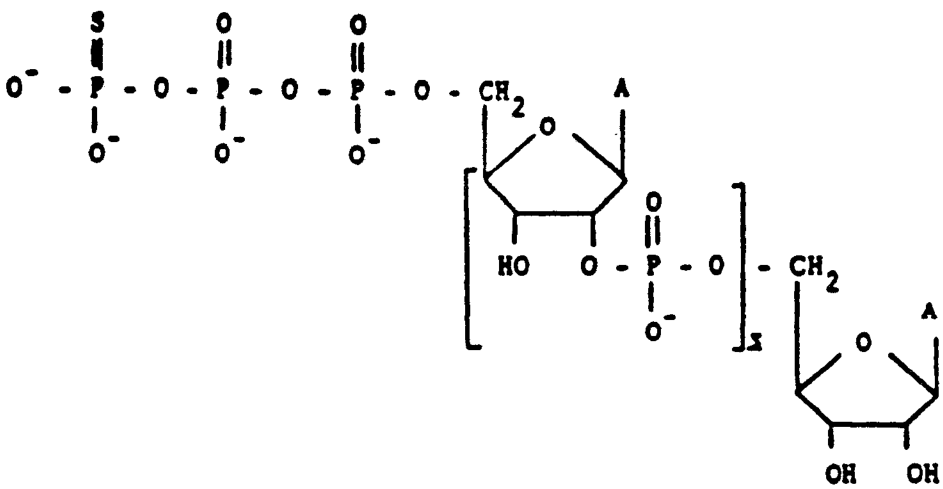

(XVIII)

,

in welcher:
- Σ die vorstehend angegebenen Bedeutungen hat; und
- A die vorstehend angegebene Bedeutung hat, und vorzugsweise Adenin bedeutet.

13. Verfahren zur Herstellung von Oligonukleotiden, dadurch gekennzeichnet, daß es umfaßt: die Polymerisation der Verbindung der folgenden Formel (XIXbis):

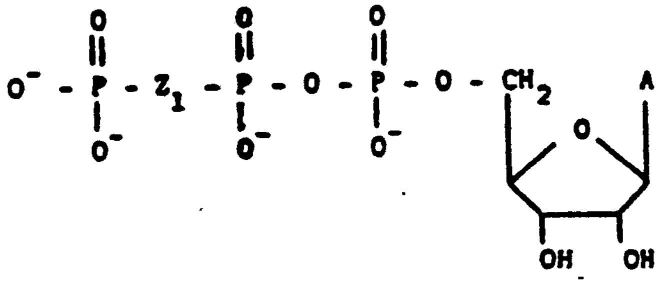

(XIXbis)

,

in welcher:
$Z_1$ für S oder NH steht, und
A die in den vorhergehenden Ansprüchen angegebene Bedeutung hat, zur Gewinnung von Verbindungen der folgenden Formel (XIX):

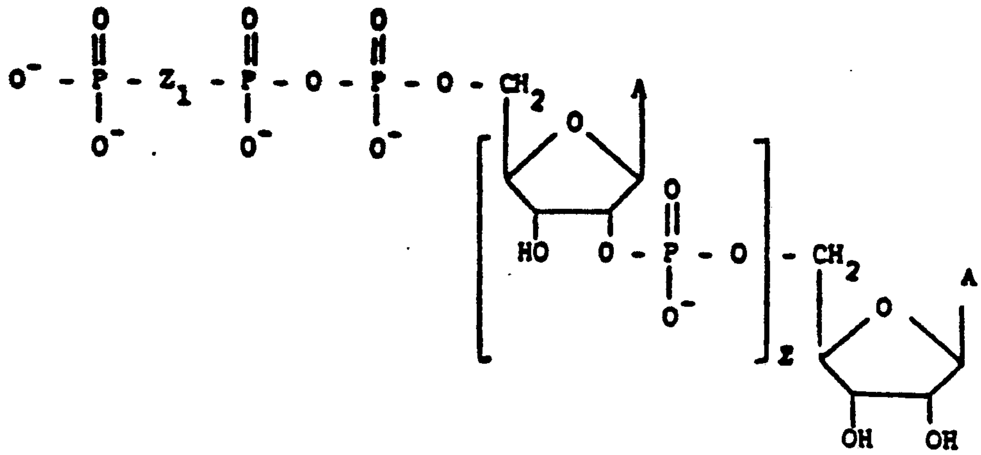

(XIX).

in welcher
- $Z_1$ für NH oder S steht;
- Σ eine ganze Zahl gleich n-1 ist, wobei n von 3 bis 10 variiert, und
- A die in den vorhergehenden Ansprüchen angegebene Bedeutung hat.

14. Verfahren zur Herstellung von Oligonukleotiden nach den Ansprüchen 1 bis 8, dadurch gekennzeichnet, daß es umfaßt:
die Polymerisation einer Verbindung der folgenden Formel (XXbis):

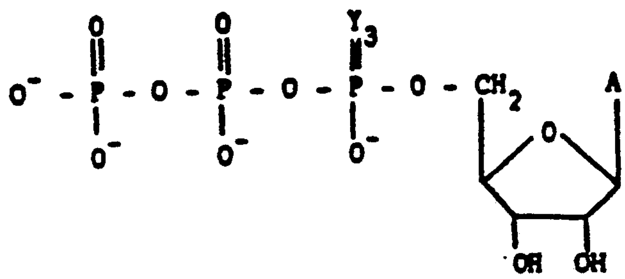

(XXbis) ,

in welcher:
- $Y_3$ für S, Se oder NH steht; und
- A die in den vorhergehenden Ansprüchen angegebenen Bedeutungen hat,
zur Gewinnung von Verbindungen der folgenden Formel (XX):

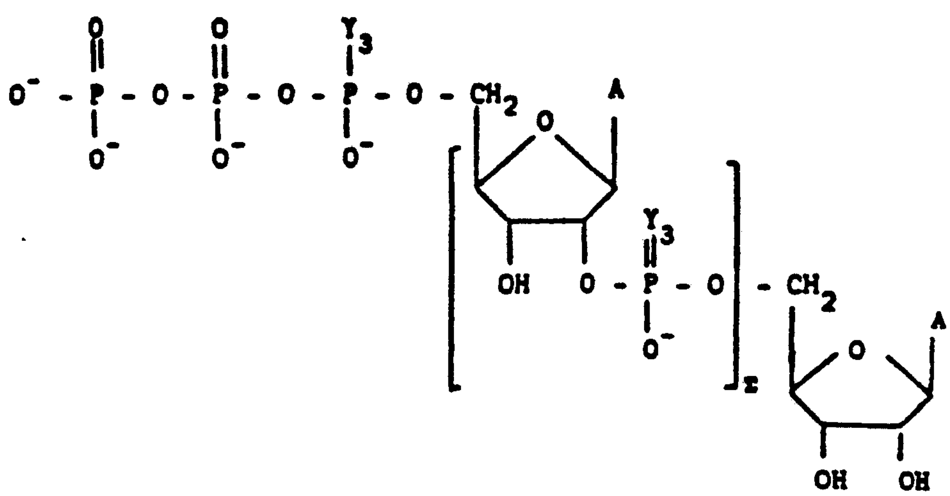

(XX) ,

in welcher:
- $Y_3$ für NH, S oder Se steht; und
- Σ und A die oben angegebenen Bedeutungen haben.

**Claims** for the Contracting States: BE, CH, DE, FR, GB, IT, LI, LU, NL, SE

1. Coupling compounds represented by the formula (I) defined below:

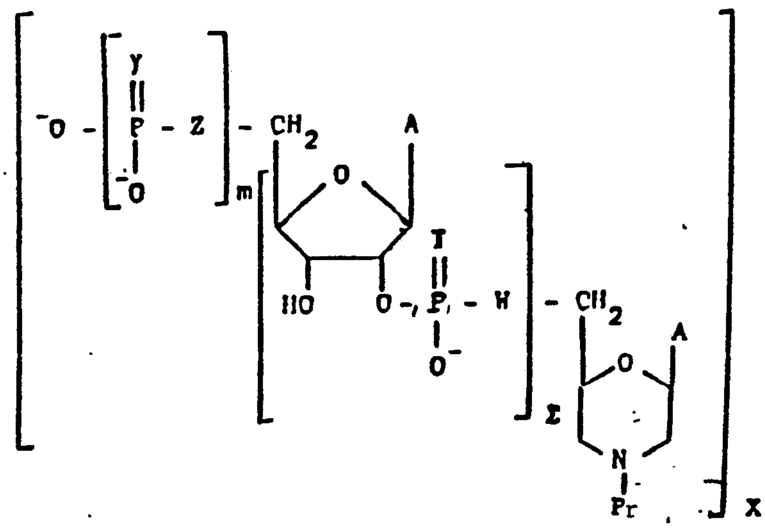

(I)

in which:
- Y and T, identical or different, represent independently of one another O, S, Se or NH;
- Z and W, identical or different, represent independently of one another O, S, or NH;
- A represents adenine or one of its derivatives, particularly those of the formula:

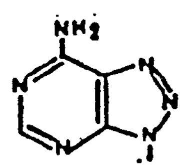
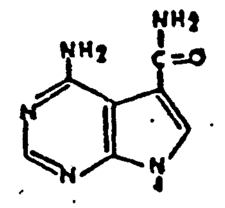
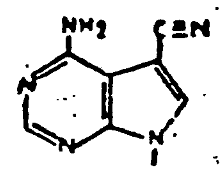

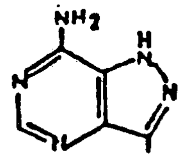
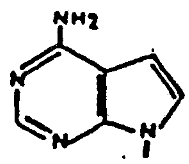
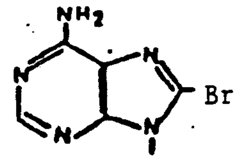

- Σ is a whole number equal to n - 1, n being a whole number greater than or equal to 3;
- m is a whole number greater than orequalto 0 and preferably greater than or equal to 2;
- X is a whole number greater than or equal to 1, preferably comprised from 2 to 10;
- Pr is a polypeptide chain comprising at least 5 amino acids, and the nitrogen atom belonging to the ring:

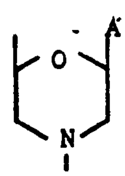

belonging also to the end of a lateral sequence of the polypeptide Pr and/or belonging to a group

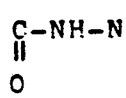

coming from a COOH group modified by hydrazine.

2. Coupling compounds according to Claim 1 of the formula:

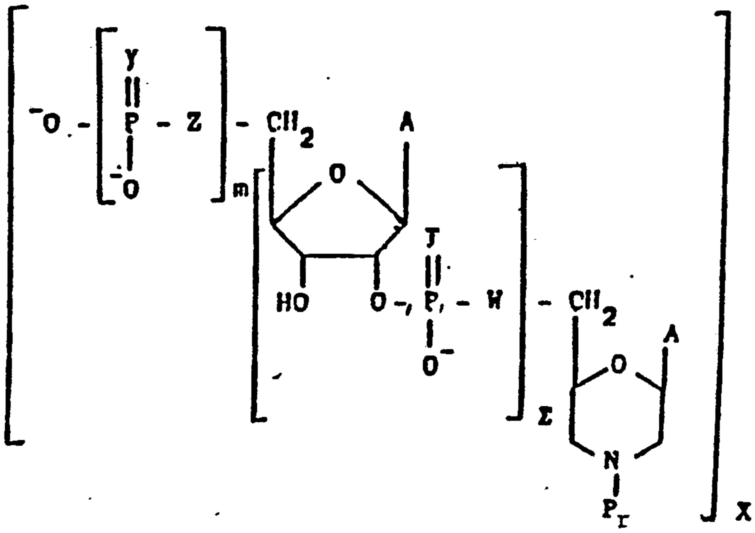

(I)

in which:
- Y and T, identical or different represent independently of one another O, S, Se or NH;

Z and W, identical or different, represent independently of one another O, S or NH;
- A represents adenine or one of its derivatives according to Claim 1;
- Σ is a whole number equal to n - 1, n being a whole number greater than or equal to 3;
- m is a whole number greater than or equal to 0 and preferably greater than or equal to 2;
- X is a whole number greater than or equal to 1, preferably comprised from 2 to 10;
- Pr is a polypeptide chain comprising at least 5 amino acids, and the nitrogen atom belonging to the ring:

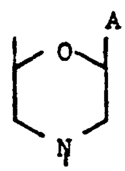

belonging also to the end of a lateral sequence of the polypeptide Pr.

3. Coupling compounds according to Claim 1 of formula (II):

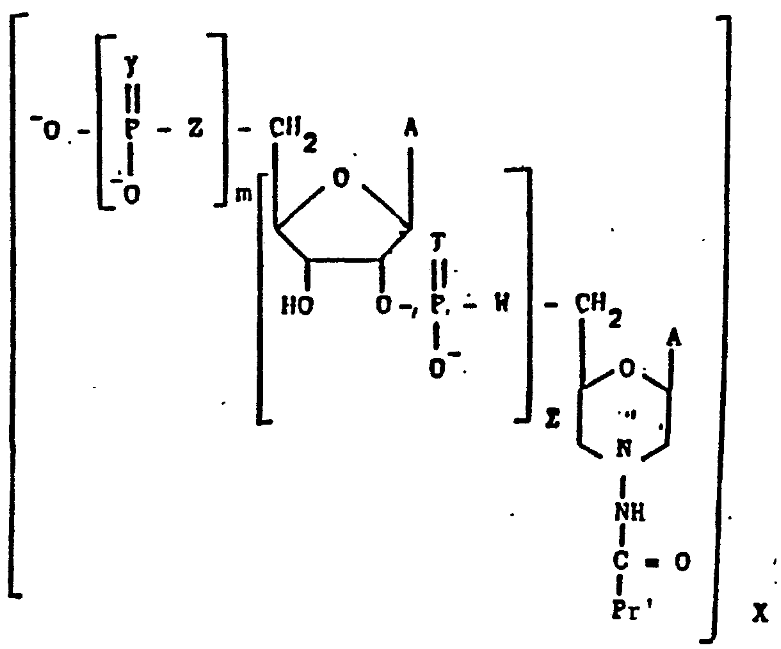

(II)

in which Y, Z, T, W, Σ, X, m and A have the abovedefined meanings, Pr' represents a polypeptide chain comprising at least 5 amino acids, and in which the

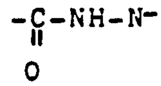

group shown in the figure comes from the terminal

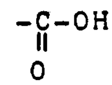

group of the polypeptide and/or from a free

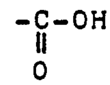

group of the aspartyl and/or glutamyl residues.

4. Coupling compounds according to Claim 1 of formula (III).

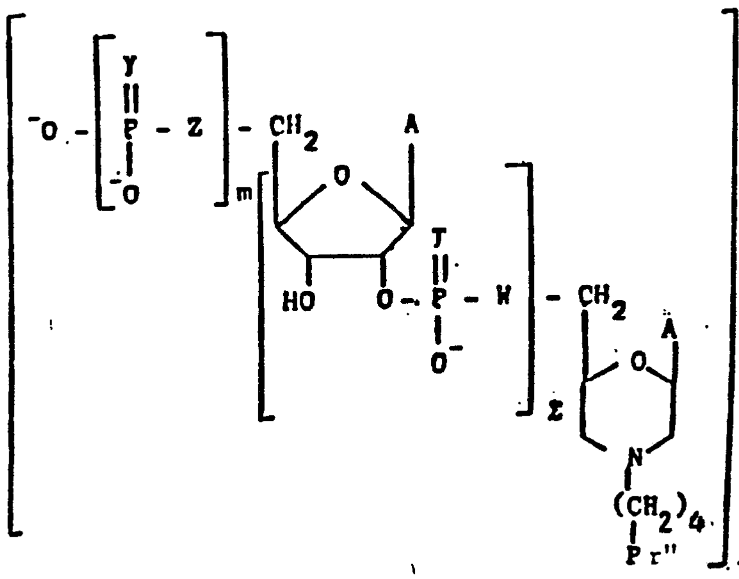

(III)

in which:

- Y and T, identical or different, represent independently of one another O, S, Se or NH;
- Z and W, identical or different, represent independently of one another O, S, or NH;
- A represents adenine or one of its derivatives according to Claim 1;
- Σ is a whole number equal to n - 1, n being a whole number greater than or equal to 3;
- X is a whole number equal to or greater than 1, preferably comprised from 2 to 10;
- m is a whole number greater than or equal to 0 and preferably greater than or equal to 2;
- Pr is a polypeptide chain comprising at least 5 amino acids and in which the group $-(CH_2)_4-$ shown in the formula belongs to the lateral sequence of a lysyl residue entering into the constitution of the Pr'' polypeptide chain.

5. Coupling compounds according to Claim 1 of formula (IV):

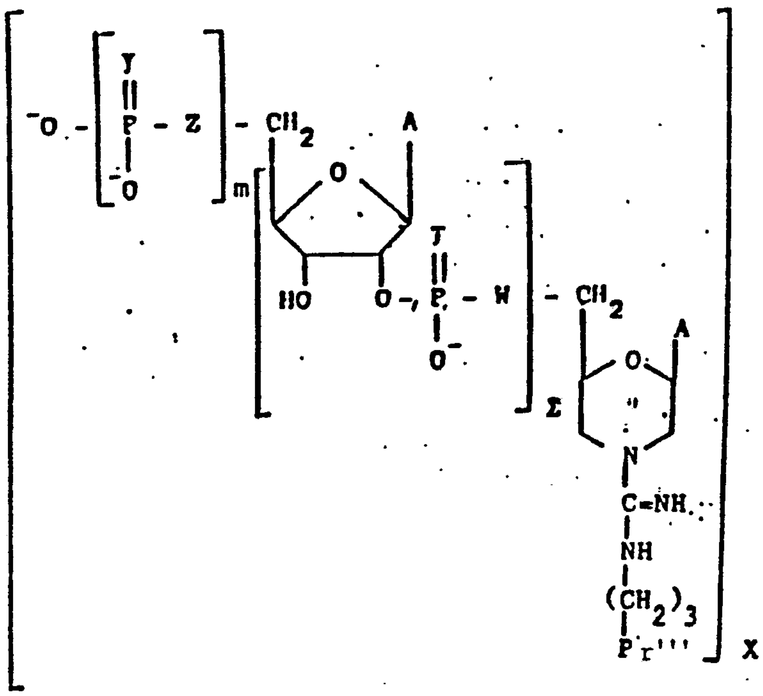

(IV)

in which Y, T, Z, W, A, Σ, n, X, m have the meanings indicated in Claim 1, Pr''' is a peptide chain comprising at least 5 amino acids and the nitrogen atom belonging to the ring:

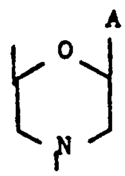

belongs also to the end of the lateral sequence of an arginyl residue entering into the constitution of the Pr'' polypeptide chain.

6. Coupling compounds according to Claim 1 of the formula (V):

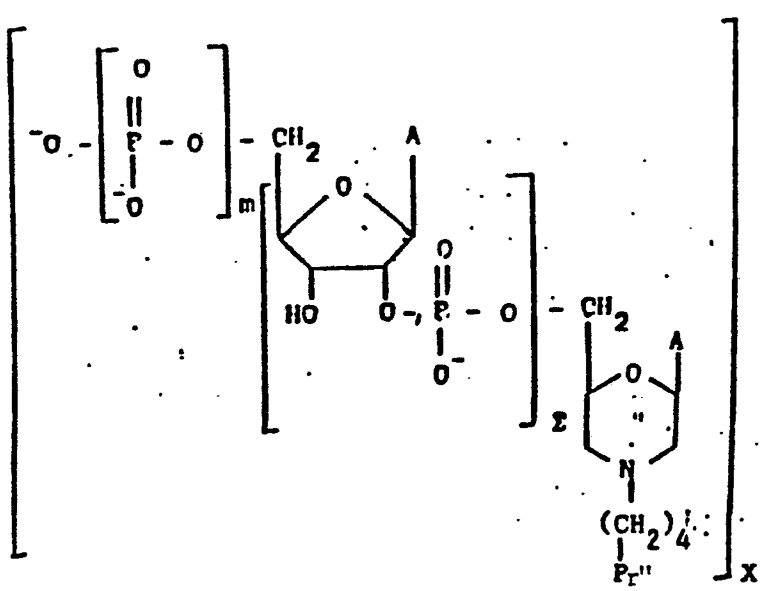

(V)

in which
- A represents adenine or one of its derivatives according to Claim 1;
- Σ is a whole number equal to n - 1, n being a whole number greater than or equal to 3;

- m is a whole number greater than or equal to 0 and preferably greater than or equal to 2;
- X is a whole number equal to 1, or preferably comprised from 2 to 10;
- Pr″ is a peptide chain comprising at least 5 amino acids and in which the $-(CH_2)_4-$ group shown in the formula belongs to the lateral sequence of a lysyl residue entering into the constitution of the Pr peptide chain.

7. Coupling compounds according to Claim 1 of the following formula:

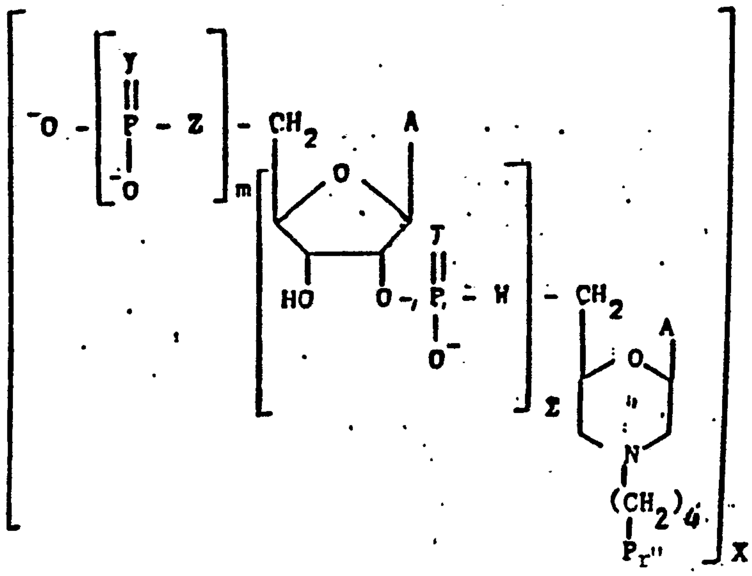

(III)

in which:

- Y and T, identical or different, represent independently of one another O, S, Se or NH;
- Z and W, identical or different, represent independently of one another O, S, or NH;
- A represents adenine or one of its derivatives according to Claim 2;

Σ is a whole number equal to n - 1, n being a whole number greater than or equal to 3;

- m is a whole number greater than or equal to 0 and preferably greater tham or equal to 1;
- X is a whole number equal to 1, or preferably comprised from 2 to 10;
- Pr″ is a peptide chain comprising at least 5 amino acids and in which the group $(CH_2)_4-$ shown in the formula belongs to the lateral sequence of a lysyl residue entering into the constitution of the Pr″ polypeptide chain; provided that one at least of the elements Y or Z is different from oxygen.

8. Coupling compounds according to Claim 1 of the following formulae:

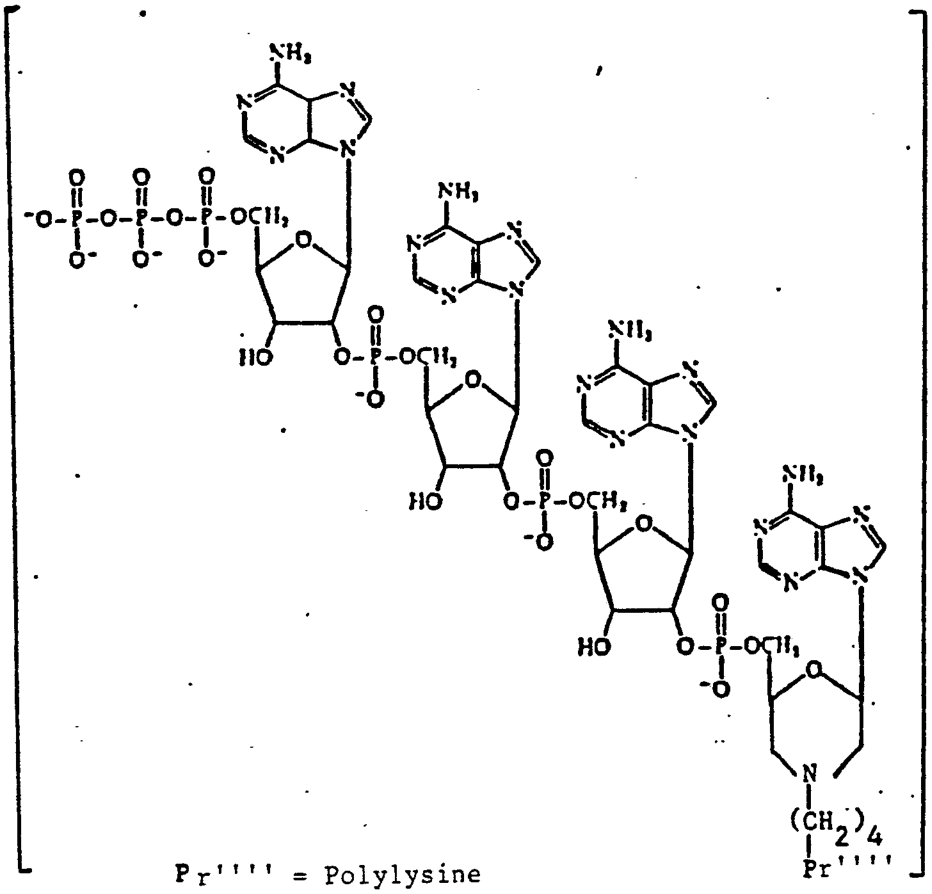

Pr'''' = Polylysine

2

(XIII)

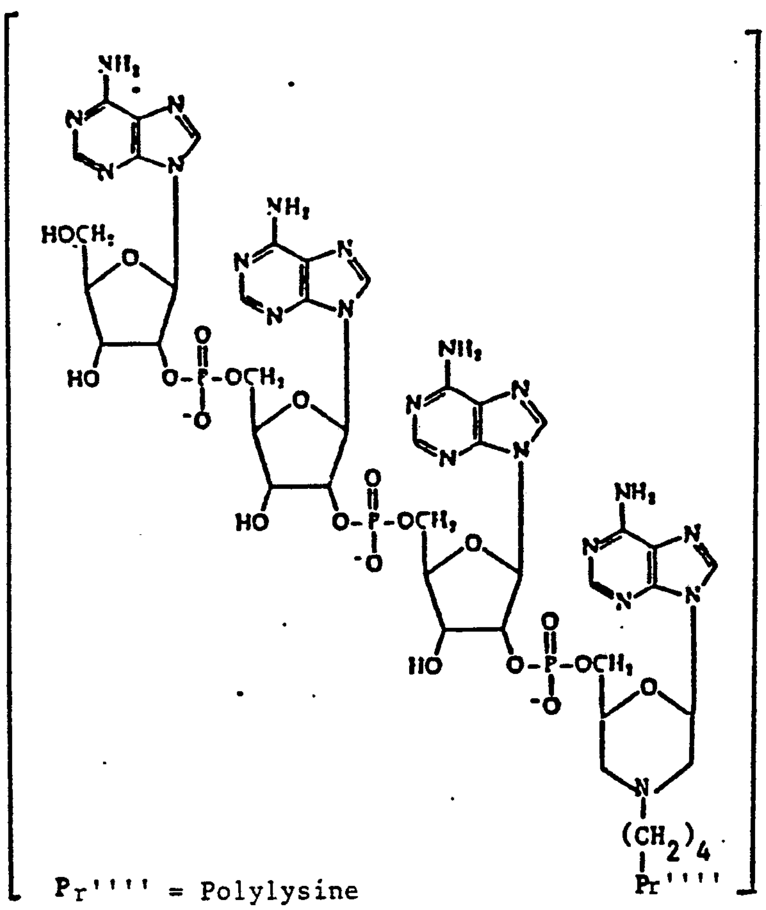

Pr'''' = Polylysine

2

(XIV)

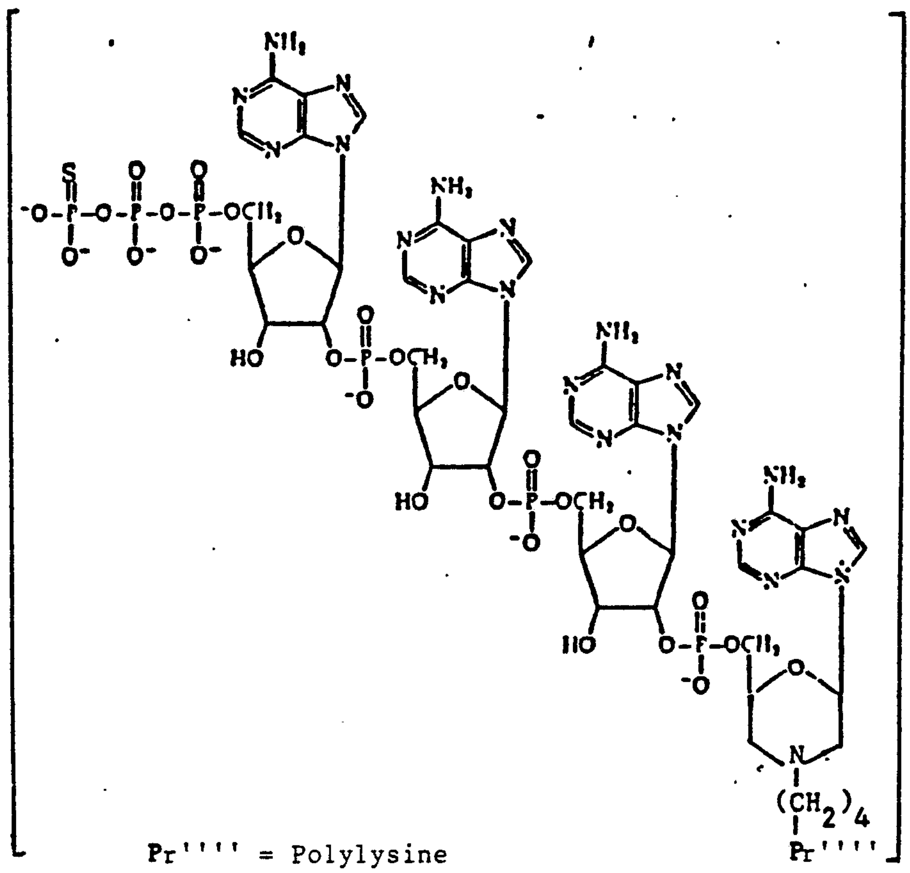

Pr'''' = Polylysine

(XV)

9. Process for the preparation of coupling compounds according to any one of the preceding Claims, characterised in that it comprises:

- the oxidation of the starting compound of the following formula (Ibis):

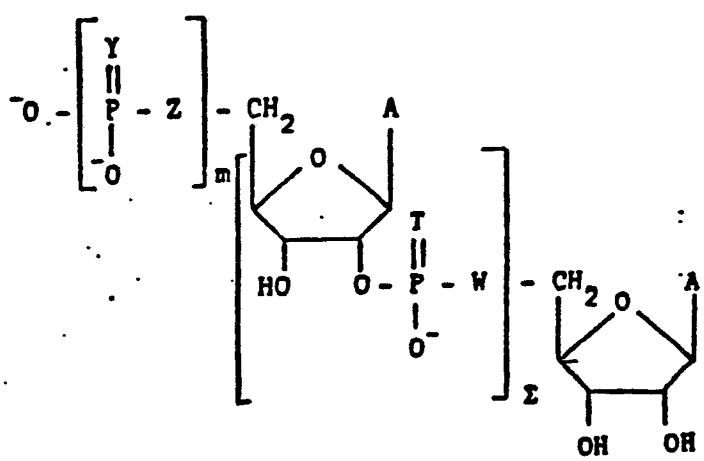

(Ibis)

in which Y, Z, T, W, A, m, Σ have the meanings indicated in one of Claims 1 to 8;

to introduce two aldehyde functions on to the carbons at the 2' and 3' positions of the last nucleoside unit and to obtain the compound of the following formula (Iter):

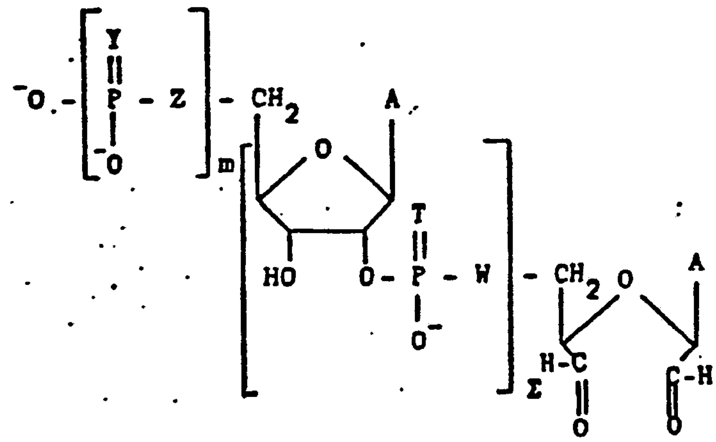

(Iter)

- the reductive alkylation of the compound (Iter) by a polypeptide $Pr\text{-}NH_2$ whose chain comprises at least one lateral sequence whose end is an amino group, particularly $NH_2$, this reaction taking place between the aldehyde functions of the compound (Iter) with the $NH_2$ group of the polypeptide to obtain the compound of formula (I):

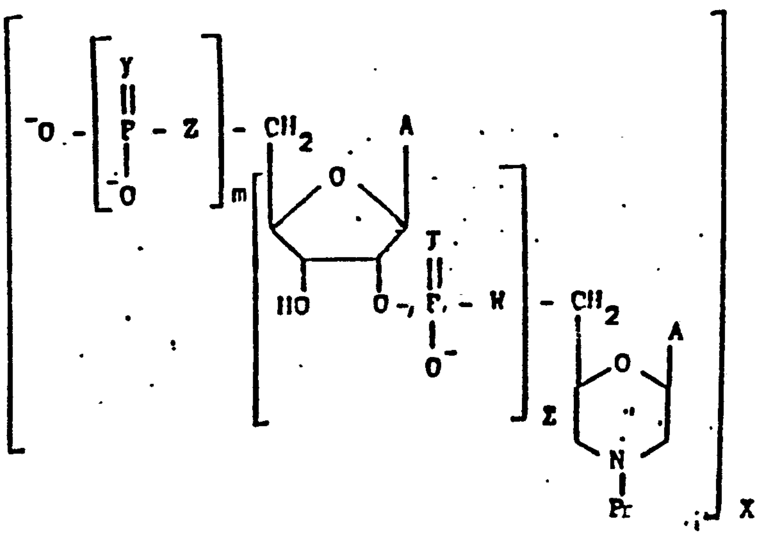

(I)

- the fractionation between the coupling compounds according to the invention and the uncoupled products particularly by molecular filtration.

**Claims** for the Contracting State: AT

1. Process for preparing coupling compounds of formula (I):

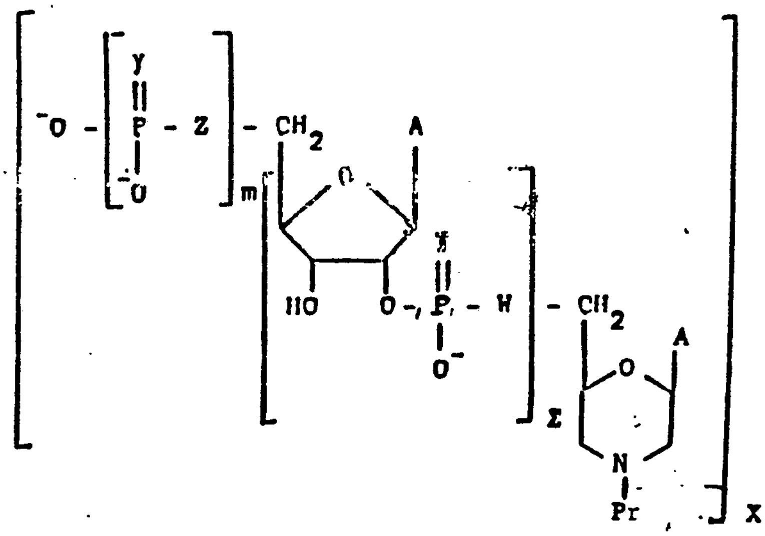

(I)

in which:

- Y and T, identical or different, represent independently of one another O, S, Se or NH;
- Z and W, identical or different, represent independently of one another O, S, or NH;
- A represents adenine or one of its derivatives, particularly those of the formula:

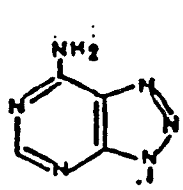
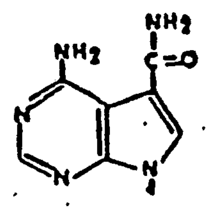
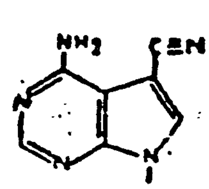
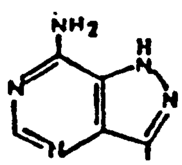
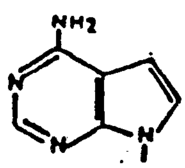
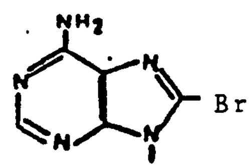

- Σ is a whole number equal to n - 1, n being a whole number greater than or equal to 3;
- m is a whole number greater than or equal to 0 and preferably greater than or equal to 2;
- X is a whole number greater than or equal to 1, preferably comprised from 2 to 10;
- Pr is a polypeptide chain comprising at least 5 amino acids, and the nitrogen atom belonging to the ring:

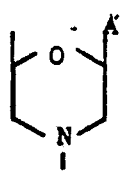

belonging also to the end of a lateral sequence of the polypeptide Pr and/or belonging to a group

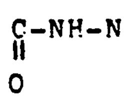

coming from a COOH group modified by hydrazine characterised in that it comprises:

- the oxidation of the starting compound of the following formula (Ibis):

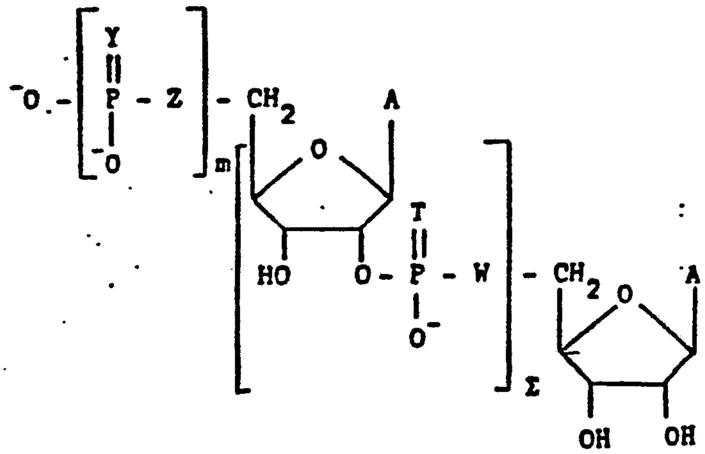

(Ibis)

in which Y, Z, T, W, A, m, Σ have the above mentioned meanings;

to introduce two aldehyde functions om to the carbons at the 2' and 3' positions of the last nucleoside unit and to obtain the compound of the following formula (Iter):

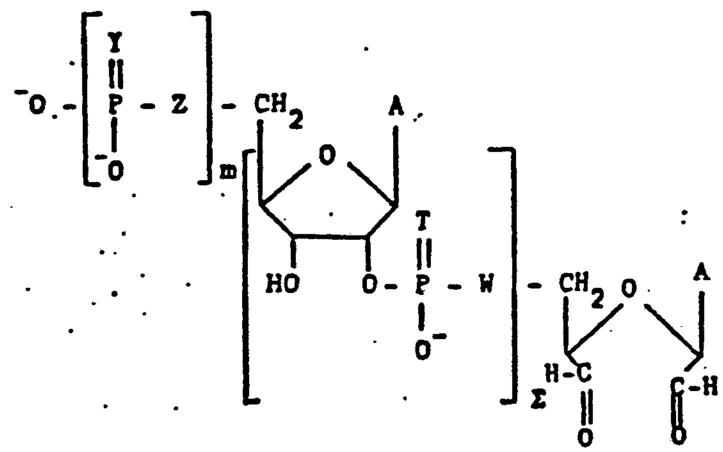

(Iter)

- the reductive alkylation of the compound (Iter) by a polypeptide Pr-$NH_2$ whose chain comprises at particularly $NH_2$, this reaction taking place between the aldehyde functions of the compound (Iter) with the $NH_2$ group of the polypeptide to obtain the compound of formula (I):

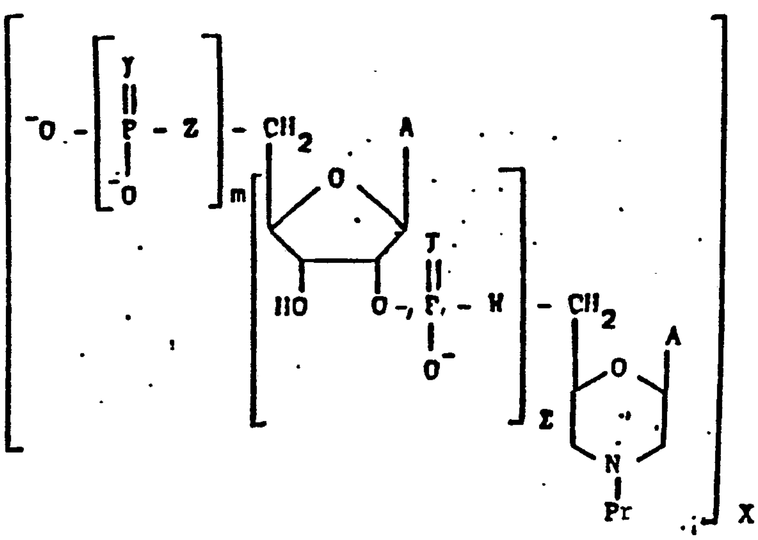

(I)

- the fractionation between the coupling compounds according to the invention and the uncoupled products particularly by molecular filtration.

2. Process for preparing, according to claim 1 coupling compounds of the formula:

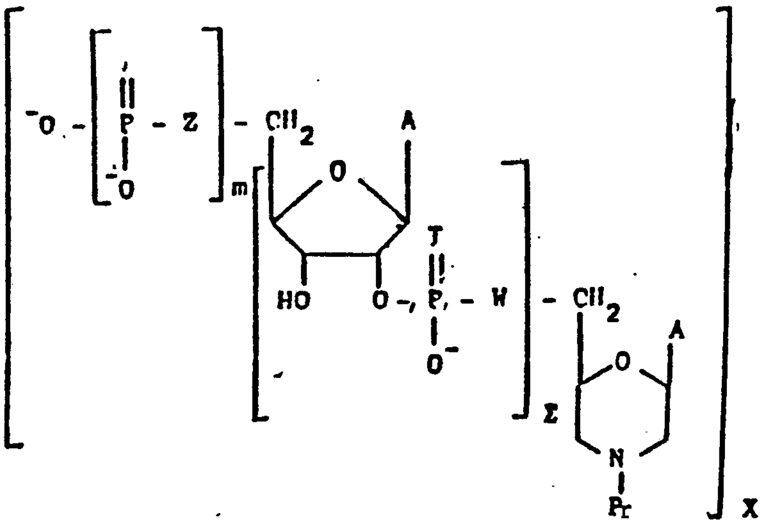

(I)

in which:

- Y and T, identical or different represent independently of one another O, S, Se or NH;
- Z and W, identical or different represent independently of one another O, S or NH;
- A represents adenine or one of its derivatives according to Claim 1;
- Σ is a whole number equal to n - 1, n being a whole number greater than or equal to 3;
- m is a whole number greater than or equal to 0 and preferably greater than or equal 2;
- X is a whole number greater than or equal to 1, preferably comprised from 2 to 10;
- Pr is a polypeptide chain comprising at least 5 amino acids, and the nitrogen atom belonging to the ring:

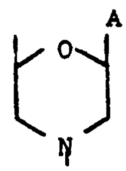

belonging also to the end of a lateral sequence of the polypeptide Pr.

3. Process for preparing, according to claim 1, coupling compounds of formula (II):

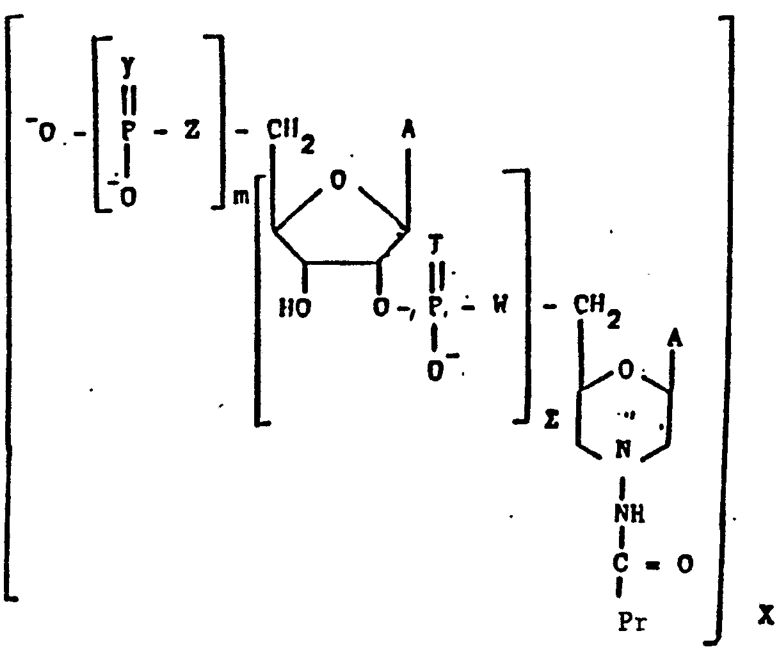

(II)

in which Y, Z, T, W, Σ, X, m and A have the abovedefined meanings, Pr' represents a polypeptide chain comprising at least 5 amino acids, and in which the

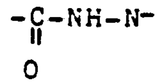

group shown in the figure comes from the terminal

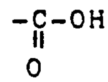

group of the polypeptide and/or from a free

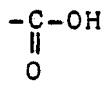

group of the aspartyl and/or glutamyl residues.

4. Process for preparing, according to claim 1, coupling compounds of formula (III):

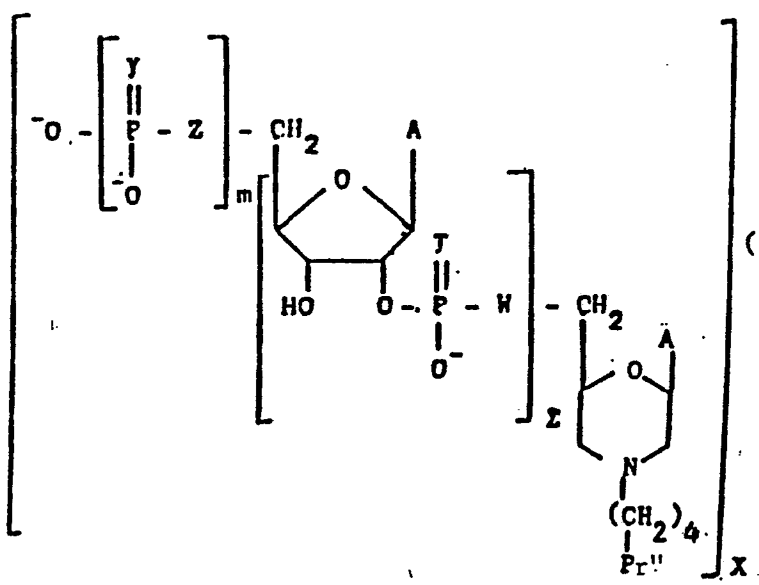

(III)

in which:

- Y and T, identical or different, represent independently of one another O, S, Se or NH;
- Z and W, identical or different, represent independently of one another O, S, or NH;
- A represents adenine or one of its derivatives according to Claim 1;
- Σ is a whole number equal to n - 1, n being a whole number greater than or equal to 3;
- X is a whole number equal to or greater than 1, preferably comprised from 2 to 10;
- m is a whole number greater than or equal to 0 and preferably greater than or equal to 2;
- Pr'' is a polypeptide chain comprising at least 5 amino acids and in which the group $-(CH_2)_4-$ shown in the formula belongs to the lateral sequence of a lysyl residue entering into the constitution of the Pr'' polypeptide chain.

5. Process for preparing, according to claim 1, coupling compounds of formula (IV):

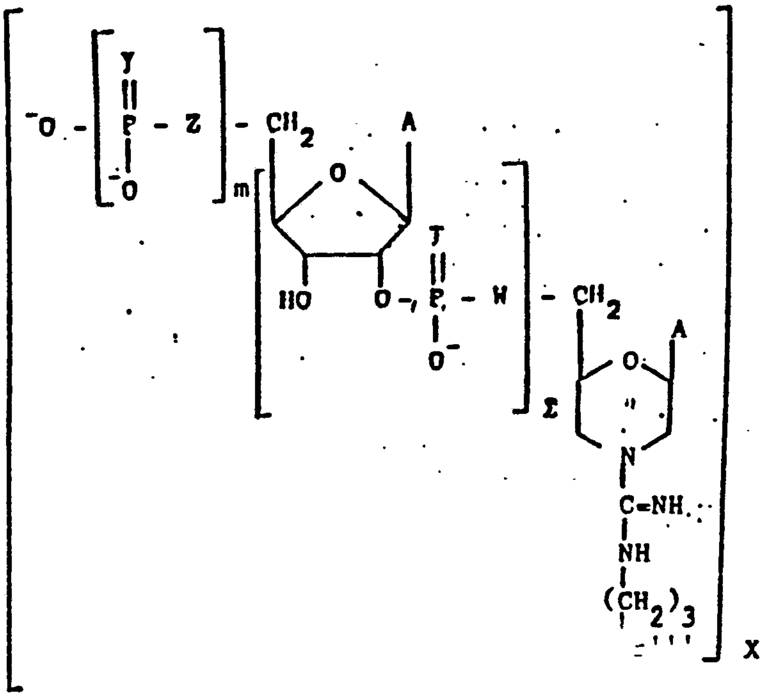

(IV)

in which Y, T, Z, W, A, Σ, n, X, m have the meanings indicated in Claim 1, Pr''' is a peptide chain comprising at least 5 amino acids and the nitrogen atom belonging to the ring:

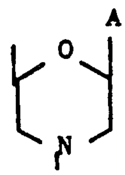

belongs also to the end of the lateral sequence of an arginyl residue entering into the constitution of the Pr''' polypeptide chain.

6. Process for preparing, according to claim 1, coupling compounds of the formula (V):

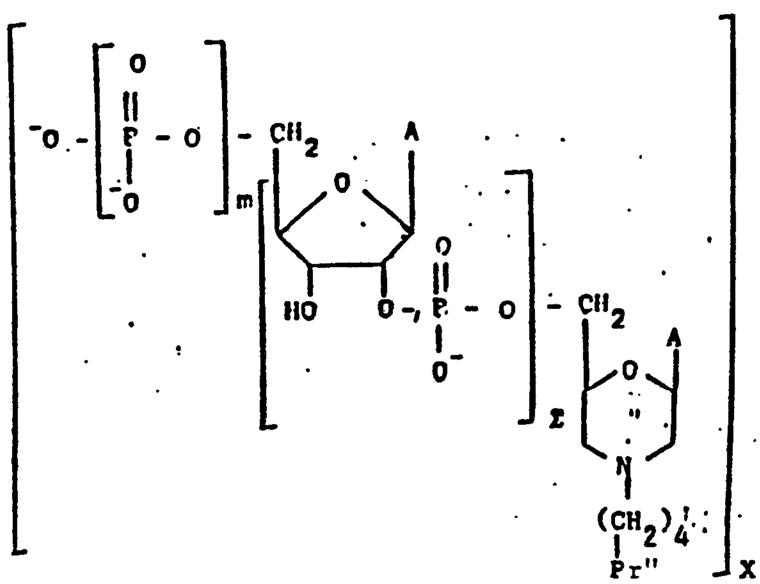

(V)

in which:

- A represents adenine or one of its derivatives according to Claim 1;
- Σ is a whole number equal to n - 1, n being a whole number greater than or equal to 3;
- m is a whole number greater than or equal to 0 and preferably greater than or equal to 1;

- X is a whole number equal to 1, or preferably comprised from 2 to 10;
- Pr'' is a peptide chain comprising at least 5 amino acids and in which the $-(CH_2)_4-$ group shown in the formula belongs to the lateral sequence of a lysyl residue entering into the constitution of the Pr'' polypeptide chain.

7. Process for preparing, according to claim 1, coupling compounds of the following formula:

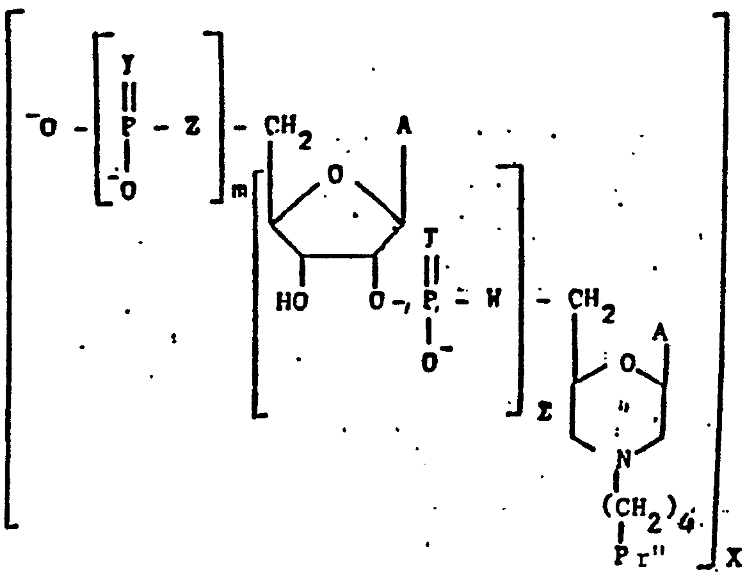

(III)

in which:

- Y and T, identical or different, represent independently of one another O, S, Se or NH;
- Z and W, identical or different, represent independently of one another O, S, or NH;
- A represents adenine or one of its derivatives according to Claim 2;
- Σ is a whole number equal to n - 1, n being a whole number greater than or equal to 3;
- m is a whole number greater than or equal to 0 and preferably greater than or equal to 1;
- X is a whole number equal to 1, or preferably comprised from 2 to 10;
- Pr'' is a peptide chain comprising at least 5 amino acids and in which the group $(CH_2)_4-$ shown in the formula belongs to the lateral sequence of a lysyl residue entering into the constitution of the Pr'' polypeptide chain;

provided that one at least of the elements Y or Z is different from oxygen.

8. Process for preparing, according to claim 1, coupling compounds of the following formulae:

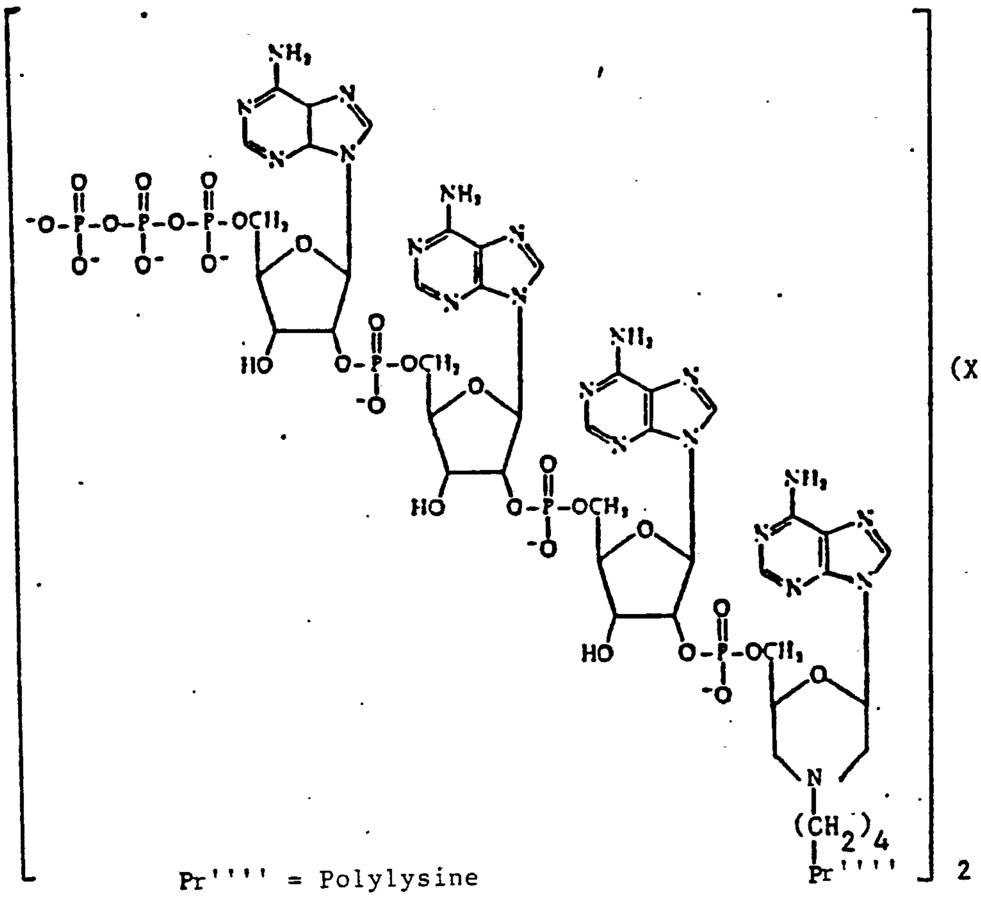

(XIII)

Pr'''' = Polylysine

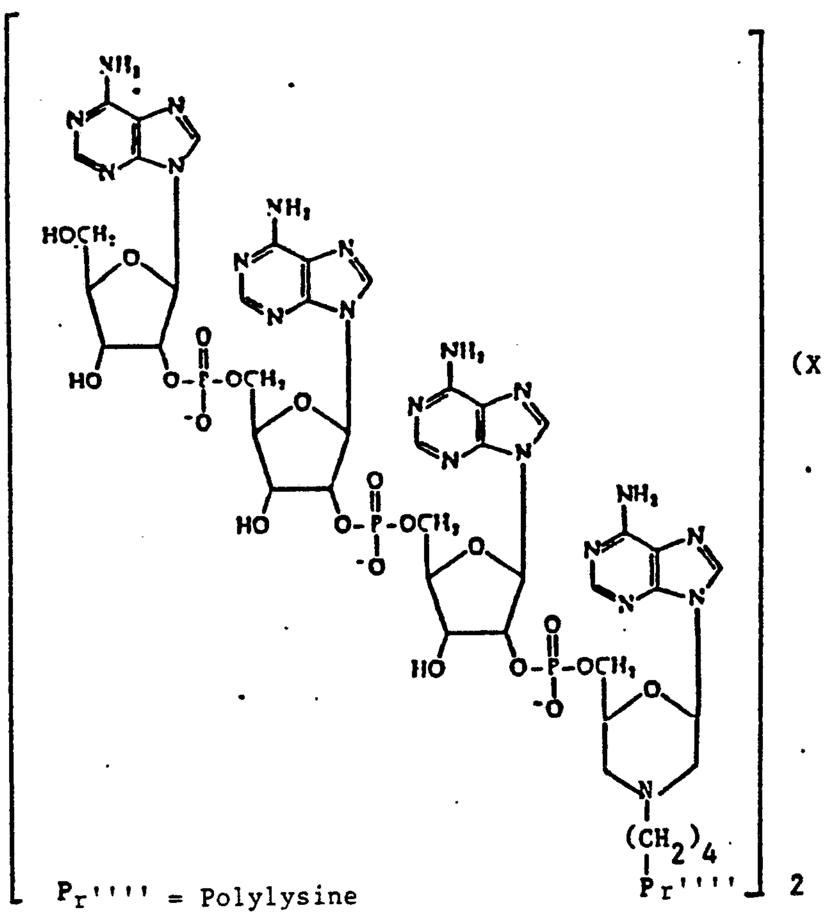

(XIV)

Pr'''' = Polylysine

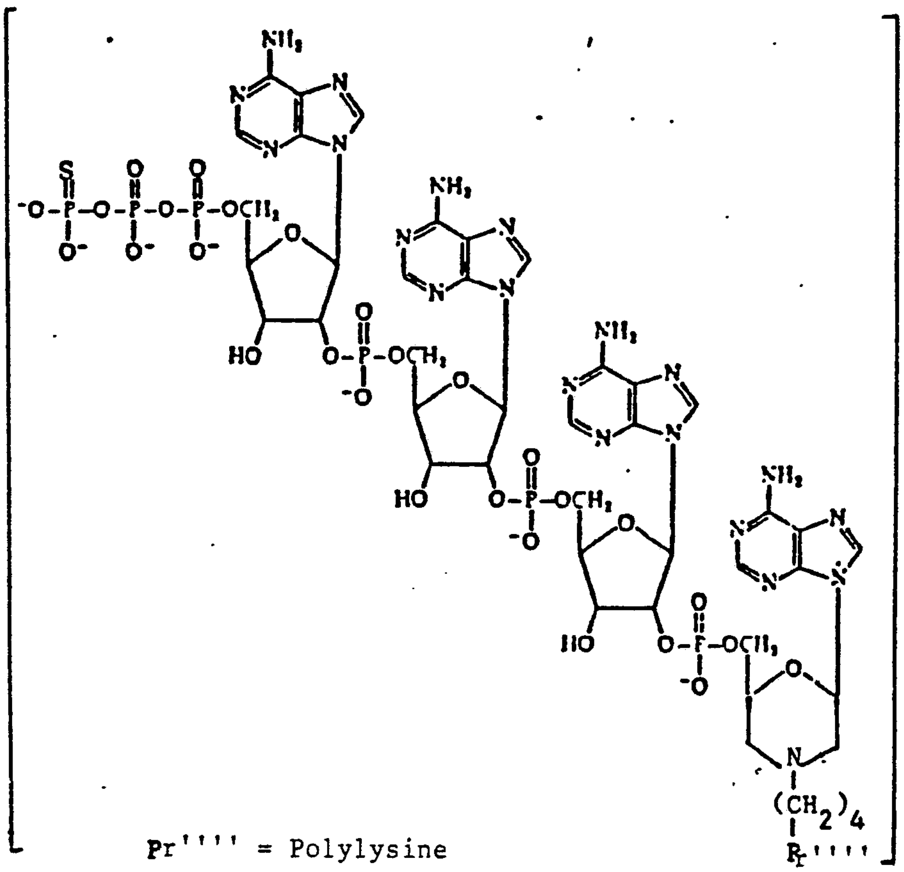

(XV)

Pr'''' = Polylysine

9. Process for preparing oligonucleotides according to claims 1 to 8, characterized in that the enzymatic synthesis of compounds of formula (Ibis) comprises:
- the polymerization of the compound of the following formula (Iquater):

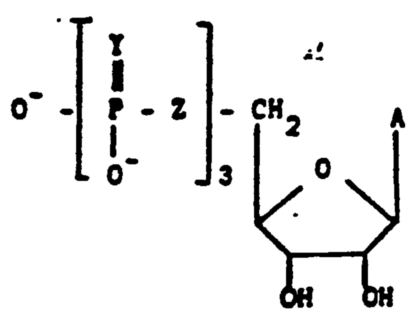

(Iquater)

in which:
- Y represents O, S, Se or NH;
- Z represents O, S or NH;
one at least of the elements Y and Z can be different from oxygen;
- A has the meanings mentioned in claims 1 to 7.

10. Process for preparing oligonucleotides according to claims 1 to 8, characterized in that it comprises:
- the polymerization of a compound of the following formula (XV Ibis):

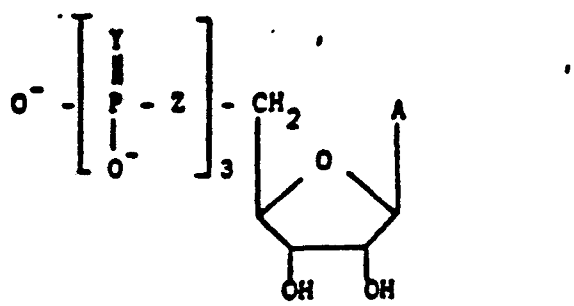

(XVIbis)

in which:
- Y represents O, S Se or NH;
- Z represents O, S or NH;
- Y and Z do not simultaneously represent oxygen;
- A has the meanings mentioned in the preceeding claims;
to obtain compounds of the following formula (XVI):

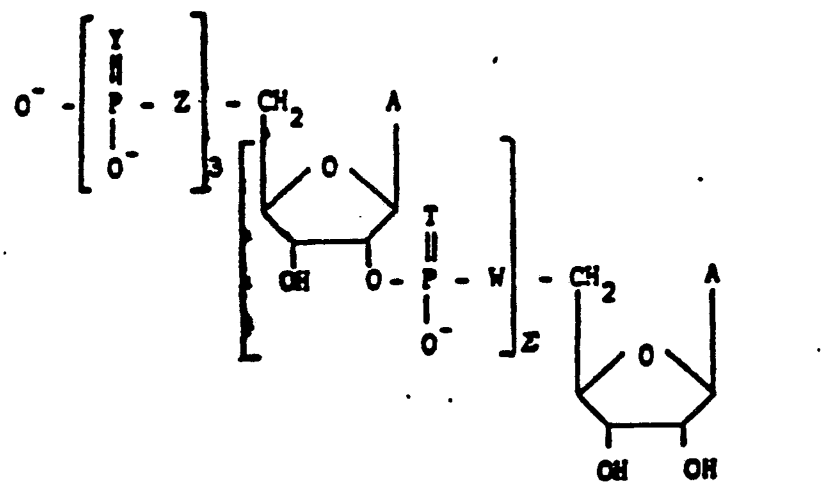

(XVI)

in which:
- Y and T, identical or different, represent O, S Se, NH;
- Z and W, identical or different, represent O, S NH;
- Y and Z do not simultaneously represent oxygen;
- Σ is an integer varying from 2 to 9;
- A is a base chosen among adenine or its derivates, particularly those of formula:

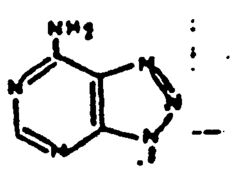
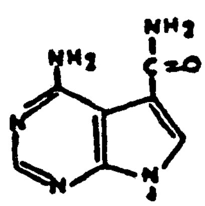
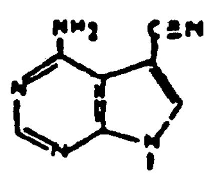
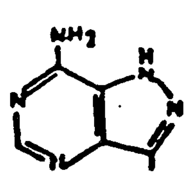
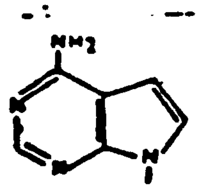
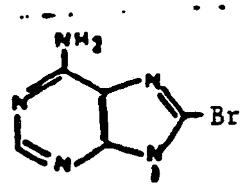

11. Process for preparing oligonucleotides according to claims 1 to 8, characterized in that it comprises:
- the polymerization of the compound ot the following formula (XVIIbis):

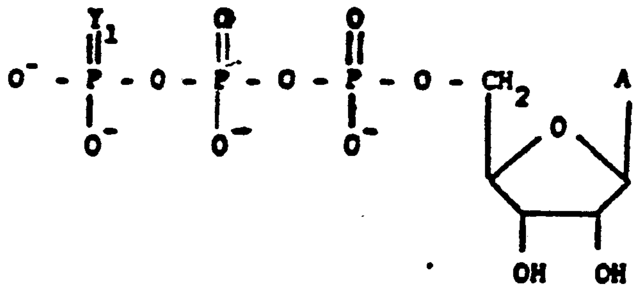

(XVIIbis)

in which:
- $Y_1$ represents NH, Se, S;
- A has the meaning mentioned in the preceeding claims to obtain the compounds of the following formula (XVII):

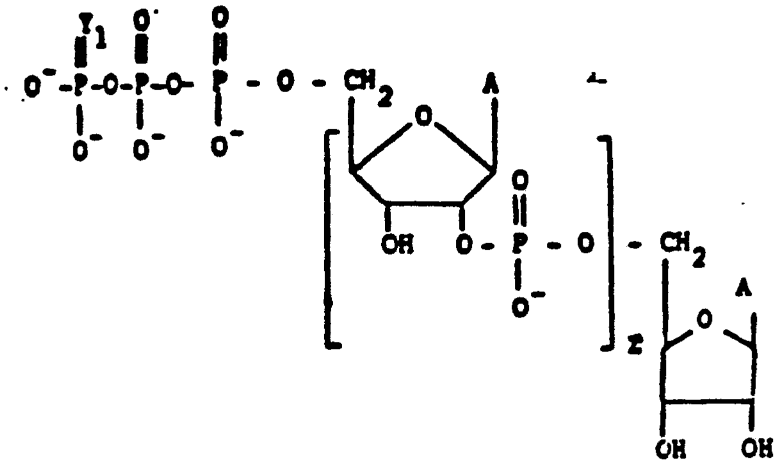

(XVII)

in which:
- $Y_1$ represents NH, Se, S;
- $\Sigma$ is an integer equal to n -1, n varying from 3 to 10;
- A has the meaning mentioned in the preceeding claims.

12. process for the preparation of oligonucleotides according to claims 1 to 8, characterized in that it comprises:
- the polymerization of a compound of the following formula (XVIIIbis):

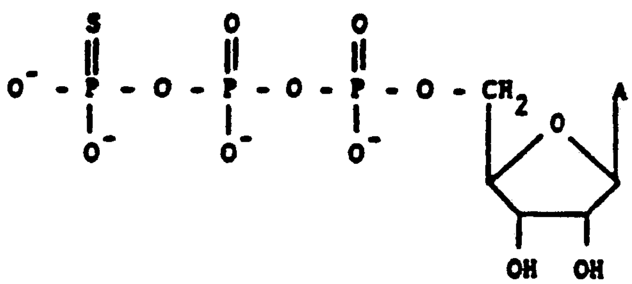

(XVIIIbis)

in which:
- A has the meaning mentioned in the preceeding claims, to obtain the compounds of the following formula (XVIII):

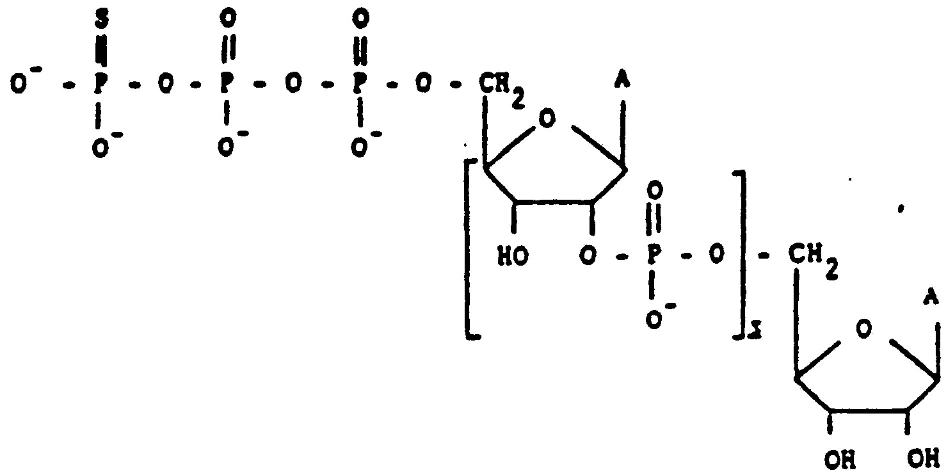

(XVIII)

in which:
- Σ has the preceeding mentioned meanings;
- A has the preceeding mentioned meaning, and preferably represents adenin.

13. Process for preparing oligonucleotides, characterized in that it comprises:
- the polymerization of compound of the following formula (XIXbis):

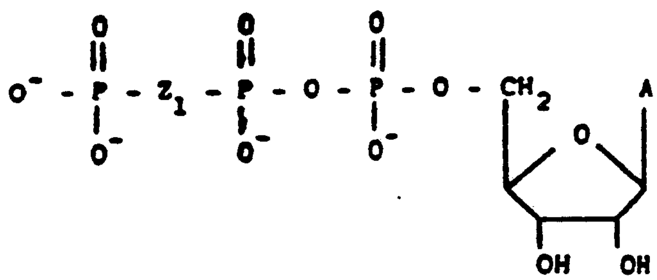

(XIXbis)

in which:
- $Z_1$ represents S or NH ans A has the meaning mentioned in the preceeding claims, to obtain compounds of the following formula (XIX):

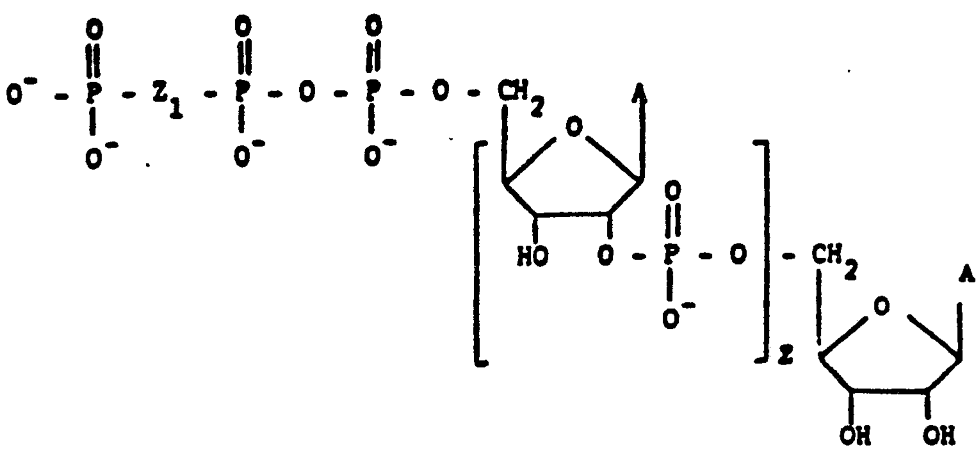

, (XIX)

in which:
- $Z_1$ represents NH or S;
- Σ is an integer equal to n -1, n varying from 3 to 10,
- A has the meaning mentioned in the preceeding claims.

14. Process for preparing oligonucleotides according to claims 1 to 8, characterized in that it comprises:
- the polymerization of the compound of the following formula (XXbis):

:

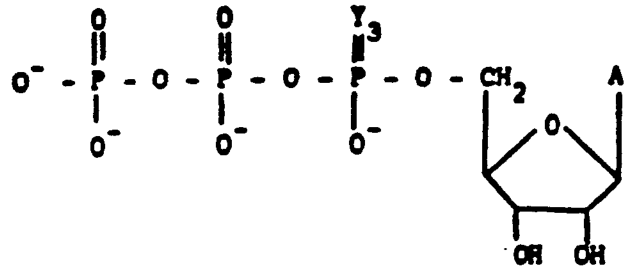

(XXbis)

in which:
- $Y_3$ represents S, Se, NH;
- A has the meaning mentioned in the preceedings claims, to obtain compounds of the following formula (XX):

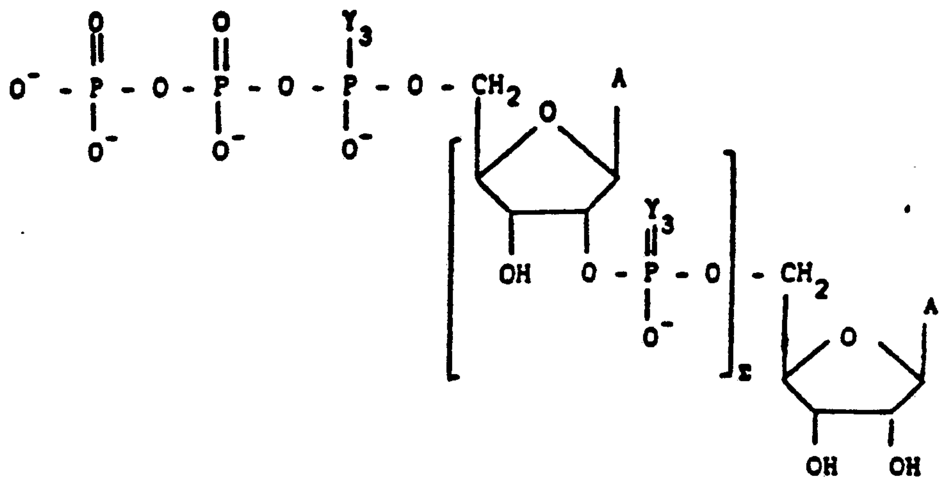

(XX)

in which:
- $Y_3$ represents NH, S or Se;
- Σ and A has the above mentioned meanings.

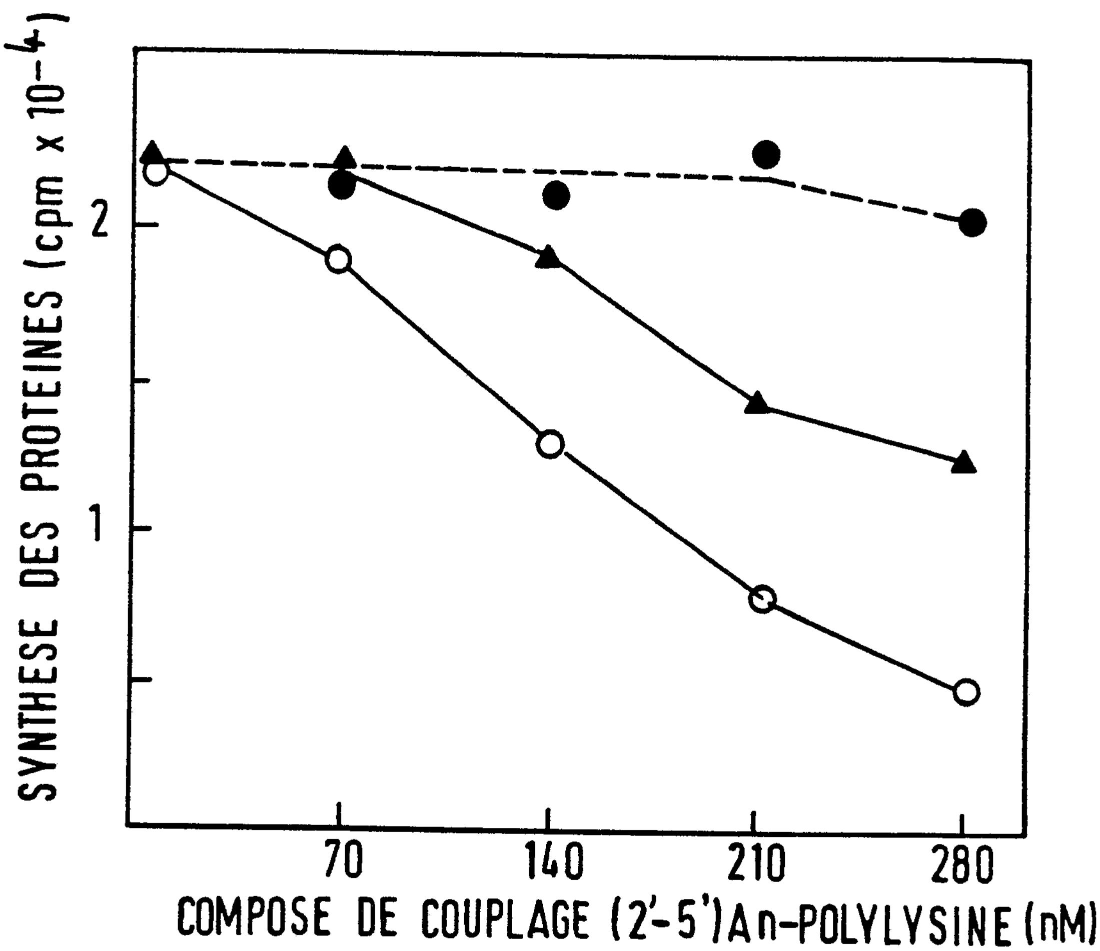
SYNTHESE DES PROTEINES (cpm x 10$^{-4}$)
2
1
70
140
210
280
COMPOSE DE COUPLAGE (2'-5')An-POLYLYSINE (nM)

FIG.1

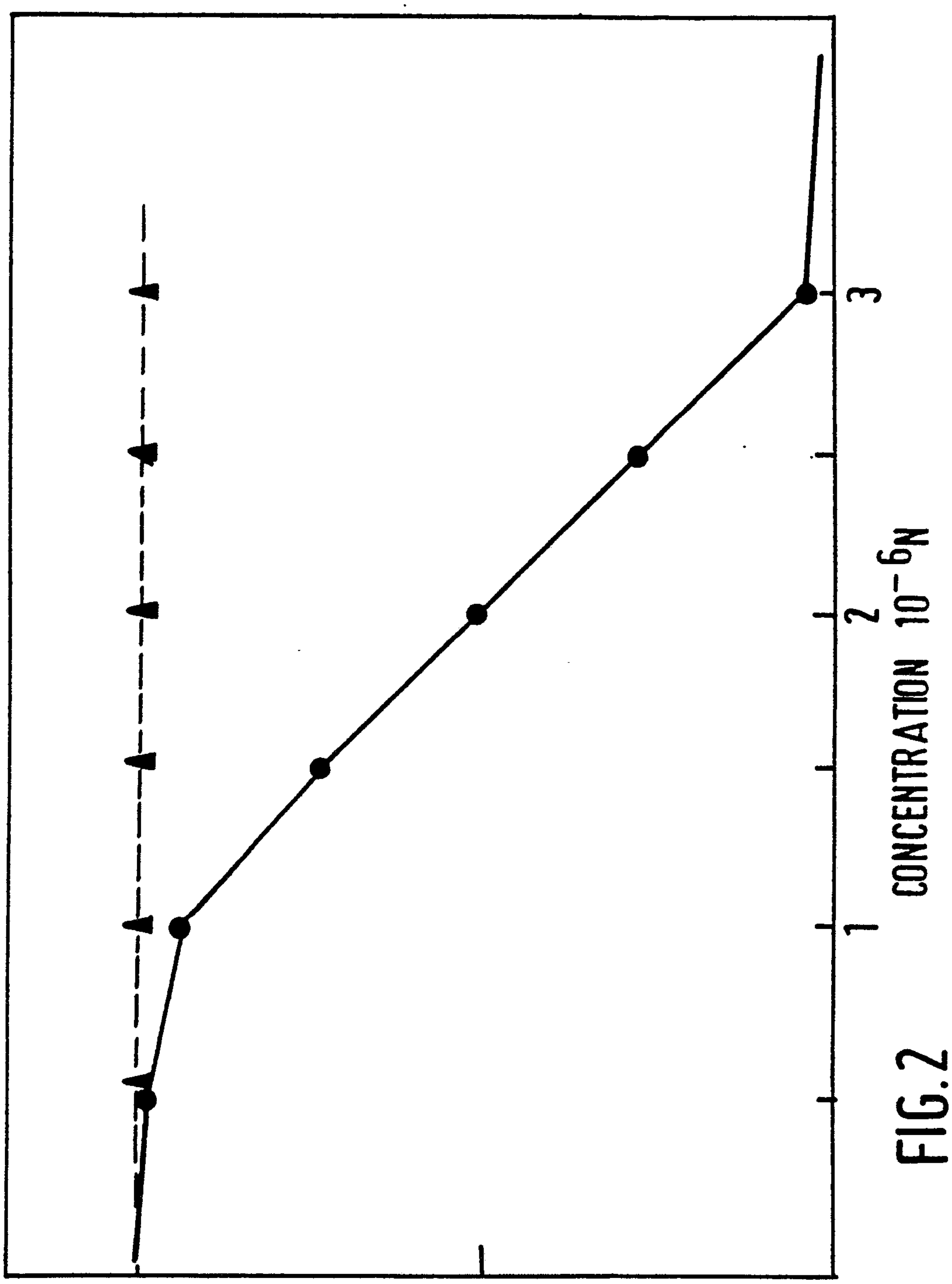

INCORPORATION DE [35S] METHIONINE
100
50
0
1
2
3
CONCENTRATION 10-6N


FIG.2